# EUROPEAN PATENT APPLICATION

(11) **EP 4 737 451 A1**
(43) Date of publication of application: **06.05.2026**
(21) Application number: 24831212.6
(22) Date of filing: 28.06.2024
(51) Int. Cl.: C07D 409/04, A61K 31/416, A61K 31/5377, A61K 31/4439, A61P 37/00, A61P 29/00, A61P 35/00, C07D 401/12, C07D 405/14, C07D 231/56

(54) **NOVEL CARBOXAMIDE DERIVATIVE COMPOUND AND PHARMACEUTICAL COMPOSITION COMPRISING SAME**

(30) Priority: 29.06.2023 KR 20230084471
(71) Applicant: Dong Wha Pharm. Co., Ltd., Seoul 04516 (KR); Cimplrx Co., Ltd., Chuncheon-si, Gangwon-do 24437 (KR)
(72) Inventor: HWANG, Yun Ha, Gunpo-si, Gyeonggi-do 15822 (KR); PARK, Whui Jung, Suwon-si, Gyeonggi-do 16509 (KR); JIN, Young Gu, Gunpo-si, Gyeonggi-do 15858 (KR); CHOI, Jung Uk, Suwon-si, Gyeonggi-do 16664 (KR); JEONG, Seo Hee, Suwon-si, Gyeonggi-do 16698 (KR); OH, Seong Jun, Yongin-si, Gyeonggi-do 16875 (KR); LEE, Sang Ho, Yongin-si, Gyeonggi-do 17166 (KR); YOUM, Ji Hyun, Suwon-si, Gyeonggi-do 16330 (KR); SEO, Ji Won, Yongin-si, Gyeonggi-do, 17085 (KR); CHO, Sung Jin, Seoul 05686 (KR); HAN, Si Yeon, Yongin-si, Gyeonggi-do 17085 (KR); HAN, Gun Hee, Suwon-si, Gyeonggi-do 16571 (KR)
(74) Representative: Gille Hrabal Partnerschaftsgesellschaft mbB Patentanwälte
(86) International application number: PCT/IB2024/056332
(87) International publication number: WO 2025/003988

(57) **Abstract**

The present invention relates to: a carboxamide derivative compound represented by chemical formula I; a stereoisomer thereof; a pharmaceutically acceptable salt thereof or a hydrate or solvate thereof; a composition comprising same; a method using same in the prevention or treatment of IRAK-4- or IRAK-1-related diseases; and uses thereof.

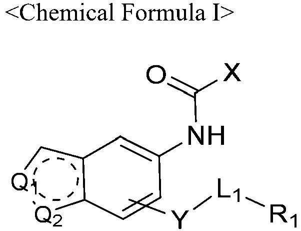

## Description

### Technical Field

The present invention relates to a novel carboxamide derivative compound, a stereoisomer thereof, a pharmaceutically acceptable salt thereof, or a hydrate or solvate thereof, and a pharmaceutical composition including the same.

### Background

An IL-1 receptor/toll-like receptor (IL-1R/TLR) signaling pathway plays an important role in immune and inflammatory reactions, and has been reported to be involved in the development of various inflammatory diseases and autoimmune diseases such as sepsis, asthma, arteriosclerosis, Alzheimer's disease, rheumatoid arthritis, atopy, hidradenitis suppurativa, psoriasis, rheumatoid arthritis, ulcerative colitis, lupus, etc. (Journal of Investigative Dermatology. 2019; 139(1): 146-156/ Annals of the New York Academy of Sciences. 2008; 1143: 21-34/ Journal of Immunology research. 2019; 1824624/International Immunopharmacology. 2007; 7(10): 1271-1285). In particular, it has recently been reported that blockade of the IL-1R/TLR signaling pathway by suppressing interleukin-1 receptorassociated kinase 4 (IRAK-4) is also effective in chronic inflammatory skin diseases such as psoriasis and atopy (Science Translational Medicine. 2023; 15: eabj3289).

It has been reported that IL-1R/TLR expression is also involved in proliferation of various cancer cells (Oncogene. 2008; 27(2): 218-224). In particular, it has been reported that ABC DLBCL, which is a type of diffuse large B-cell lymphoma (DLBCL), and Waldenstrom macroglobulinemia (WM), which is a lymphoid malignant tumor, are over-activated in IL-1R/TLR signaling due to MyD88 mutation (Nature. 2011; 470(7332): 115-119/ New England journal of medicine. 2012; 367(9): 826-833). Recently, it has been reported that IRAK-4 and IRAK-1 are also involved in acute myeloid leukemia (AML) and myelodysplastic syndrome (MDS) (Current Opinion in Hematology. 2022; 29(1): 8-19).

When a pathogen-associated molecular pattern (PAMP) commonly expressed in various pathogens and a damage-associated molecular pattern (DAMP) isolated from stressinduced or dying cells bind to IL-1R/TLR, a downstream signaling mechanism is activated, and an inflammatory reaction occurs. If this inflammatory reaction continues to occur, it develops into cancer (Nature Immunology. 2011 Jul 19; 12(8): 715-723).

Through the above reports, studies have been actively conducted to develop materials which block the TLR per se, which plays an important role in an IL-1R/TLR signaling path, or inhibit the IRAK, which deals with a downstream signal of the TLR. In particular, there are many studies targeting IRAK-4.

The IRAK is a serine/threonine kinase and has four subtypes (IRAK-1, IRAK-2, IRAK-M, IRAK-4). Among them, studies on IRAK-4 and IRAK-1 with a kinase function are mainly being conducted. In particular, IRAK-4, which plays an important role in forming a myddosome in the IL-1R/TLR signaling pathway, is the most frequently studied, and recently, studies on IRAK-1 are also being actively conducted. There is no drug which has been released to target IRAK-4 or IRAK-1 yet, but phase 1 and 2 clinical trials for various diseases (autoimmune diseases, inflammatory diseases, and blood cancer) are in progress.

### Detailed Description of the Invention

### Technical Problem

The present invention provide a novel carboxamide derivative compound, a stereoisomer thereof, a pharmaceutically acceptable salt thereof, or a hydrate or solvate thereof.

The present invention provide a pharmaceutical composition comprising a novel carboxamide derivative compound, a stereoisomer thereof, a pharmaceutically acceptable salt thereof, or a hydrate or solvate thereof.

The present invention provide a pharmaceutical composition for preventing or treating IRAK-4- or IRAK-1-related diseases, comprising a novel carboxamide derivative compound, a stereoisomer thereof, a pharmaceutically acceptable salt thereof, or a hydrate or solvate thereof as an active ingredient.

The present invention provide a method for preventing or treating IRAK-4- or IRAK-1-related diseases, comprising administering a novel carboxamide derivative compound, a stereoisomer thereof, a pharmaceutically acceptable salt thereof, or a hydrate or solvate thereof into a subject.

The present invention provide a use of a novel carboxamide derivative compound, a stereoisomer thereof, a pharmaceutically acceptable salt thereof, or a hydrate or solvate thereof for preventing or treating IRAK-4- or IRAK-1-related diseases.

The present invention provide a use of a novel carboxamide derivative compound, a stereoisomer thereof, a pharmaceutically acceptable salt thereof, or a hydrate or solvate thereof for preparing a medicament for preventing or treating IRAK-4- or IRAK-1-related diseases.

### Technical Solution

Accordingly, the present applicant has developed a compound having a novel structure which strongly inhibits IRAK-4 or IRAK-1. An activity of IRAK-4 and IRAK-1 enzymes and a change in secretion of inflammatory cytokines were evaluated, thus confirming that the compound of the present invention is a material with excellent activity.

The present invention relates to a compound useful for preventing or treating autoimmune diseases, inflammatory diseases or tumors related to interleukin-1 receptorassociated kinase (IRAK), and specifically, to a compound which inhibits the function of IRAK-4 or IRAK-1, and a pharmaceutical composition and a health functional food composition, including the same.

Hereinafter, the present invention will be described in more detail. All the combinations of various elements disclosed in the present invention may fall within the scope of the present invention. In addition, it may not be seen that the scope of the present invention is limited to the specific description below.

This research on the present invention was supported by the Korea Drug Development Fund funded by the Ministry of Science and ICT, the Ministry of Trade, Industry, and Energy, and the Ministry of Health and Welfare (task unique number: RS-2023-00284094, Republic of Korea). The task unique number may be used in combination as "1711200965."

### Compound represented by chemical formula I

The present invention provide a compound represented by chemical formula I, a stereoisomer thereof, a pharmaceutically acceptable salt thereof, or a hydrate or solvate thereof.

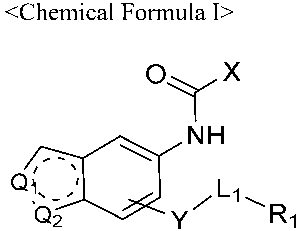

In above chemical formula I,
X is phenyl or pyridinyl;
at least one H of X is substituted with halogen, C1-C6 alkyl, C(=O)Ra, NH₂, NO₂, S(=O)₂Rb, 5- to 12-membered heteroaryl including one to three heteroatoms independently selected from the group consisting of N, O and S in a ring;
Ra and Rb are each independently C1-C6 alkyl, NH₂, or 3- to 12-membered heterocycloalkyl including one to three heteroatoms independently selected from the group consisting of N, O and S in a ring;
Y is a single bond, 6- to 14-membered arylene, 3- to 12-membered heterocycloalkylene including one to three heteroatoms independently selected from the group consisting of N, O and S in a ring, or 5- to 12-membered heteroarylene including one to three heteroatoms independently selected from the group consisting of N, O and S in a ring;
L₁ is a single bond, C1-C6 alkylene, C(=O), C(=O)NH, C(=O)NH-(C1-C6 alkylene), C(=O)NH-(C1-C6 alkylene)-C(=O)O, or C(=O)NH-(C1-C6 alkylene)-C(=O)NH;
R₁ is H, halogen, C1-C6 alkyl, C1-C6 alkoxy, OH, CF₃, NRcRd, 3- to 12-membered heterocycloalkyl including one to three heteroatoms independently selected from the group consisting of N, O and S in a ring, or 5- to 12-membered heteroaryl including one to three heteroatoms independently selected from the group consisting of N, O and S in a ring;
Rc and Rd are each independently H or C1-C6 alkyl;
Q₁ and Q₂ are each independently N or N-L₂-R₂, in which Q₁ and Q₂ are not simultaneously N;
when Q₂ is N, 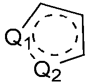 is 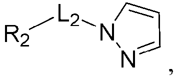 and when Q₁ is N, 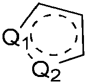 is 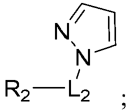
L₂ is C1-C6 alkylene or (C1-C6 alkylene)-O-(C1-C6 alkylene);
R₂ is 3-to 12-membered heterocycloalkyl including one to three heteroatoms independently selected from the group consisting of N, O and S in a ring, C1-C6 alkoxy, CONH₂, NReRf, SO₂Rg or OH;
Re, Rf and Rg are each independently H or C1-C6 alkyl.

In one embodiment, it may be provided that in above chemical formula I,
X is phenyl or pyridinyl;
at least one H of X is substituted with halogen, C(=O)Ra, NH₂, NO₂, S(=O)₂Rb, 5-to 12-membered heteroaryl including one to three heteroatoms independently selected from the group consisting of N, O and S in a ring;
Ra and Rb are each independently NH₂, or 3- to 12-membered heterocycloalkyl including one to three heteroatoms independently selected from the group consisting of N, O and S in a ring;
Y is a single bond, 6- to 14-membered arylene, 3- to 12-membered heterocycloalkylene including one to three heteroatoms independently selected from the group consisting of N, O and S in a ring, or 5- to 12-membered heteroarylene including one to three heteroatoms independently selected from the group consisting of N, O and S in a ring;
L₁ is a single bond, C1-C6 alkylene, C(=O), C(=O)NH, C(=O)NH-(C1-C6 alkylene), C(=O)NH-(C1-C6 alkylene)-C(=O)O, or C(=O)NH-(C1-C6 alkylene)-C(=O)NH;
R₁ is H, halogen, C1-C6 alkyl, C1-C6 alkoxy, OH, CF₃, NRcRd, 3- to 12-membered heterocycloalkyl including one to three heteroatoms independently selected from the group consisting of N, O and S in a ring, or 5- to 12-membered heteroaryl including one to three heteroatoms independently selected from the group consisting of N, O and S in a ring;
Rc and Rd are each independently C1-C6 alkyl;
Q₁ and Q₂ are each independently N or N-L₂-R₂, in which Q₁ and Q₂ are not simultaneously N;
when Q₂ is N, 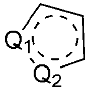 is 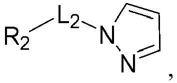 and when Q₁ is N, 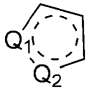 is 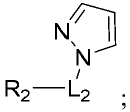
L₂ is C1-C6 alkylene or (C1-C6 alkylene)-O-(C1-C6 alkylene);
R₂ is 3-to 12-membered heterocycloalkyl including one to three heteroatoms independently selected from the group consisting of N, O and S in a ring, C1-C6 alkoxy, CONH₂, NReRf, SO₂Rg or OH;
Re, Rf and Rg are each independently C1-C6 alkyl.

In one embodiment, it may be provided that in above chemical formula I,
X is phenyl or pyridinyl;
at least one H of X is substituted with F, Cl, C(=O)Ra, NH₂, NO₂, S(=O)₂Rb, 5- or 6-membered heteroaryl including one to three heteroatoms independently selected from the group consisting of N and S in a ring;
Ra and Rb are each independently NH₂, or 5- to 7-membered heterocycloalkyl including one to three heteroatoms independently selected from the group consisting of N and O in a ring;
Y is a single bond, 6- to 12-membered arylene, 5- to 7-membered heterocycloalkylene including one to three heteroatoms independently selected from the group consisting of N, O and S in a ring, or 5- or 6-membered heteroarylene including one or two heteroatoms independently selected from the group consisting of N, O and S in a ring;
L₁ is a single bond, C1-C4 alkylene, C(=O), C(=O)NH, C(=O)NH-(C1-C2 alkylene), C(=O)NH-(C1-C5 alkylene)-C(=O)O, or C(=O)NH-(C1-C3 alkylene)-C(=O)NH;
R₁ is H, F, Cl, C1-C3 alkyl, C1-C3 alkoxy, OH, CF₃, NRcRd, 5- to 7-membered heterocycloalkyl including one to three heteroatoms independently selected from the group consisting of N and O in a ring, or 5- or 6-membered heteroaryl including one or two heteroatoms independently selected from the group consisting of N and O in a ring;
Rc and Rd are each independently C1-C3 alkyl;
Q₁ and Q₂ are each independently N or N-L₂-R₂, in which Q₁ and Q₂ are not simultaneously N;
when Q₂ is N, 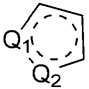 is 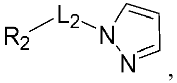 and when Q₁ is N, 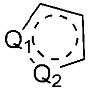 is 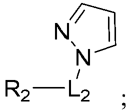
L₂ is C1-C6 alkylene or (C1-C3 alkylene)-O-(C1-C3 alkylene);
R₂ is 5- to 7-membered heterocycloalkyl including one to three heteroatoms independently selected from the group consisting of N and O in a ring, C1-C3 alkoxy, CONH₂, NReRf, SO₂Rg or OH;
Re, Rf and Rg are each independently C1-C3 alkyl.

In one embodiment, it may be provided that in above chemical formula I,
X is 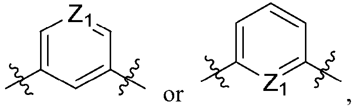 and Z₁ is CH or N.

In the present invention, the "Cm-Cn" (in which m and n are each independently an integer of 1 or more) may mean the number of carbons and, for example, "C1-C5 alkyl" may represent an alkyl having one to five carbon atoms.

In the present invention, "substitution" or "substituted with~" may be defined to include implicit conditions, in which the substitution follows a permitted valency of a substituted atom and a substituent and induces a compound stabilized by substitution, for example, a compound which is not naturally modified by rearrangement, cyclization, removal, etc.

In the present invention, a "single bond" may mean a case in which adjacent atoms or groups of atoms are directly bonded to each other.

In the present invention, "alkyl" may mean a linear (or straight-chain) saturated hydrocarbon group or a branched (or side-chain) saturated hydrocarbon group, unless otherwise specified. Examples of alkyl may include at least one selected from methyl, ethyl, n-propyl, isopropyl, n-butyl, sec-butyl, isobutyl, tert-butyl, n-pentyl, n-hexyl, n-heptyl, and the like, but are not limited thereto.

In the present invention, "alkylene" may mean a divalent functional group which is induced from alkyl as defined above, unless otherwise stated,

In the present invention, "cycloalkyl" may mean a monocyclic or polycyclic saturated hydrocarbon ring, unless otherwise stated. Examples of cycloalkyl may include at least one selected from cyclopropyl, cyclobutyl, cyclopentyl, cyclohexyl, cycloheptyl, cyclooctyl, bicyclopentyl, bicyclohexyl, bicycloheptyl, bicyclooctyl, spiropentyl, spirohexyl, spiroheptyl, spirooctyl, etc., but are not limited thereto.

In the present invention, "cycloalkylene" may mean a divalent functional group which is induced from cycloalkyl defined as above, unless otherwise stated.

In the present invention, "cycloalkenyl" may mean a monocyclic or polycyclic unsaturated hydrocarbon ring including at least one double bond, unless otherwise stated. Examples of cycloalkenyl may include at least one selected from cyclopropenyl, cyclobutenyl, cyclopentenyl, cyclohexenyl, etc., but are not limited thereto.

In the present invention, "cycloalkenylene" may mean a divalent functional group which is induced from cycloalkenyl defined as above, unless otherwise stated.

In the present invention, "heterocycloalkyl" may mean a monocyclic or polycyclic ring in which at least one carbon atom constituting a cycloalkyl ring defined as above is substituted with a heteroatom selected from the group consisting of N, O and S, unless otherwise stated. Examples of heterocycloalkyl may include at least one selected from oxiranyl, dioxolanyl, oxetanyl, tetrahydrofuranyl, tetrahydropyranyl, azetidinyl, pyrrolidinyl, piperidinyl, azepanyl, morpholinyl, piperazinyl, thiomorpholinyl, tetrahydrothiophenyl, tetrahydrothiopyranyl, etc., but are not limited thereto.

In the present invention, "heterocycloalkylene" may mean a divalent functional group which is induced from heterocycloalkyl defined as above, unless otherwise stated.

In the present invention, "heterocycloalkenyl" may mean a monocyclic or polycyclic ring in which at least one carbon atom constituting a cycloalkenyl ring defined as above is substituted with a heteroatom selected from the group consisting of N, O and S, unless otherwise stated. Examples of heterocycloalkenyl may include at least one selected from dihydropyridinyl, dihydropyranyl, dihydrothiopyranyl, etc., but are not limited thereto.

In the present invention, "heterocycloalkenylene" may mean a divalent functional group which is induced from heterocycloalkenyl defined as above, unless otherwise stated.

In the present invention, "aryl" may mean a monocyclic or polycyclic aromatic cyclic group having at least one fused or non-fused aromatic ring, unless otherwise stated. Examples of aryl may include at least one selected from phenyl, naphthalenyl, indenyl, anthracenyl and the like, but are not limited thereto.

In the present invention, "arylene" may mean a divalent functional group which is induced from aryl defined as above, unless otherwise stated.

In the present invention, "heteroaryl" may mean a monocyclic or polycyclic aromatic heterocyclic group including at least one heteroatom selected from the group consisting of N, O and S in a ring and having at least one fused or non-fused aromatic ring, unless otherwise stated. Examples of heteroaryl may include at least one selected from pyridinyl, thiophenyl, triazolyl, tetrazolyl, benzothiazolyl, benzothiophenyl, quinolinyl, indolyl, isoindolyl, benzofuranyl, benzopyrroyl, furanyl, pyrrolyl, thiazolyl, isothiazolyl, imidazolyl, pyrazolyl, oxazolyl, isoxazolyl, pyrazinyl, pyridazinyl, pyrimidinyl, isoquinoline, benzoxazolyl, benzoimidazolyl, dihydrobenzothiophenyl, purinyl, indolizinyl, chromenyl, pyrrolopyridinyl, pyrazolopyridinyl, thiadiazolopyridinyl, triazinyl, triazolopyrimidinyl, triazolopyridinyl, triazolopyridazinyl, indazolyl, imidazopyridinyl, imidazopyridazinyl, oxadiazolopyridinyl, benzothiadiazolyl, benzotriazolyl, benzooxadiazole, etc., but are not limited thereto.

In the present invention, "heteroarylene" may mean a divalent functional group which is induced from heteroaryl defined as above, unless otherwise stated.

In the present invention, "halogen" may be F, Cl, Br or I, unless otherwise stated.

Besides, the terms and abbreviations used in the present specification may have their original meanings, unless defined otherwise.

The present invention may provide a compound described in table 1 below, a stereoisomer thereof, a pharmaceutically acceptable salt thereof, or a hydrate or solvate thereof.

**[Table 1]**

| **Comp ound No.** | **Compound structure** | **Compound name** | **¹H-NMR δ ppm** |
|---|---|---|---|
| **1** | 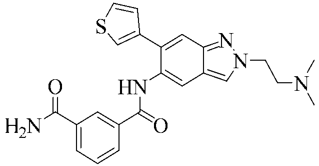 | N-(2-(2-(dimethylamino)ethyl)-6-(thiophen-3-yl)-2H-indazol-5-yl)isophthalamide | ¹H-NMR (DMSO-d⁶) 2.16(d, 6H), 2.77(t, 2H), 4.50(t, 2H), 7.29(m, 1H), 7.42(s, 1H), 7.49(m, 1H), 7.51(m, 1H), 7.58(s, 1H), 7.65(s, 1H), 7.75(s, 1H), 7.95(m, 1H), 7.98(m, 1H), 8.01(m, 1H), 9.33(s, 1H), 9.38(s, 1H), 9.89(s, 1H) |
| **2** | 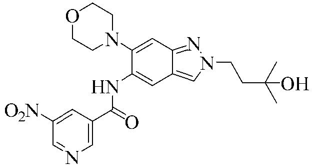 | N-(2-(3-hydroxy-3-methylbutyl)-6-morpholino-2H-indazol-5-yl)-5-nitronicotinamide | ¹H-NMR (DMSO-d⁶) 1.12(d, 6H), 2.00(t, 2H), 2.89(m, 4H), 3.75(m, 4H), 4.41(m, 2H), 4.48(s, 1H), 7.36(s, 1H), 8.26(s, 1H), 8.32(s, 1H), 8.95(s, 1H), 9.44(s, 1H), 9.53(s, 1H), 10.11(s, 1H) |
| **3** | 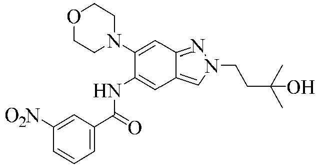 | N-(2-(3-hydroxy-3-methylbutyl)-6-morpholino-2H-indazol-5-yl)-3-nitrobenzamide | ¹H-NMR (DMSO-d⁶) 1.12(d, 6H), 2.00(t, 2H), 2.90(m, 4H), 3.78(m, 4H), 4.41(t, 2H), 4.47(s, 1H), 7.39(s, 1H), 7.86(m, 1H), 8.31(s, 1H), 8.35(m, 2H), 8.37(m, 1H), 8.70(s, 1H), 10.01(s, 1H) |
| **4** | 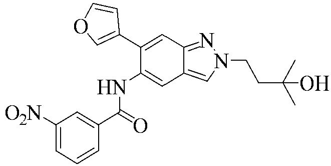 | N-(6-(furan-3-yl)-2-(3-hydroxy-3-methylbutyl)-2H-indazol-5-yl)-3-nitrobenzamide | ¹H-NMR (DMSO-d⁶) 1.12(d, 6H), 2.04(t, 2H), 4.49(m, 3H), 6.79(s, 1H), 7.65(s, 1H), 7.71(m, 1H), 7.74(s, 1H), 7.81(t, 1H), 7.84(s, 1H), 8.34(d, 1H), 8.40(s, 2H), 8.73(s, 1H), 10.28(s, 1H) |
| **5** | 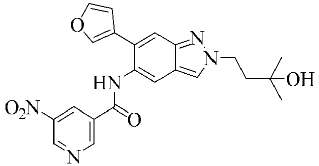 | N-(6-(furan-3-yl)-2-(3-hydroxy-3-methylbutyl)-2H-indazol-5-yl)-5-nitronicotinamide | ¹H-NMR (DMSO-d⁶) 1.12(s, 6H), 2.02(t, 2H), 4.48(m, 3H), 6.81(m, 1H), 7.66(m, 1H), 7.71(m, 1H), 7.77(m, 1H), 7.88(m, 1H), 8.41(s, 1H), 8.97(s, 1H), 9.41(s, 1H), 9.50(s, 1H), 10.43(s, 1H) |
| **6** | 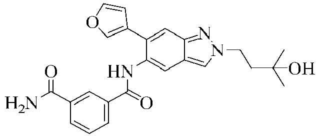 | N-(6-(furan-3-yl)-2-(3-hydroxy-3-methylbutyl)-2H-indazol-5-yl)isophthalamide | ¹H-NMR (DMSO-d⁶) 1.12(s, 6H), 2.02(m, 2H), 4.48(m, 3H), 6.80(s, 1H), 7.44(m, 1H), 7.56(m, 1H), 7.64(s, 1H), 7.70(m, 2H), 7.83(s, 1H), 8.02(m, 3H), 8.39(m, 2H), 9.97(s, 1H) |
| **7** | 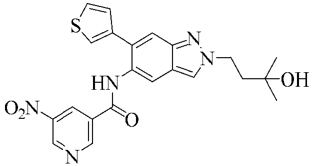 | N-(2-(3-hydroxy-3-methylbutyl)-6-(thiophen-3-yl)-2H-indazol-5-yl)-5-nitronicotinamide | ¹H-NMR (DMSO-d⁶) 1.13(s, 6H), 2.03(t, 2H), 4.50(m, 3H), 7.30(m, 1H), 7.52(m, 1H), 7.60(s, 1H), 7.67(s, 1H), 7.80(s, 1H), 8.43(s, 1H), 8.87(s, 1H), 9.30(s, 1H), 9.48(s, 1H), 10.38(s, 1H) |
| **8** | 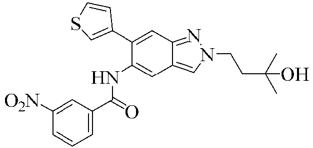 | N-(2-(3-hydroxy-3-methylbutyl)-6-(thiophen-3-yl)-2H-indazol-5-yl)-3-nitrobenzamide | ¹H-NMR (DMSO-d⁶) 1.23(s, 6H), 2.02(t, 2H), 4.49(m, 3H), 7.38(m, 1H), 7.64(m, 2H), 7.80(m, 2H), 8.02(m, 3H), 8.74(m, 2H), 10.20(s, 1H) |
| **9** | 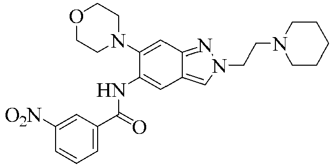 | N-(6-morpholino-2-(2-(piperidin-1-yl)ethyl)-2H-indazol-5-yl)-3-nitrobenzamide | ¹H-NMR (DMSO-d⁶) 1.36(m, 2H), 1.61(m, 4H), 1.79(m, 2H), 2.91(m, 4H), 3.47(m, 2H), 3.66(m, 2H), 3.79(m, 4H), 4.80(m, 2H), 7.41(m, 1H), 7.87(m, 1H), 8.40(m, 3H), 8.71(m, 1H), 9.08(m, 1H), 10.03(s, 1H) |
| **10** | 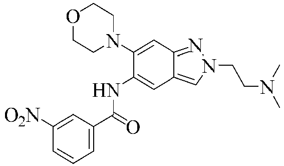 | N-(2-(2-(dimethylamino)ethyl)-6-morpholino-2H-indazol-5-yl)-3-nitrobenzamide | ¹H-NMR (DMSO-d⁶) 2.75(s, 6H), 2.90(m, 4H), 3.62(m, 2H), 3.78(m, 4H), 4.77(m, 2H), 7.40(m, 1H), 7.86(m, 1H), 8.38(m, 3H), 8.70(m, 1H), 9.23(m, 1H), 10.02(s, 1H) |
| **11** | 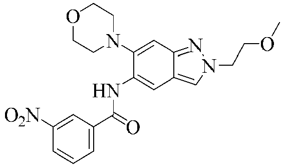 | N-(2-(2-methoxyethyl)-6-morpholino-2H-indazol-5-yl)-3-nitrobenzamide | ¹H-NMR (DMSO-d⁶) 2.90(m, 4H), 3.20(s, 3H), 3.78(m, 6H), 4.51(m, 2H), 7.40(s, 1H), 7.87(m, 1H), 8.28(s, 1H), 8.35(m, 2H), 8.43(m, 1H), 8.70(m, 1H), 10.01(s, 1H) |
| **12** | 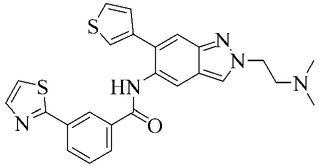 | N-(2-(2-(dimethylamino)ethyl)-6-(thiophen-3-yl)-2H-indazol-5-yl)-3-(thiazol-2-yl)benzamide | ¹H-NMR (DMSO-d⁶) 2.26(s, 6H), 2.92(m, 2H), 4.56(m, 2H), 7.32(m, 1H), 7.53(m, 1H), 7.61(m, 2H), 7.66(m, 1H), 7.82(m, 2H), 7.90(m, 1H), 7.94(m, 1H), 8.09(m, 1H), 8.35(s, 1H), 8.41(s, 1H), 10.03(s, 1H) |
| **13** | 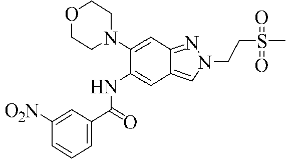 | N-(2-(2-(methylsulfonyl)ethyl)-6-morpholino-2H-indazol-5-yl)-3-nitrobenzamide | ¹H-NMR (DMSO-d⁶) 2.87(s, 3H), 2.94(m, 4H), 3.59(m, 4H), 3.75(t, 2H), 4.78(t, 2H), 6.99(d, 1H), 7.63(d, 1H), 7.71(m, 1H), 8.40(m, 3H), 8.78(s, 1H), 10.14(s, 1H) |
| **14** | 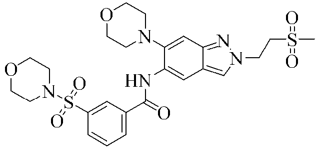 | N-(2-(2-(methylsulfonyl)ethyl)-6-morpholino-2H-indazol-5-yl)-3-(morpholinosulfonyl)benzami de | ¹H-NMR (DMSO-d⁶) 2.87(s, 3H), 2.89(m, 8H), 3.62(m, 4H), 3.78(m, 4H), 3.82(m, 2H), 4.80(m, 2H), 7.41(1H), 7.87(m, 1H), 7.94(m, 1H), 8.22(m, 1H), 8.26(m, 1H), 8.40(m, 2H), 9.97(s, 1H) |
| **15** | 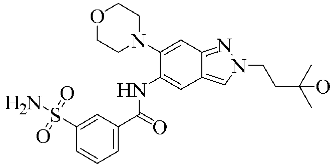 | N-(2-(3-hydroxy-3-methylbutyl)-6-morpholino-2H-indazol-5-yl)-4-sulfamoylbenzamide | ¹H-NMR (DMSO-d⁶) 1.12(d, 6H), 2.00(t, 2H), 2.88(m, 4H), 3.77(m, 4H), 4.41(m, 2H), 4.47(s, 1H), 7.42(s, 1H), 7.48(s, 2H), 7.78(t, 1H), 8.02(d, 1H), 8.12(d, 1H), 8.31(s, 1H), 8.37(s, 1H), 8.41(s, 1H), 9.88(s, 1H) |
| **16** | 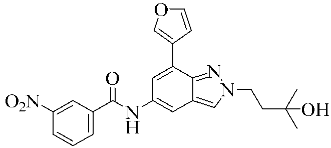 | N-(7-(furan-3-yl)-2-(3-hydroxy-3-methylbutyl)-2H-indazol-5-yl)-3-nitrobenzamide | ¹H-NMR (DMSO-d⁶) 1.11(s, 6H), 1.42(m, 2H), 1.81(d, 2H), 2.02(t, 2H), 2.32(m, 1H), 2.99(t, 2H), 3.77(d, 2H), 4.43(t, 2H), 4.47(s, 1H), 6.82(s, 1H), 7.48(s, 1H), 7.57(s, 1H), 7.85(s, 1H), 7.99(s, 1H), 8.36(s, 1H), 8.56(s, 1H), 9.63(s, 1H) |
| **17** | 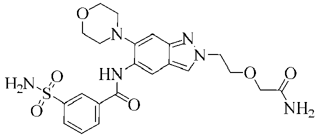 | N-(2-(2-(2-amino-2-oxoethoxy)ethyl)-6-morpholino-2H-indazol-5-yl)-3-sulfamoylbenzamide | ¹H-NMR (DMSO-d⁶) 2.91(m, 4H), 3.76-3.78(m, 6H), 3.93(t, 2H), 4.56(t, 2H), 7.19-7.23(m, 2H), 7.42(s, 1H), 7.48(s, 2H), 7.78(t, 1H), 8.02(d, 1H), 8.14(d, 1H), 8.36(s, 1H), 8.37(s, 1H), 8.43(s, 1H), 9.89(s, 1H) |
| **18** | 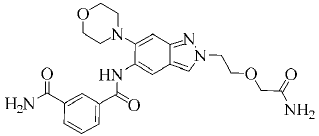 | N-(2-(2-(2-amino-2-oxoethoxy)ethyl)-6-morpholino-2H-indazol-5-yl)isophthalamide | ¹H-NMR (DMSO-d⁶) 2.90(br. s, 4H), 3.76(s, 2H), 3.78(br. s, 4H), 3.91(t, 2H), 4.56(t, 2H), 7.20(d, 2H), 7.40(s, 1H), 7.87(t, 1H), 8.19(d, 2H), 8.36(s, 3H), 8.42(d, 1H), 8.71(s, 1H), 10.00(s, 1H) |
| **19** | 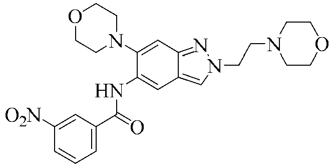 | N-(6-morpholino-2-(2-morpholinoethyl)-2H-indazol-5-yl)-3-nitrobenzamide | ¹H-NMR (DMSO-d⁶) 2.41(m, 4H), 2.83(t, 2H), 2.90(m, 4H), 3.51(t, 4H), 3.78(m, 4H), 4.53(t, 2H), 7.39(s, 1H), 7.86(m, 1H), 8.31(s, 1H), 8.35(m, 2H), 8.37(m, 1H), 8.70(s, 1H), 10.01(s, 1H) |
| **20** | 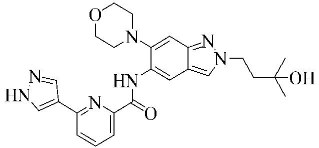 | N-(2-(3-hydroxy-3-methylbutyl)-6-morpholino-2H-indazol-5-yl)-6-(1H-pyrazol-4-yl)picolinamide | ¹H-NMR (DMSO-d⁶) 1.12(s, 6H), 2.00(m, 2H), 2.90(t, 4H), 3.79(t, 4H), 4.42(m, 2H), 4.48(s, 1H), 7.39(s, 1H), 7.94(m, 1H), 7.97(m, 1H), 9.30(s, 1H), 8.31(s, 1H), 8.50(s, 1H), 8.67(s, 1H), 10.94(s, 1H) |
| **21** | 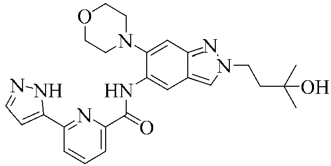 | N-(2-(3-hydroxy-3-methylbutyl)-6-morpholino-2H-indazol-5-yl)-6-(1H-pyrazol-5-yl)picolinamide | ¹H-NMR (DMSO-d⁶) 1.12(s, 6H), 2.00(m, 2H), 2.89(s, 4H), 3.83(s, 4H), 4.42(m, 2H), 4.48(s, 1H), 7.10(s, 1H), 7.39(s, 1H), 7.94(s, 1H), 8.09(s, 1H), 8.17(m, 1H), 8.31(s, 1H), 8.69(s, 1H), 11.00(s, 1H) |
| **22** | 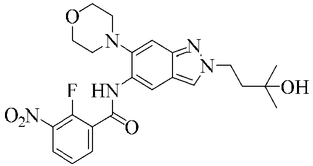 | 2-fluoro-N-(2-(3-hydroxy-3-methylbutyl)-6-morpholino-2H-indazol-5-yl)-3-nitrobenzamide | ¹H-NMR (DMSO-d⁶) 1.12(s, 6H), 1.99(t, 2H), 2.88(m, 4H), 3.77(m, 4H), 4.42(t, 2H), 4.44(s, 1H), 7.43(s, 1H), 7.59(t, 1H), 8.23(t, 1H), 8.29-8.32(m, 2H), 8.53(s, 1H), 10.03(s, 1H) |
| **23** | 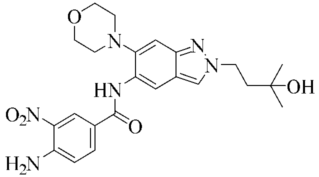 | 4-amino-N-(2-(3-hydroxy-3-methylbutyl)-6-morpholino-2H-indazol-5-yl)-3-nitrobenzamide | ¹H-NMR (DMSO-d⁶) 1.11(s, 6H), 1.98(t, 2H), 2.88(m, 4H), 3.81(m, 4H), 4.40(t, 2H), 4.47(s, 1H), 7.11(d, 1H), 7.40(s, 1H), 7.87(s, 2H), 7.92(d, 1H), 8.28(s, 1H), 8.39(s, 1H), 8.59(s, 1H), 9.69(s, 1H) |
| **24** | 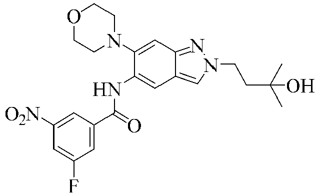 | 3-fluoro-N-(2-(3-hydroxy-3-methylbutyl)-6-morpholino-2H-indazol-5-yl)-5-nitrobenzamide | ¹H-NMR (DMSO-d⁶) 1.12(s, 6H), 1.91(t, 2H), 2.89(m, 4H), 3.76(m, 4H), 4.41(t, 2H), 4.48(s, 1H), 7.38(s, 1H), 8.22(d, 1H), 8.26(s, 1H), 8.32(s, 1H), 8.36(d, 1H), 8.57(s, 1H), 10.04(s, 1H) |
| **25** | 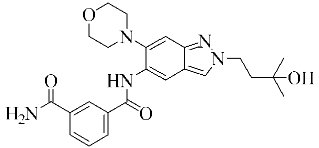 | N-(2-(3-hydroxy-3-methylbutyl)-6-morpholino-2H-indazol-5-yl)isophthalamide | ¹H-NMR (DMSO-d⁶) 1.12(d, 6H), 2.00(t, 2H), 2.88(m, 4H), 3.76(m, 4H), 4.41(t, 2H), 4.47(s, 1H), 7.40(s, 1H), 7.87(t, 1H), 8.19(d, 2H), 8.31(s, 1H), 8.33(m, 2H), 8.41(d, 1H), 8.69(s, 1H), 10.01(s, 1H) |
| **26** | 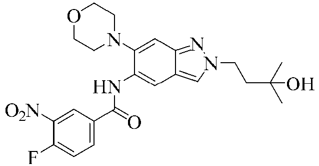 | 4-fluoro-N-(2-(3-hydroxy-3-methylbutyl)-6-morpholino-2H-indazol-5-yl)-3-nitrobenzamide | ¹H-NMR (DMSO-d⁶) 1.12(s, 6H), 1.99(t, 2H), 2.88(m, 4H), 3.76(m, 4H), 4.43(t, 2H), 4.47(s, 1H), 7.38(s, 1H), 7.79(t, 1H), 8.29(s, 1H), 8.31(s, 1H), 8.34(d, 1H), 8.65(d, 1H), 9.95(s, 1H) |
| **27** | 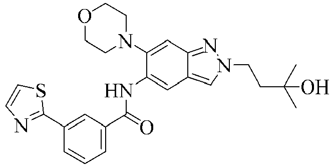 | N-(2-(3-hydroxy-3-methylbutyl)-6-morpholino-2H-indazol-5-yl)-3-(thiazol-2-yl)benzamide | ¹H-NMR (DMSO-d⁶) 1.12(s, 6H), 2.00(m, 2H), 2.91(t, 4H), 3.82(t, 4H), 4.42(m, 2H), 4.48(s, 1H), 7.43(s, 1H), 7.70(t, 1H), 7.86(d, 1H), 7.97(d, 1H), 8.03(d, 1H), 8.16(d, 1H), 8.31(s, 1H), 8.45(s, 1H), 8.51(s, 1H), 9.94(s, 1H) |
| **28** | 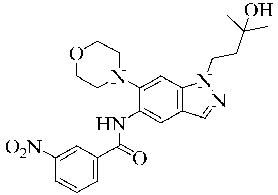 | N-(1-(3-hydroxy-3-methylbutyl)-6-morpholino-1H-indazol-5-yl)-3-nitrobenzamide | ¹H-NMR (DMSO-d⁶) 1.12(d, 6H), 1.90(t, 2H), 2.80(m, 4H), 3.69(m, 4H), 4.39(t, 2H), 4.42(s, 1H), 7.35(s, 1H), 7.70(m, 1H), 8.20(s, 1H), 8.24(m, 2H), 8.29(m, 1H), 8.60(s, 1H), 10.01(s, 1H) |
| **29** | 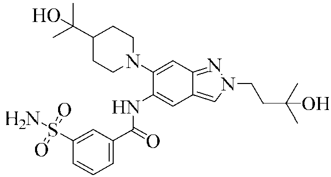 | N-(2-(3-hydroxy-3-methylbutyl)-6-(4-(2-hydroxypropan-2-yl)piperidin-1-yl)-2H-indazol-5-yl)-3-sulfamoylbenzamide | ¹H-NMR (DMSO-d⁶) 1.06(s, 6H), 1.11(s, 6H), 1.35(m, 1H), 1.43(m, 2H), 1.80(m, 2H), 1.99(m, 2H), 2.62-2.67(m, 2H), 3.07(d, 2H), 4.11(s, 1H), 4.41(t, 2H), 4.47(s, 1H), 7.36(s, 1H), 7.46(s, 2H), 7.74(t, 1H), 8.02(d, 1H), 8.12(d, 1H), 8.29(s, 1H), 8.36(s, 1H), 8.44(s, 1H), 9.81(s, 1H) |
| **30** | 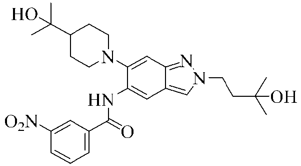 | N-(2-(3-hydroxy-3-methylbutyl)-6-(4-(2-hydroxypropan-2-yl)piperidin-1-yl)-2H-indazol-5-yl)-3-nitrobenzamide | ¹H-NMR (DMSO-d⁶) 1.05(s, 6H), 1.12(s, 6H), 1.34-1.37(m, 1H), 1.44-1.46(m, 2H), 1.78-1.81(m, 2H), 1.97-2.00(m, 2H), 2.64(t, 2H), 3.09-3.11(m, 2H), 4.11(s, 1H), 4.41(t, 2H), 4.47(s, 1H), 7.35(s, 1H), 7.83(t, 1H), 8.29(s, 1H), 8.36(d, 1H), 8.39(s, 1H), 8.42(m, 1H), 8.67(s, 1H), 9.94(s, 1H) |
| **31** | 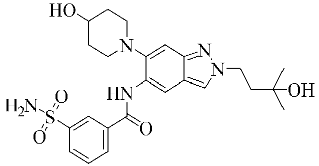 | N-(2-(3-hydroxy-3-methylbutyl)-6-(4-hydroxypiperidin-1-yl)-2H-indazol-5-yl)-3-sulfamoylbenzamide | ¹H-NMR (DMSO-d⁶) 1.12(s, 6H), 1.50-1.52(m, 2H), 1.79-1.81(m, 2H), 2.00(t, 2H), 2.60-2.62(m, 2H), 3.20-3.23(m, 2H), 3.52-3.54(m, 1H), 4.43(m, 3H), 7.36(s, 1H), 7.47(s, 2H), 7.74(t, 1H), 8.04(d, 1H), 8.15(d, 1H), 8.21(s, 1H), 8.30(s, 1H), 8.35(s, 1H), 9.79(s, 1H) |
| **32** | 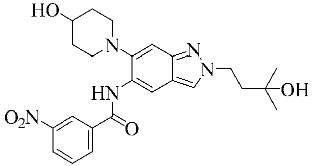 | N-(2-(3-hydroxy-3-methylbutyl)-6-(4-hydroxypiperidin-1-yl)-2H-indazol-5-yl)-3-nitrobenzamide | ¹H-NMR (DMSO-d⁶) 1.12(s, 6H), 1.46-1.52(m, 2H), 1.78-1.82(m, 2H), 2.00(t, 2H), 2.60-2.62(m, 2H), 3.19-3.22(m, 2H), 3.51-3.54(m, 1H), 4.41(m, 2H), 4.48(s, 1H), 7.35(s, 1H), 7.83(t, 1H), 8.29(s, 1H), 8.36(d, 1H), 8.38(s, 1H), 8.41(m, 1H), 8.62(s, 1H), 10.01(s, 1H) |
| **33** | 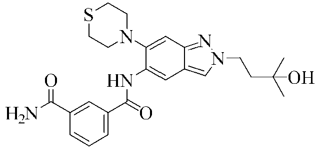 | N-(2-(3-hydroxy-3-methylbutyl)-6-thiomorpholino-2H-indazol-5-yl)isophthalamide | ¹H-NMR (DMSO-d⁶) 1.12(d, 6H), 2.00(t, 2H), 2.59(m, 4H), 3.41(m, 4H), 4.41(t, 2H), 4.47(s, 1H), 7.40(s, 1H), 7.87(t, 1H), 8.19(d, 2H), 8.35(m, 3H), 8.41(d, 1H), 8.69(s, 1H), 10.01(s, 1H) |
| **34** | 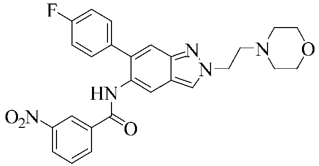 | N-(6-(4-fluorophenyl)-2-(2-morpholinoethyl)-2H-indazol-5-yl)-3-nitrobenzamide | ¹H-NMR (DMSO-d⁶) 2.41(br. S, 4H), 2.83(t, 2H), 3.51(t, 4H), 4.53(t, 2H), 7.32(t, 2H), 7.52(s, 1H), 7.58(t, 2H), 7.78(t, 1H), 8.17(d, 1H), 8.37(d, 1H), 8.43(s, 1H), 8.47(s, 1H), 8.49(s, 1H), 9.63(s, 1H) |
| **35** | 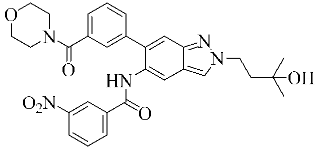 | N-(2-(3-hydroxy-3-methylbutyl)-6-(3-(morpholine-4-carbonyl)phenyl)-2H-indazol-5-yl)-3-nitrobenzamide | ¹H-NMR (DMSO-d⁶) 1.13(s, 6H), 2.06(t, 2H), 3.13(t, 4H), 3.59(t, 4H), 4.50(s, 1H), 4.51(t, 2H), 7.18(s, 1H), 7.44(m, 2H), 7.58(m, 3H), 8.14(s, 2H), 8.29(s, 1H), 8.44(s, 1H), 8.59(s, 1H), 10.27(s, 1H) |
| **36** | 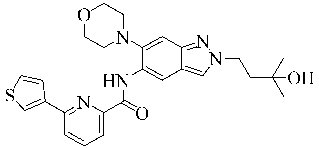 | N-(2-(3-hydroxy-3-methylbutyl)-6-morpholino-2H-indazol-5-yl)-6-(thiophen-3-yl)picolinamide | ¹H-NMR (DMSO-d⁶) 1.12(s, 6H), 1.99(m, 2H), 2.89(t, 4H), 3.76(t, 4H), 4.43(m, 2H), 4.48(s, 1H), 7.40(s, 1H), 7.75(dd, 1H), 7.99(dd, 1H), 8.10(m, 3H), 8.32(s, 1H), 8.39(dd, 1H), 8.69(s, 1H), 11.00(s, 1H) |
| **37** | 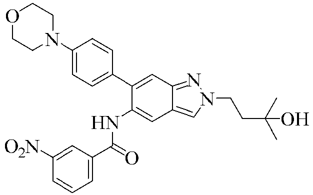 | N-(2-(3-hydroxy-3-methylbutyl)-6-(4-morpholinophenyl)-2H-indazol-5-yl)-3-nitrobenzamide | ¹H-NMR (DMSO-d⁶) 1.13(s, 6H), 2.03(t, 2H), 3.07(t, 4H), 3.68(t, 4H), 4.48(t, 2H), 4.50(s, 1H), 6.91(d, 2H), 7.32(d, 2H), 7.48(s, 1H), 7.75(s, 1H), 8.33(t, 2H), 8.39(s, 1H), 8.51(s, 2H), 10.07(s, 1H) |
| **38** | 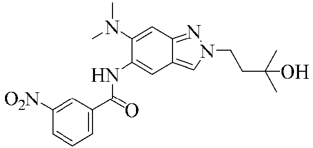 | N-(6-(dimethylamino)-2-(3-hydroxy-3-methylbutyl)-2H-indazol-5-yl)-3-nitrobenzamide | ¹H-NMR (DMSO-d⁶) 1.12(s, 6H), 1.98(t, 2H), 2.67(s, 6H), 4.41(m, 2H), 4.48(s, 1H), 7.27(s, 1H), 7.83(t, 1H), 8.17(s, 1H), 8.27(s, 1H), 8.35(d, 1H), 8.41(d, 1H), 8.70(s, 1H), 9.91(s, 1H) |
| **39** | 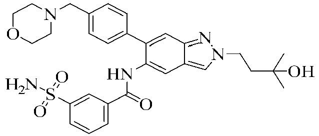 | N-(2-(3-hydroxy-3-methylbutyl)-6-(4-(morpholinomethyl)phenyl)-2H-indazol-5-yl)-3-sulfamoylbenzamide | ¹H-NMR (DMSO-d⁶) 1.13(s, 6H), 2.05(t, 2H), 2.28(br. s, 4H), 3.48(br. s, 4H), 3.62(s, 2H), 4.47-4.51(m, 3H), 7.13(d, 2H), 7.28(d, 2H), 7.40(br. s, 2H), 7.49(s, 1H), 7.61(t, 1H), 7.72(s, 1H), 7.91(d, 2H), 8.20(s, 1H), 8.40(s, 1H), 9.97(s, 1H) |
| **40** | 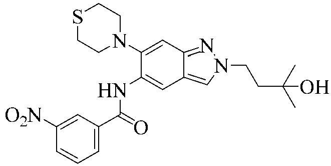 | N-(2-(3-hydroxy-3-methylbutyl)-6-thiomorpholino-2H-indazol-5-yl)-3-nitrobenzamide | ¹H-NMR (DMSO-d⁶) 1.12(d, 6H), 2.00(t, 2H), 2.59(m, 4H), 3.42(m, 4H), 4.41(t, 2H), 4.47(s, 1H), 7.20(s, 1H), 7.76(m, 1H), 8.33(s, 1H), 8.35(m, 2H), 8.37(m, 1H), 8.74(s, 1H), 10.05(s, 1H) |
| **41** | 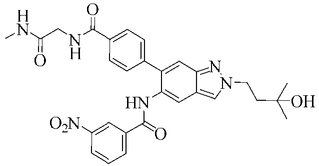 | N-(2-(3-hydroxy-3-methylbutyl)-6-(4-((2-(methylamino)-2-oxoethyl)carbamoyl)phenyl)-2H-indazol-5-yl)-3-nitrobenzamide | ¹H-NMR (DMSO-d⁶) 1.13(s, 6H), 2.03-2.05(m, 2H), 2.64(d, 3H), 4.38(d, 2H), 4.53-4.57(m, 3H), 7.45(s, 1H), 7.59(s, 1H), 7.73-7.74(m, 2H), 7.82(d, 2H), 7.99(d, 1H), 8.62(d, 1H), 8.57-8.58(m, 2H), 8.78(s, 1H), 9.01(t, 1H), 9.79(s, 1H) |
| **42** | 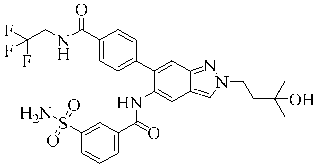 | N-(2-(3-hydroxy-3-methylbutyl)-6-(4-((2,2,2-trifluoroethyl)carbamoyl)phe nyl)-2H-indazol-5-yl)-3-sulfamoylbenzamide | ¹H-NMR (DMSO-d⁶) 1.13(s, 6H), 2.04-2.05(m, 2H), 3.26(s, 2H), 4.01-4.04(m, 2H), 4.50-4.53(m, 3H), 7.40(s, 1H), 7.56(d, 1H), 7.59(s, 1H), 7.62(t, 1H), 7.72(s, 1H), 7.82(d, 2H), 7.91(t, 2H), 8.21(s, 1H), 8.44(s, 1H), 9.00(t, 1H), 10.20(s, 1H) |
| **43** | 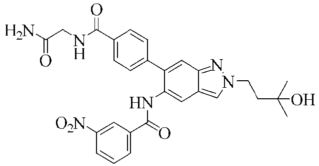 | N-(6-(4-((2-amino-2-oxoethyl)carbamoyl)phenyl)-2-(3-hydroxy-3-methylbutyl)-2H-indazol-5-yl)-3-nitrobenzamide | ¹H-NMR (DMSO-d⁶) 1.13(s, 6H), 2.04-2.06(m, 2H), 3.75(d, 2H), 4.50-4.53(m, 3H), 6.97(s, 1H), 7.30(s, 1H), 7.54(d, 2H), 7.59(s, 1H), 7.74(d, 2H), 7.82(d, 2H), 8.16(d, 1H), 8.36(d, 1H), 8.45(s, 1H), 8.56-8.58(m, 2H), 10.29(s, 1H) |
| **44** | 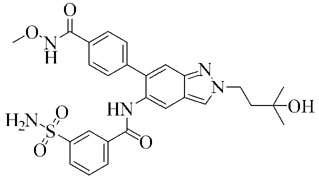 | N-(2-(3-hydroxy-3-methylbutyl)-6-(4-(methoxycarbamoyl)phenyl)-2H-indazol-5-yl)-3-sulfamoylbenzamide | ¹H-NMR (DMSO-d⁶) 1.14(s, 6H), 2.03(t, 2H), 3.61(s, 3H), 4.49-4.50(m, 3H), 7.36-7.40(m, 5H), 7.50-7.53(m, 2H), 7.70(s, 1H), 7.92-7.98(m, 3H), 8.24(s, 1H), 8.40(s, 1H), 9.60(s, 1H), 9.99(s, 1H) |
| **45** | 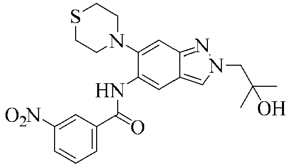 | N-(2-(2-hydroxy-2-methylpropyl)-6-thiomorpholino-2H-indazol-5-yl)-3-nitrobenzamide | ¹H-NMR (DMSO-d⁶) 1.10(s, 6H), 2.59(m, 4H), 3.42(m, 4H), 4.31(t, 2H), 4.82(s, 1H), 7.18(s, 1H), 7.73(m, 1H), 8.30(s, 1H), 8.31(m, 2H), 8.37(m, 1H), 8.74(s, 1H), 10.05(s, 1H) |
| **46** | 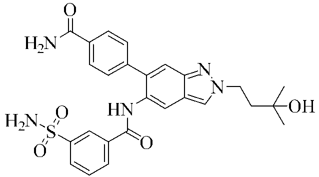 | N-(6-(4-carbamoylphenyl)-2-(3-hydroxy-3-methylbutyl)-2H-indazol-5-yl)-3-sulfamoylbenzamide | ¹H-NMR (DMSO-d⁶) 1.14(s, 6H), 2.03(t, 2H), 4.50-4.52(m, 3H), 7.29(s, 1H), 7.40(s, 2H), 7.52(d, 2H), 7.58(s, 1H), 7.63(t, 1H), 7.72(s, 1H), 7.81(d, 2H), 7.89(s, 1H), 7.93(d, 2H), 8.21(s, 1H), 8.44(s, 1H), 10.10(s, 1H) |
| **47** | 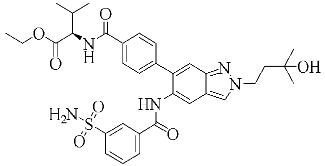 | ethyl (4-(2-(3-hydroxy-3-methylbutyl)-5-(3-sulfamoylbenzamido)-2H-indazol-6-yl)benzoyl)-D-valinate | ¹H-NMR (DMSO-d⁶) 0.90(d, 3H), 0.93(d, 3H), 1.13-1.15(m, 9H), 2.04-2.05(t, 2H), 2.12-2.16(m, 1H), 4.45-4.49(m, 5H), 4.62-4.64(m, 1H), 7.40(s, 2H), 7.54(d, 2H), 7.58(s, 1H), 7.73(s, 1H), 7.83(d, 2H), 7.90(d, 2H), 7.96(d, 1H), 8.23(s, 1H), 8.44(s, 1H), 8.48(d, 1H), 10.10(s, 1H) |
| **48** | 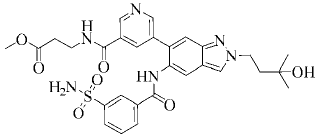 | methyl 3-(5-(2-(3-hydroxy-3-methylbutyl)-5-(3-sulfamoylbenzamido)-2H-indazol-6-yl)nicotinamido)propanoate | ¹H-NMR (DMSO-d⁶) 1.13(s, 6H), 2.06(t, 2H), 2.64(t, 2H), 2.85(t, 2H), 3.56(s, 3H), 4.50(s, 1H), 4.53(t, 2H), 7.39(d, 2H), 7.70(s, 1H), 7.74(s, 1H), 7.93(d, 2H), 7.97(s, 1H), 8.15(s, 1H), 8.27(s, 1H), 8.46(s, 1H), 8.68(s, 1H), 8.73(d, 1H), 8.82(d, 1H), 10.35(s, 1H) |
| **49** | 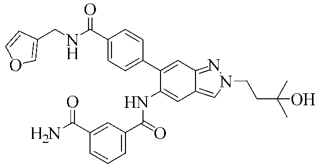 | N-(6-(4-((furan-3-ylmethyl)carbamoyl)phenyl)-2-(3-hydroxy-3-methylbutyl)-2H-indazol-5-yl)isophthalamide | ¹H-NMR (DMSO-d⁶) 1.13(s, 6H), 2.04(m, 2H), 4.41(d, 2H), 4.47-4.50(m, 3H), 5.72(s, 1H), 6.21(d, 1H), 6.34(s, 1H), 7.40(s, 1H), 7.48(t, 1H), 7.52(d, 2H), 7.54(s, 1H), 7.57(s, 1H), 7.72(s, 1H), 7.82-7.88(m, 2H), 7.98(d, 1H), 8.00(s, 1H), 8.27(s, 1H), 8.43(s, 1H), 8.89(t, 1H), 9.96(s, 1H) |
| **50** | 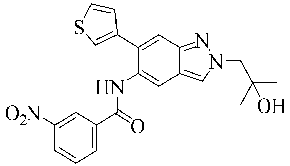 | N-(2-(2-hydroxy-2-methylpropyl)-6-(thiophen-3-yl)-2H-indazol-5-yl)-3-nitrobenzamide | ¹H-NMR (DMSO-d⁶) 1.10(s, 6H), 4.34(s, 2H), 4.86(s, 1H), 7.29(d, 1H), 7.50(m, 1H), 7.59(s, 1H), 7.67(s, 1H), 7.76-7.79(m, 2H), 8.24(d, 1H), 8.32(s, 1H), 8.37(d, 1H), 8.62(s, 1H) |
| **51** | 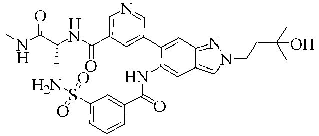 | (R)-5-(2-(3-hydroxy-3-methylbutyl)-5-(3-sulfamoylbenzamido)-2H-indazol-6-yl)-N-(1-(methylamino)-1-oxopropan-2-yl)nicotinamide | ¹H-NMR (DMSO-d⁶) 1.14(s, 6H), 1.15(d, 3H), 2.04(t, 2H), 2.65(d, 3H), 4.50-4.54(m, 3H), 4.62-4.65(m, 1H), 7.72(s, 1H), 7.78(t, 1H), 7.82 (s, 2H), 8.02(d, 1H), 8.15(s, 1H), 8.34(s, 1H), 8.37(s, 1H), 8.39(s, 1H), 8.48(s, 1H), 8.68(d, 1H), 8.94(s, 1H), 9.21(d, 1H), 9.46(m, 1H), 9.94(s, 1H) |
| **52** | 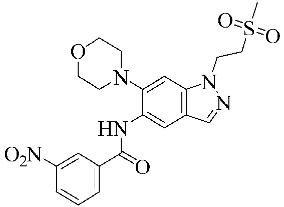 | N-(1-(2-(methylsulfonyl)ethyl)-6-morpholino-1H-indazol-5-yl)-3-nitrobenzamide | ¹H-NMR (DMSO-d⁶) 2.90(s, 3H), 2.95(m, 4H), 3.72(t, 2H), 3.78(m, 4H), 4.79(t, 2H), 7.60(s, 1H), 7.87(t, 1H), 8.07(s, 1H), 8.31(s, 1H), 8.38(d, 1H), 8.43(d, 1H), 8.72(s, 1H), 10.01(s, 1H) |
| **53** | 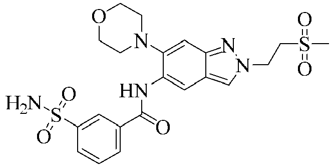 | N-(2-(2-(methylsulfonyl)ethyl)-6-morpholino-2H-indazol-5-yl)-3-sulfamoylbenzamide | ¹H-NMR (DMSO-d⁶) 2.90(s, 3H), 2.98(m, 4H), 3.73(t, 2H), 3.79(m, 4H), 4.78(t, 2H), 7.59(s, 1H), 7.69(s, 2H), 7.80(t, 1H), 7.99(d, 1H), 8.07(s, 1H), 8.18(m, 1H), 8.33(s, 1H), 8.38(s, 1H), 9.91(s, 1H) |

### Method for preparing compound represented by chemical formula I

The present invention may provide a method for preparing a compound represented by chemical formula I, a compound of above table 1, a stereoisomer thereof, a pharmaceutically acceptable salt thereof, or a hydrate or solvate thereof.

A compound represented by chemical formula I of the present invention, a compound of above table 1, a stereoisomer thereof, a pharmaceutically acceptable salt thereof, or a hydrate or solvate thereof may be prepared, for example, according to a method of reaction formula 1 below, but is not limited thereto.

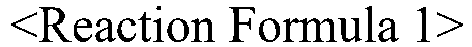

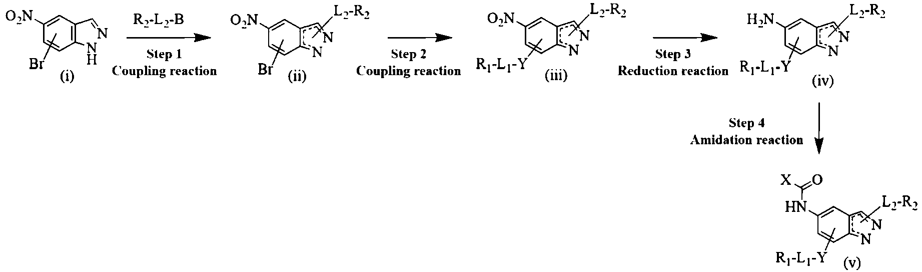

In above reaction formula 1, each of R₁, R₂, L₁, L₂, X and Y may be the same as defined in an item of the compound represented by above chemical formula I.

### [Step 1] Coupling reaction

It may be possible to prepare a 1- or 2-substituted indazole derivative (compound (ii)), proceeding from a starting material (compound (i)) (see reaction formula 1 above). Reactions useful for this purpose may optionally include reactions using a base with R₂-L₂-B (B is chloride, bromide, iodide or 4-methylbenzenesulfonate) in a solvent. Optionally, when B in R₂-L₂-B is chloride or bromide, it may be also possible to add an alkali metal iodide, such as potassium iodide or sodium iodide.

As the base, both an organic base and an inorganic base may be used, and for example, the inorganic base may be potassium carbonate, cesium carbonate, or sodium hydride. In the case of reactive halides, it may be also possible in some cases to use N-cyclohexyl-N-methylcyclohexanamine as the organic base. As the solvent, for example, 1-methylpyrrolidin-2-one, DMF, DMSO, or THF may be used.

### [Step 2] Coupling reaction

This may be a step of preparing compound (iii) in such a way that a palladium catalyst or a nickel catalyst; and an equivalent or excessive amount of boronic acid, boronic acid pinacol ester (for Suzuki-Miyaura coupling), an organotin reagent (for Stille coupling), or an alkene compound (for Heck reaction) are subjected to a coupling reaction with a condensation product in a pressure tube or a general reactor in the presence of a base in a solvent inert to the reaction, and stirred under heating conditions for 0.5 to 24 hours. Specifically, compound (iii) may be prepared by stirring at 20 to 120°C for 0.5 to 24 hours.

The solvent may not be particularly limited as long as it is inert to this reaction, but methanol, ethanol, tetrahydrofuran, 1,2-dimethoxyethane, 1,4-dioxane, water, N,N-dimethylformamide, dimethyl sulfoxide, benzene, toluene, xylene, a mixture thereof, or the like may be used.

As the palladium catalyst, tetrakis(triphenylphosphine)palladium, [1,1'-bis(diphenylphosphino)ferrocene]dichloropalladium, tris(dibenzylideneacetone)dipalladium, palladium acetate, acetylacetonpalladium, bis(triphenylphosphine)palladium dichloride, or the like may be used. As the nickel catalyst, [1,1'-bis(diphenylphosphino)ferrocene]nickel dichloride, bis(triphenylphosphine)nickel dichloride, or the like may be used.

As the base, both organic and inorganic bases may be available and, for example, an organic base such as triethylamine, diisopropylethylamine, 1,8-diazabicyclo[5.4.0]-7-undecene (DBU), 1,5-diazabicyclo[4.3.0]-5-nonene (DBN), or the like; or an inorganic base such as potassium hydrogen carbonate, sodium hydrogen carbonate, potassium carbonate, sodium carbonate, potassium hydroxide, sodium hydroxide, potassium phosphate, sodium phosphate, or the like may be used.

### [Step 3] Reduction reaction

This may be a step of preparing compound (iv) in such a way that compound (iii) is stirred under cooling or heating conditions for 3 to 96 hours using palladium/charcoal and hydrogen gas in a solvent. Specifically, compound (iv) may be prepared by stirring at room temperature (10 to 25°C) to 60°C for 0.5 to 48 hours.

The solvent may not be particularly limited, as long as it is a solvent that does not inhibit this reaction, but methanol, ethanol, tetrahydrofuran, ethyl acetate, dichloromethane, a mixture thereof, or the like may be used.

### [Step 4] Amidation reaction

This may be a step of preparing compound 1C in such a way that compound (iv) is stirred under cooling or heating conditions for 1 to 24 hours using an equivalent or excessive amount of corresponding X-substituted aromatic carboxylic acid and a condensing agent in a solvent inert to the reaction. Specifically, compound (v) may be prepared by stirring at room temperature to 120°C for 1 to 8 hours.

The solvent may not be particularly limited, as long as it is inert to this reaction, but N,N-dimethylformamide, dimethylacetamide, dichloromethane, 1,2-dichloroethane, chloroform, tetrahydrofuran, 1,2-dimethoxyethane, acetonitrile, a mixture thereof, or the like may be used.

As the condensing agent, pentafluorophenyl trifluoroacetate, dicyclocarbodiimide (DCC), 1-ethyl-3-(3-dimethylaminopropyl)carbodiimide (EDCI), 1,1'-carbonyldiimidazole, etc., may be used.

In addition, additives or bases may be further used in the reaction. As the additive, N-hydroxysuccinimide (HOSu), 1-hydroxybenzotriazole (HOBt), 1-hydroxy-7-azabenzotriazole (HOAt), etc may be used. As the base, an organic base such as triethylamine, diisopropylethylamine, etc.; or an inorganic base such as potassium carbonate, sodium carbonate, potassium hydroxide, sodium hydroxide, or the like may be used.

### Composition comprising compound represented by chemical formula I, method for using compound represented by chemical formula I, and use of compound represented by chemical formula I

The present invention provide a pharmaceutical composition comprising a compound represented by above chemical formula I, a compound of above table 1, a stereoisomer thereof, a pharmaceutically acceptable salt thereof, or a hydrate or solvate thereof as an active ingredient.

The present invention provide a health functional food composition comprising a compound represented by above chemical formula I, a compound of above table 1, a stereoisomer thereof, a pharmaceutically acceptable salt thereof, or a hydrate or solvate thereof.

In the present invention, "stereoisomers" may include diastereomers and optical isomers, and the optical isomers may include not only enantiomers, but also a mixture of the enantiomers as well as racemates.

In the present invention, "pharmaceutically acceptable" may mean that it is physiologically acceptable and does not usually cause an allergic reaction, such as gastrointestinal disorder or dizziness, or a reaction similar thereto when administered to a subject.

The pharmaceutically acceptable salt of the present invention may be prepared by a conventional method known to those skilled in the art.

The "hydrate" of the present invention may refer to one in which a compound represented by chemical formula I, a compound of above table 1, a stereoisomer thereof, or a pharmaceutically acceptable salt thereof and water are bound by a non-covalent intermolecular force, and may include a stoichiometric or non-stoichiometric amount of water. Specifically, the hydrate may include water at a ratio of about 0.25 mol to about 10 mol based on 1 mol of an active ingredient, and more specifically include about 0.5 mol, about 1 mol, about 1.5 mol, about 2 mol, about 2.5 mol, about 3 mol, about 5 mol, etc.

The "solvate" of the present invention may refer to one in which a compound represented by chemical formula I, a compound of above table 1, a stereoisomer thereof, or a pharmaceutically acceptable salt thereof and a solvent other than water are bound by a non-covalent intermolecular force, and may include a stoichiometric or non-stoichiometric amount of the solvent. Specifically, the solvate may include a solvent molecule at a ratio of about 0.25 mol to about 10 mol based on 1 mol of an active ingredient, and more specifically include about 0.5 mol, about 1 mol, about 1.5 mol, about 2 mol, about 2.5 mol, about 3 mol, about 5 mol, etc.

A compound represented by above chemical formula I, a compound of above table 1, a stereoisomer thereof, a pharmaceutically acceptable salt thereof, or a hydrate or solvate thereof, and a pharmaceutical composition comprising the same as an active ingredient may exhibit an IRAK-4 and/or IRAK-1 inhibitory activity, and may be used for preventing, treating or ameliorating IRAK-4- or IRAK-1-related diseases.

In the present invention, "IRAK-4 or IRAK-1-related disease" may be an autoimmune disease, an inflammatory disease or a tumor.

The pharmaceutical composition of the present invention may further include at least one pharmaceutically acceptable carrier, in addition to a compound represented by above chemical formula I, a compound of above table 1, a stereoisomer thereof, a pharmaceutically acceptable salt thereof, or a hydrate or solvate thereof.

In this case, the pharmaceutically acceptable carrier may be one commonly used in the art in the preparation, and may be at least one selected from lactose, dextrose, sucrose, sorbitol, mannitol, starch, acacia rubber, calcium phosphate, alginate, gelatin, calcium silicate, microcrystalline cellulose, polyvinyl pyrrolidone, cellulose, water, syrup, methyl cellulose, methyl hydroxybenzoate, propyl hydroxybenzoate, talc, magnesium stearate, mineral oil and the like, but is not limited thereto.

In addition, the pharmaceutical composition of the present invention may further include lubricant, humectant, a sweetening agent, a flavoring agent, emulsifier, a suspending agent, preservative, or the like in addition to the ingredients.

The present invention provide a method for preventing or treating IRAK-4- or IRAK-1-related diseases, comprising administering a compound represented by above chemical formula I, a compound of above table 1, a stereoisomer thereof, a pharmaceutically acceptable salt thereof, or a hydrate or solvate thereof, or a pharmaceutical composition comprising the same into a subject.

The method for preventing or treating IRAK-4- or IRAK-1-related diseases of the present invention may comprise administering a therapeutically effective amount of a compound represented by above chemical formula I, a compound of above table 1, a stereoisomer thereof, a pharmaceutically acceptable salt thereof, or a hydrate or solvate thereof, or a pharmaceutical composition comprising the same.

In the present invention, the "prevention" may mean all the acts, which inhibit or delay the occurrence of IRAK-4- or IRAK-1-related diseases by administering a compound represented by chemical formula I of the present invention, a compound of above table 1, a stereoisomer thereof, a pharmaceutically acceptable salt thereof, or a hydrate or solvate thereof.

In the present invention, the "treatment" may mean all the acts, by which a symptom of IRAK-4- or IRAK-1-related diseases gets better or takes a favorable turn by administering a compound represented by chemical formula I of the present invention, a compound of above table 1, a stereoisomer thereof, a pharmaceutically acceptable salt thereof, or a hydrate or solvate thereof.

The present invention provide a use of a compound represented by above chemical formula I, a compound of above table 1, a stereoisomer thereof, a pharmaceutically acceptable salt thereof, or a hydrate or solvate thereof, or a pharmaceutical composition comprising the same for preventing or treating IRAK-4- or IRAK-1-related diseases.

The present invention provide a use of a compound represented by above chemical formula I, a compound of above table 1, a stereoisomer thereof, a pharmaceutically acceptable salt thereof, or a hydrate or solvate thereof, or a pharmaceutical composition comprising the same for preparing a medicament for preventing or treating IRAK-4- or IRAK-1-related diseases.

### Advantageous Effects

A compound represented by chemical formula I of the present invention, a stereoisomer thereof, a pharmaceutically acceptable salt thereof, or a hydrate or solvate thereof can exhibit an inhibitory effect on IRAK-4 or IRAK-1, and can be advantageously used for preventing or treating IRAK-4 or IRAK-1 activity-related diseases.

### Mode for Invention

Hereinafter, the present invention will be described in more detail. All examples provided herein, or the use of an exemplary language, are merely intended to better illustrate the invention and do not limit the scope of the claimed invention.

### <Example>

A compound represented by chemical formula I of the present invention and a compound of above table 1 may be prepared through the method described below. Unless otherwise described, a starting material may be commercially available or may be prepared by known methods.

### Example 1. N-(2-(2-(dimethylamino)ethyl)-6-(thiophen-3-yl)-2H-indazol-5-yl)isophthalamide

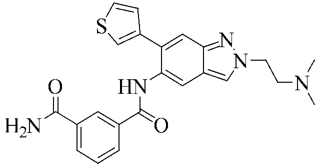

### [Step 1] Preparation of 2-(6-bromo-5-nitro-2H-indazol-2-yl)-N,N-dimethylethan-1-amine

Potassium carbonate (11.42 g, 82.64 mmole), potassium iodide (0.5 g, 3.01 mmole), 2-chloro-N,N-dimethylethan-1-amine hydrochloride (2.98 g, 20.66 mmole), and dimethylformamide (50 mL) were added to 6-bromo-5-nitro-2H-indazole (5 g, 20.66 mmole), and stirred at 80°C for 17 hours. After completion of a reaction, ethyl acetate (50 mL) was added to wash with purified water (50 mL), and anhydrous magnesium sulfate was added thereto and filtered. A resulting product was concentrated under reduced pressure and purified by MPLC (combiFlash NEXTGEN 300+) to obtain a title compound (0.88 g).
¹H-NMR (DMSO-d⁶) 2.12(s, 6H), 2.77(t, 2H), 4.55(t, 2H), 8.15(s, 1H), 8.61(s, 1H), 8.63(s, 1H)

### [Step 2] Preparation of N,N-dimethyl-2-(5-nitro-6-(thiophen-3-yl)-2H-indazol-2-yl)ethan-1-amine

Sodium carbonate (677 mg, 6.39 mmole), tetrakis(triphenylphosphine)palladium (74 mg, 0.06 mmole), thiophen-3-boronic acid pinacol ester (402 mg, 1.916 mmole), 1,4-dioxane (10 mL), and purified water (1 mL) were added to the compound (0.4 g, 1.277 mmole) obtained in above [Step 1], and stirred at 100°C for 17 hours. After completion of a reaction, a reaction solution was cooled to room temperature, and ethyl acetate (30 mL) was added to wash with purified water (30 mL), and anhydrous magnesium sulfate was added thereto and filtered. A resulting product was concentrated under reduced pressure and purified by MPLC (combiFlash NEXTGEN 300+) to obtain a title compound (0.46 g).
¹H-NMR (DMSO-d⁶) 2.12(s, 6H), 2.77(t, 2H), 4.55(t, 2H), 6.55(s, 1H), 7.71-7.72(m, 2H), 8.15(s, 1H), 8.61(s, 1H), 8.63(s, 1H)

### [Step 3] Preparation of 2-(2-(dimethylamino)ethyl)-6-(furan-3-yl)-2H-indazol-5-amine

Palladium on activated carbon (50 mg, 0.2 w/w) and methanol (20 mL) were added to the compound (250 mg, 0.79 mmole) obtained in above [Step 2], and hydrogenated at room temperature for three hours under a standard hydrogen pressure. After completion of a reaction, a reaction solution was filtered through celite and washed with ethyl acetate. A filtrate was concentrated under reduced pressure to obtain a title compound (230 mg).
¹H-NMR (DMSO-d⁶) 2.08(s, 6H), 2.61(t, 2H), 4.38(t, 2H), 4.50(s, 2H), 6.41(s, 1H), 7.63(s, 1H), 7.64(s, 1H), 7.66(s, 1H), 7.95(s, 1H), 7.99(s, 1H)

### [Step 4] Preparation of N-(2-(2-(dimethylamino)ethyl)-6-(thiophen-3-yl)-2H-indazol-5-yl)isophthalamide (Compound 1)

Dimethylformamide (1 mL) was added dropwise to the compound (104 mg, 0.363 mmole) obtained in above [Step 3], and DIPEA (0.247 mL, 1.452 mmole) and HATU (207 mg, 0.545 mmole) were added thereto and stirred for 30 minutes. After that, 3-carbamoylbenzoic acid (60 mg, 0.363 mmole) was added thereto and stirred at room temperature for 17 hours. After completion of a reaction, ethyl acetate (30 mL) was added to wash with purified water (30 mL), and anhydrous magnesium sulfate was added thereto and filtered. A resulting product was concentrated under reduced pressure and purified by MPLC (combiFlash NEXTGEN 300+) to obtain a title compound (70 mg).
¹H-NMR (DMSO-d⁶) 2.16(d, 6H), 2.77(t, 2H), 4.50(t, 2H), 7.29(m, 1H), 7.42(s, 1H), 7.49(m, 1H), 7.51(m, 1H), 7.58(s, 1H), 7.65(s, 1H), 7.75(s, 1H), 7.95(m, 1H), 7.98(m, 1H), 8.01(m, 1H), 9.33(s, 1H), 9.38(s, 1H), 9.89(s, 1H)
LC-MS (ESI, m/z) = 434.7 (M+H⁺)

### Example 2. N-(2-(3-hydroxy-3-methylbutyl)-6-morpholino-2H-indazol-5-yl)-5-nitronicotinamide

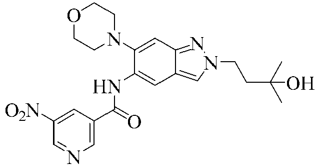

### [Step 1] Preparation of 4-(5-nitro-1H-indazol-6-yl)morpholine

Morpholine (21.6 mL, 247.92 mmole) was added to 6-bromo-5-nitro-1H-indazole (5 g, 20.66 mmole) and stirred at 140°C for 23 hours. After completion of a reaction, a reaction solution was concentrated under reduced pressure and purified by MPLC (combiFlash NEXTGEN 300+) to obtain a title compound (5.14 g).
¹H-NMR (DMSO-d⁶) 2.94(t, 4H), 3.68(t, 4H), 7.22(s 1H), 8.16(s, 1H), 8.35(s, 1H)

### [Step 2] Synthesis of 2-methyl-4-(6-morpholino-5-nitro-2H-indazol-2-yl)butan-2-ol

Under the same conditions as in [Step 1] of Example 1, 4-bromo-2-methylbutan-2-ol (1.89 mL, 15.5 mmole), potassium carbonate (5.7 g, 41.32 mmole), potassium iodide (257 mg, 1.55 mmole), and dimethylformamide (10 mL) were added to the compound (2.57 g, 10.33 mmole) obtained in above [Step 1] and stirred at 90°C for four hours. After completion of a reaction, ethyl acetate (50 mL) was added to wash with purified water (50 mL), and anhydrous magnesium sulfate was added thereto and filtered. A resulting product was concentrated under reduced pressure and purified by MPLC (combiFlash NEXTGEN 300+) to obtain a title compound (1.46 g).
¹H-NMR (DMSO-d⁶) 1.11(s, 6H), 1.99(m, 2H), 2.89(t, 4H), 3.66(t, 4H), 4.47(m, 2H), 4.48(s, 1H), 7.32(s, 1H), 8.31(s, 1H), 8.57(s, 1H)

### [Step 3] Preparation of 4-(5-amino-6-morpholino-2H-indazol-2-yl)-2-methylbutan-2-ol

Under the same conditions as in [Step 3] of Example 1, the compound (900 mg, 2.692 mmole) obtained in above [Step 2] was dissolved in ethyl acetate (9 mL) and hydrogenated by adding palladium on activated carbon (180 mg, 0.2 w/w). A reaction solution was filtered through celite and a filtrate was concentrated under reduced pressure to obtain a title compound (820 mg).
¹H-NMR (DMSO-d⁶) 1.11(s, 6H), 1.95(m, 2H), 2.75(t, 4H), 3.48(t, 4H), 4.35(m, 2H), 4.36(s, 1H), 4.50(s, 2H), 7.29(s, 1H), 7.75(s, 1H), 8.05(s, 1H)

### [Step 4] Preparation of N-(2-(3-hydroxy-3-methylbutyl)-6-morpholino-2H-indazol-5-yl)-5-nitronicotinamide (Compound 2)

Under the same conditions as in [Step 4] of Example 1, the compound (0.1 g, 0.3285 mmole) obtained in above [Step 3], 5-nitronicotinic acid (0.07 g, 0.299 mmole), dimethylformamide (5 mL), DIPEA (0.284 mL, 1.314 mmole), and HATU (0.24 g, 0.4928 mmole) were used and subjected to a reaction in the same manner, and then purified water was added thereto to filter and dry a resulting solid, and then ethyl acetate was added thereto and filtered to obtain a title compound (12 mg).
¹H-NMR (DMSO-d⁶) 1.12(s, 6H), 2.00(t, 2H), 2.89(m, 4H), 3.75(m, 4H), 4.41(m, 2H), 4.48(s, 1H), 7.36(s, 1H), 8.26(s, 1H), 8.32(s, 1H), 8.95(s, 1H), 9.44(s, 1H), 9.53(s, 1H), 10.11(s, 1H)
LC-MS (ESI, m/z) = 455.4 (M+H⁺)

### Example 3. N-(2-(3-hydroxy-3-methylbutyl)-6-morpholino-2H-indazol-5-yl)-3-nitrobenzamide

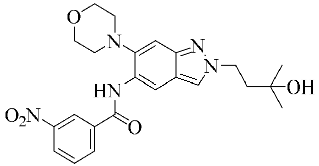

### [Step 4] Preparation of N-(2-(3-hydroxy-3-methylbutyl)-6-morpholino-2H-indazol-5-yl)-3-nitrobenzamide (Compound 3)

Under the same conditions as in [Step 4] of Example 1, the compound (0.1 g, 0.3285 mmole) obtained in [Step 3] of Example 2, 3-nitrobenzoic acid (0.07 g, 0.299 mmole), dimethylformamide (5 mL), HATU (0.24 g, 0.4928 mmole), and DIPEA (0.284 mL, 1.314 mmole) were used and subjected to a reaction in the same manner, and then purified water was added thereto to filter and dry a resulting solid, and then methanol was added thereto and filtered to obtain a title compound (53 mg).
¹H-NMR (DMSO-d⁶) 1.12(d, 6H), 2.00(t, 2H), 2.90(m, 4H), 3.78(m, 4H), 4.41(t, 2H), 4.47(s, 1H), 7.39(s, 1H), 7.86(m, 1H), 8.31(s, 1H), 8.35(m, 2H), 8.37(m, 1H), 8.70(s, 1H), 10.01(s, 1H)
LC-MS (ESI, m/z) = 454.4 (M+H⁺)

### Example 4. N-(6-(furan-3-yl)-2-(3-hydroxy-3-methylbutyl)-2H-indazol-5-yl)-3-nitrobenzamide

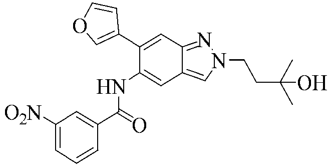

### [Step 1] Preparation of 4-(6-bromo-5-nitro-2H-indazol-2-yl)-2-methylbutan-2-ol

Under the same conditions as in [Step 1] of Example 1, 6-bromo-5-nitro-2H-indazole (3 g, 12.4 mmole), potassium carbonate (6.85 g, 49.6 mmole), potassium iodide (0.21 g, 1.81 mmole), 4-bromo-2-methylbutan-2-ol (2.27 mL, 18.6 mmole), and dimethylformamide (30 mL) were used and subjected to a reaction in the same manner, purified by MPLC (combiFlash NEXTGEN 300+), and concentrated to obtain a title compound (1.91 g).
¹H-NMR (DMSO-d⁶) 1.12(s, 6H), 2.02(m, 2H), 4.46-4.49(m, 3H), 8.10(s, 1H), 8.42(s, 1H), 8.49(s, 1H)

### [Step 2] Preparation of 4-(6-(furan-3-yl)-5-nitro-2H-indazol-2-yl)-2-methylbutan-2-ol

Under the same conditions as in [Step 2] of Example 1, the compound (1.9 g, 5.79 mmole) obtained in above [Step 1], sodium carbonate (3.07 g, 28.9 mmole), tetrakis(triphenylphosphine)palladium (0.33 g, 0.29 mmole), 4,4,5,5-tetramethyl-2-(furan-3-yl)-1,3,2-dioxaborolane (1.69 g, 8.69 mmole), 1,4-dioxane (20 mL) and purified water (2 mL) were used and subjected to a reaction in the same manner, purified by MPLC (combiFlash NEXTGEN 300+), and concentrated to obtain a title compound (1.5 g).
¹H-NMR (DMSO-d⁶) 1.12(s, 6H), 2.02(m, 2H), 4.46-4.49(m, 3H), 6.93(s, 1H), 7.66(s, 1H), 7.79(s, 1H), 8.11(s, 1H), 8.43(s, 1H), 8.50(s, 1H)

### [Step 3] Preparation of 4-(5-amino-6-(furan-3-yl)-2H-indazol-2-yl)-2-methylbutan-2-ol

Under the same conditions as in [Step 3] of Example 1, palladium on activated carbon (0.3 g, 0.2 w/w) was added to the compound (1.5 g, 4.76 mmole) obtained in above [Step 2] and hydrogenated. A reaction mixture was filtered through celite and a filtrate was concentrated to obtain a title compound (1.06 g).
¹H-NMR (DMSO-d⁶) 1.09(s, 6H), 1.94(m, 2H), 4.32-4.35(m, 3H), 4.52(s, 2H), 6.72(s, 1H), 7.46(s, 1H), 7.58(s, 1H), 7.91(s, 1H), 8.22(s, 1H), 8.33(s, 1H)

### [Step 4] Preparation of N-(6-(furan-3-yl)-2-(3-hydroxy-3-methylbutyl)-2H-indazol-5-yl)-3-nitrobenzamide (Compound 4)

Under the same conditions as in [Step 4] of Example 1, the compound (87 mg, 0.305 mmole) obtained in above [Step 3], 3-nitrobenzoic acid (42 mg, 0.254 mmole), dimethylformamide (1 mL), HATU (145 mg, 0.381 mmole), and DIPEA (0.173 mL, 0.016 mmole) were used and subjected to a reaction in the same manner, purified by MPLC (combiFlash NEXTGEN 300+), and concentrated to obtain a title compound (15 mg).
¹H-NMR (DMSO-d⁶) 1.12(d, 6H), 2.04(t, 2H), 4.49(m, 3H), 6.79(s, 1H), 7.65(s, 1H), 7.71(m, 1H), 7.74(s, 1H), 7.81(t, 1H), 7.84(s, 1H), 8.34(d, 1H), 8.40(s, 2H), 8.73(s, 1H), 10.28(s, 1H)
LC-MS (ESI, m/z) = 435.4 (M+H⁺)

### Example 5. N-(6-(furan-3-yl)-2-(3-hydroxy-3-methylbutyl)-2H-indazol-5-yl)-5-nitronicotinamide

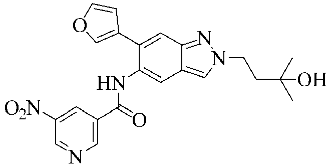

### [Step 4] Preparation of N-(6-(furan-3-yl)-2-(3-hydroxy-3-methylbutyl)-2H-indazol-5-yl)-5-nitronicotinamide (Compound 5)

Under the same conditions as in [Step 4] of Example 1, the compound (87 mg, 0.305 mmole) obtained in [Step 3] of Example 4, 5-nitronicotinic acid (43 mg, 0.254 mmole), dimethylformamide (1 mL), HATU (145 mg, 0.381 mmole), and DIPEA (0.173 mL, 1.016 mmole) were used and subjected to a reaction in the same manner, purified by MPLC (combiFlash NEXTGEN 300+), and concentrated to obtain a title compound (15 mg).
¹H-NMR (DMSO-d⁶) 1.12(s, 6H), 2.02(t, 2H), 4.48(m, 3H), 6.81(m, 1H), 7.66(m, 1H), 7.71(m, 1H), 7.77(m, 1H), 7.88(m, 1H), 8.41(s, 1H), 8.97(s, 1H), 9.41(s, 1H), 9.50(s, 1H), 10.43(s, 1H)
LC-MS (ESI, m/z) = 436.4 (M+H⁺)

### Example 6. N-(6-(furan-3-yl)-2-(3-hydroxy-3-methylbutyl)-2H-indazol-5-yl)isophthalamide

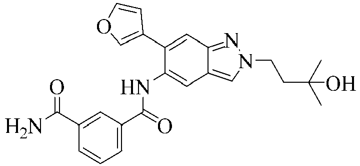

### [Step 4] Preparation of N-(6-(furan-3-yl)-2-(3-hydroxy-3-methylbutyl)-2H-indazol-5-yl)isophthalamide (Compound 6)

Under the same conditions as in [Step 4] of Example 1, the compound (87 mg, 0.305 mmole) obtained in [Step 3] of Example 4, 3-carbamoylbenzoic acid (42 mg, 0.254 mmole), dimethylformamide (1 mL), HATU (145 mg, 0.381 mmole), and DIPEA (0.173 mL, 1.016 mmole) were used and subjected to a reaction in the same manner, purified by MPLC (combiFlash NEXTGEN 300+), and concentrated to obtain a title compound (29 mg).
¹H-NMR (DMSO-d⁶) 1.12(s, 6H), 2.02(m, 2H), 4.48(m, 3H), 6.80(s, 1H), 7.44(m, 1H), 7.56(m, 1H), 7.64(s, 1H), 7.70(m, 2H), 7.83(s, 1H), 8.02(m, 3H), 8.39(m, 2H), 9.97(s, 1H)
LC-MS (ESI, m/z) = 433.5 (M+H⁺)

### Example 7. N-(2-(3-hydroxy-3-methylbutyl)-6-(thiophen-3-yl)-2H-indazol-5-yl)-S-nitronicotinamide

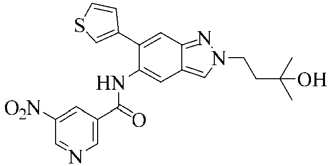

### [Step 2] Preparation of 2-methyl-4-(5-nitro-6-(thiophen-3-yl)-2H-indazol-2-yl)butan-2-ol

Under the same conditions as in [Step 2] of Example 1, the compound (0.9 g, 2.74 mmole) obtained in [Step 1] of Example 4, sodium carbonate (1.45 g, 13.7 mmole), tetrakis(triphenylphosphine)palladium (0.16 g, 0.14 mmole), 4,4,5,5-tetramethyl-2-(thiophen-3-yl)-1,3,2-dioxaborolane (0.86 g, 4.11 mmole), 1,4-dioxane (10 mL) and purified water (1 mL) were used and subjected to a reaction in the same manner, purified by MPLC (combiFlash NEXTGEN 300+), and concentrated to obtain a title compound (0.44 g).
¹H-NMR (DMSO-d⁶) 1.12(s, 6H), 2.04(t, 2H), 4.50(s, 1H), 4.53(m, 2H), 7.08(s, 1H), 7.58(s, 2H), 8.13(s, 1H), 8.59(s, 1H), 8.75(s, 1H)

### [Step 3] Preparation of 4-(5-amino-6-(thiophen-3-yl)-2H-indazol-2-yl)-2-methylbutan-2-ol

Under the same conditions as in [Step 3] of Example 1, palladium on activated carbon (0.08 g, 0.2 w/w) was added to the compound (0.4 g, 1.22 mmole) obtained in above [Step 2] and hydrogenated. A reaction mixture was filtered through celite and a filtrate was concentrated to obtain a title compound (0.33 g).
¹H-NMR (DMSO-d⁶) 1.09(s, 6H), 1.85(t, 2H), 3.88(s, 2H), 4.40(s, 1H), 4.50(m, 2H), 6.94(s, 1H), 7.34(m, 2H), 7.66(m, 2H), 7.70(s, 1H)

### [Step 4] Preparation of N-(2-(3-hydroxy-3-methylbutyl)-6-(thiophen-3-yl)-2H-indazol-5-yl)-5-nitronicotinamide (Compound 7)

Under the same conditions as in [Step 4] of Example 1, the compound (0.113 g, 0.375 mmole) obtained in above [Step 3], 5-nitronicotinic acid (52.5 mg, 0.3125 mmole), dimethylformamide (1 mL), HATU (0.178 g, 0.469 mmole) and DIPEA (0.213 mL, 1.25 mmole) were used and subjected to a reaction in the same manner, purified by MPLC (combiFlash NEXTGEN 300+), and concentrated to obtain a title compound (47 mg).
¹H-NMR (DMSO-d⁶) 1.13(s, 6H), 2.03(t, 2H), 4.50(m, 3H), 7.30(m, 1H), 7.52(m, 1H), 7.60(s, 1H), 7.67(s, 1H), 7.80(s, 1H), 8.43(s, 1H), 8.87(s, 1H), 9.30(s, 1H), 9.48(s, 1H), 10.38(s, 1H)
LC-MS (ESI, m/z) = 452.5 (M+H⁺)

### Example 8. N-(2-(3-hydroxy-3-methylbutyl)-6-(thiophen-3-yl)-2H-indazol-5-yl)-3-nitrobenzamide

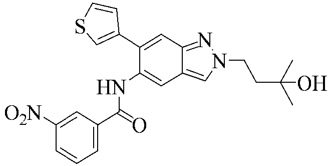

### [Step 4] Preparation of N-(2-(3-hydroxy-3-methylbutyl)-6-(thiophen-3-yl)-2H-indazol-5-yl)-3-nitrobenzamide (Compound 8)

Under the same conditions as in [Step 4] of Example 1, the compound (0.113 g, 0.375 mmole) obtained in [Step 3] of Example 7, 3-nitrobenzoic acid (52 mg, 0.3125 mmole), dimethylformamide (1 mL), HATU (0.178 g, 0.469 mmole) and DIPEA (0.213 mL, 1.25 mmole) were used and subjected to a reaction in the same manner, purified by MPLC (combiFlash NEXTGEN 300+), and concentrated to obtain a title compound (47 mg).
¹H-NMR (DMSO-d⁶) 1.23(s, 6H), 2.02(t, 2H), 4.49(m, 3H), 7.38(m, 1H), 7.64(m, 2H), 7.80(m, 2H), 8.02(m, 3H), 8.74(m, 2H), 10.20(s, 1H)
LC-MS (ESI, m/z) = 451.5 (M+H⁺)

### Example 9. N-(6-morpholino-2-(2-(piperidin-1-yl)ethyl)-2H-indazol-5-yl)-3-nitrobenzamide

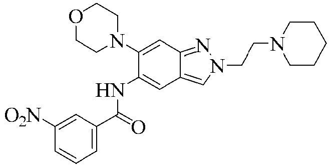

### [Step 1] Preparation of 6-bromo-5-nitro-2-(2-(piperidin-1-yl)ethyl)-2H-indazole

Under the same conditions as in [Step 1] of Example 1, 6-bromo-5-nitro-2H-indazole (5 g, 20.66 mmole), potassium carbonate (17.3 g, 125.18 mmole), potassium iodide (0.5 g, 3.012 mmole), 1-(2-chloroethyl)piperidine (5.7 g, 31 mmole), and dimethylformamide (50 mL) were used and subjected to a reaction in the same manner, purified by MPLC (combiFlash NEXTGEN 300+), and concentrated to obtain a title compound (1.83 g).
¹H-NMR (DMSO-d⁶) 1.35(m, 2H), 1.61(m, 4H), 1.78(m, 2H), 3.47(m, 2H), 3.66(m, 2H), 4.80(m, 2H), 7.25(s, 1H), 8.29(s, 1H), 8.42(s, 1H)

### [Step 2] Preparation of 4-(5-nitro-2-(2-(piperidin-1-yl)ethyl)-2H-indazol-6-yl)morpholine

Under the same conditions as in [Step 1] of Example 2, morpholine (5 mL) was added to the compound (100 mg, 0.283 mmole) obtained in above [Step 1] and subjected to a reaction in the same manner, and then purified by MPLC (combiFlash NEXTGEN 300+) to obtain a title compound (30 mg).
¹H-NMR (DMSO-d⁶) 1.35(m, 2H), 1.61(m, 4H), 1.78(m, 2H), 2.91(m, 4H), 3.47(m, 2H), 3.66(m, 2H), 3.79(m, 4H), 4.80(m, 2H), 7.87(m, 1H), 8.56(m, 1H), 8.71(m, 1H)

### [Step 3] Preparation of 6-morpholino-2-(2-(piperidin-1-yl)ethyl)-2H-indazol-5-amine

Under the same conditions as in [Step 3] of Example 1, the compound (30 mg, 0.091 mmole) obtained in above [Step 2] was dissolved in ethyl acetate (5 mL) and hydrogenated by adding palladium on activated carbon (3 mg, 0.03 w/w). A reaction solution was filtered through celite and a filtrate was concentrated under reduced pressure to obtain a title compound (27 mg).
¹H-NMR (DMSO-d⁶) 1.35(m, 2H), 1.61(m, 4H), 1.78(m, 2H), 2.81(m, 4H), 3.37(m, 2H), 3.46(m, 2H), 3.59(m, 4H), 4.49(m, 2H), 4.51(s, 2H), 7.57(m, 1H), 8.36(m, 1H), 8.51(m, 1H)

### [Step 4] Preparation of N-(6-morpholino-2-(2-(piperidin-1-yl)ethyl)-2H-indazol-5-yl)-3-nitrobenzamide (Compound 9)

Under the same conditions as in [Step 4] of Example 1, the compound (27 mg, 0.082 mmole) obtained in above [Step 3], 3-nitrobenzoic acid (13 mg, 0.09 mmole), dimethylformamide (0.06 mL), HATU (44 mg, 0.123 mmole), and DIPEA (0.06 mL, 0.328 mmole) were used and subjected to a reaction in the same manner, purified by MPLC (combiFlash NEXTGEN 300+), and concentrated to obtain a title compound (8 mg).
¹H-NMR (DMSO-d⁶) 1.36(m, 2H), 1.61(m, 4H), 1.79(m, 2H), 2.91(m, 4H), 3.47(m, 2H), 3.66(m, 2H), 3.79(m, 4H), 4.80(m, 2H), 7.41(m, 1H), 7.87(m, 1H), 8.40(m, 3H), 8.71(m, 1H), 9.08(m, 1H), 10.03(s, 1H)
LC-MS (ESI, m/z) = 479.5 (M+H⁺)

### Example 10. N-(2-(2-(dimethylamino)ethyl)-6-morpholino-2H-indazol-5-yl)-3-nitrobenzamide

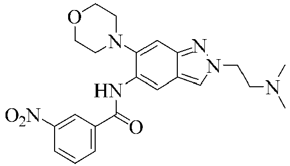

### [Step 2] Preparation of N,N-dimethyl-2-(6-morpholino-5-nitro-2H-indazol-2-yl)ethan-1-amine

Under the same conditions as in [Step 1] of Example 2, morpholine (5 mL) was added to the compound (500 mg, 1.6 mmole) obtained in [Step 1] of Example 1 and subjected to a reaction in the same manner, and then purified by MPLC (combiFlash NEXTGEN 300+) to obtain a title compound (135 mg).
¹H-NMR (DMSO-d⁶) 2.78(s, 6H), 2.92(m, 4H), 3.63(m, 2H), 3.78(m, 4H), 4.77(m, 2H), 7.32(s, 1H), 8.34(s, 1H), 8.59(s, 1H)

### [Step 3] Preparation of 2-(2-(dimethylamino)ethyl)-6-morpholino-2H-indazol-5-amine

Under the same conditions as in [Step 3] of Example 1, the compound (135 mg, 0.467 mmole) obtained in above [Step 2] was dissolved in ethyl acetate (5 mL) and hydrogenated by adding palladium on activated carbon (14 mg, 0.03 w/w). A reaction solution was filtered through celite and a filtrate was concentrated under reduced pressure to obtain a title compound (122 mg).
¹H-NMR (DMSO-d⁶) 2.69(s, 6H), 2.85(m, 4H), 3.59(m, 2H), 3.70(m, 4H), 4.47(m, 2H), 4.51(s, 2H), 7.30(s, 1H), 7.75(s, 1H), 8.05(s, 1H)

### [Step 4] Preparation of N-(2-(2-(dimethylamino)ethyl)-6-morpholino-2H-indazol-5-yl)-3-nitrobenzamide (Compound 10)

Under the same conditions as in [Step 4] of Example 1, the compound (61 mg, 0.21 mmole) obtained in above [Step 3], 3-nitrobenzoic acid (32 mg, 0.232 mmole), dimethylformamide (5 mL), HATU (110 mg, 0.316 mmole), and DIPEA (0.13 mL, 0.843 mmole) were used and subjected to a reaction in the same manner, purified by MPLC (combiFlash NEXTGEN 300+), and concentrated to obtain a title compound (8 mg).
¹H-NMR (DMSO-d⁶) 2.75(s, 6H), 2.90(m, 4H), 3.62(m, 2H), 3.78(m, 4H), 4.77(m, 2H), 7.40(m, 1H), 7.86(m, 1H), 8.38(m, 3H), 8.70(m, 1H), 9.23(m, 1H), 10.02(s, 1H)
LC-MS (ESI, m/z) = 439.5 (M+H⁺)

### Example 11. N-(2-(2-methoxyethyl)-6-morpholino-2H-indazol-5-yl)-3-nitrobenzamide

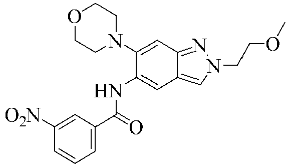

### [Step 1] Preparation of 6-bromo-2-(2-methoxyethyl)-5-nitro-2H-indazole

Under the same conditions as in [Step 1] of Example 1, 6-bromo-5-nitro-2H-indazole (5 g, 20.66 mmole), potassium carbonate (11.42 g, 82.64 mmole), potassium iodide (0.5 g, 3.012 mmole), 2-chloroethyl methyl ester (5.7 g, 31 mmole), and dimethylformamide (50 mL) were used and subjected to a reaction in the same manner, purified by MPLC (combiFlash NEXTGEN 300+), and concentrated to obtain a title compound (2.09 g).
¹H-NMR (DMSO-d⁶) 3.13(s, 3H), 3.75(m, 2H), 4.51(m, 2H), 7.35(s, 1H), 8.30(s, 1H), 8.42(s, 1H)

### [Step 2] Preparation of 4-(2-(2-methoxyethyl)-5-nitro-2H-indazol-6-yl)morpholine

Under the same conditions as in [Step 1] of Example 2, morpholine (10 mL) was added to the compound (800 mg, 2.67 mmole) obtained in above [Step 1] and subjected to a reaction in the same manner, and then purified by MPLC (combiFlash NEXTGEN 300+) to obtain a title compound (180 mg).
¹H-NMR (DMSO-d⁶) 2.81(m, 4H), 3.11(s, 3H), 3.59(m, 4H), 3.72(m, 2H), 4.51(m, 2H), 7.37(s, 1H), 8.32(s, 1H), 8.42(s, 1H)

### [Step 3] Preparation of 2-(2-methoxyethyl)-6-morpholino-2H-indazol-5-amine

Under the same conditions as in [Step 3] of Example 1, the compound (180 mg, 0.588 mmole) obtained in above [Step 2] was dissolved in ethyl acetate (20 mL) and hydrogenated by adding palladium on activated carbon (36 mg, 0.2 w/w). A reaction solution was filtered through celite and a filtrate was concentrated under reduced pressure to obtain a title compound (162 mg).
¹H-NMR (DMSO-d⁶) 2.90(m, 4H), 3.20(s, 3H), 3.78(m, 6H), 4.51(m, 2H), 7.17(s, 1H), 8.12(s, 1H), 8.20(s, 1H)

### [Step 4] Preparation of N-(2-(2-methoxyethyl)-6-morpholino-2H-indazol-5-yl)-3-nitrobenzamide (Compound 11)

3-nitrobenzoic acid (90 mg, 0.539 mmole), dimethylformamide (5 mL), EDC·HCl (310 mg, 1.572 mmole), and HOBt·H₂O (218 mg, 1.613 mmole) were added to the compound (0.162 g, 0.586 mmole) obtained in above [Step 3] and stirred at room temperature for 17 hours. After completion of a reaction, ethyl acetate (40 mL) was added thereto and washed with purified water (40 mL). Anhydrous magnesium sulfate was added, filtered, concentrated under reduced pressure, and purified by MPLC (combiFlash NEXTGEN 300+) to obtain a title compound (54 mg).
¹H-NMR (DMSO-d⁶) 2.90(m, 4H), 3.20(s, 3H), 3.78(m, 6H), 4.51(m, 2H), 7.40(s, 1H), 7.87(m, 1H), 8.28(s, 1H), 8.35(m, 2H), 8.43(m, 1H), 8.70(m, 1H), 10.01(s, 1H)
LC-MS (ESI, m/z) = 426.4 (M+H⁺)

### Example 12. N-(2-(2-(dimethylamino)ethyl)-6-(thiophen-3-yl)-2H-indazol-5-yl)-3-(thiazol-2-vl)benzamide

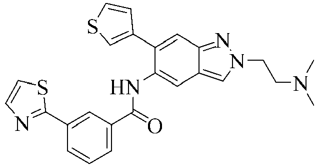

### [Step 4] Preparation of N-(2-(2-(dimethylamino)ethyl)-6-(thiophen-3-yl)-2H-indazol-5-yl)-3-(thiazol-2-yl)benzamide (Compound 12)

Under the same conditions as in [Step 4] of Example 1, the compound (104 mg, 0.363 mmole) obtained in [Step 3] of Example 1, 3-(1,3-thiazol-2-yl)benzoic acid (70 mg, 0.363 mmole), dimethylformamide (1 mL), HATU (207 mg, 0.545 mmole) and DIPEA (0.247 mL, 1.452 mmole) were used and subjected to a reaction in the same manner, purified by MPLC (combiFlash NEXTGEN 300+), and concentrated to obtain a title compound (89 mg).
¹H-NMR (DMSO-d⁶) 2.26(s, 6H), 2.92(m, 2H), 4.56(m, 2H), 7.32(m, 1H), 7.53(m, 1H), 7.61(m, 2H), 7.66(m, 1H), 7.82(m, 2H), 7.90(m, 1H), 7.94(m, 1H), 8.09(m, 1H), 8.35(s, 1H), 8.41(s, 1H), 10.03(s, 1H)
LC-MS (ESI, m/z) = 474.5 (M+H⁺)

### Example 13. N-(2-(2-(methylsulfonyl)ethyl)-6-morpholino-2H-indazol-5-yl)-3-nitrobenzamide

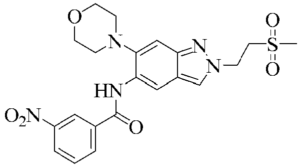

### [Step 1] Preparation of 6-bromo-2-(2-(methylsulfonyl)ethyl)-5-nitro-2H-indazole

Under the same conditions as in [Step 1] of Example 1, 2-bromoethyl methyl sulfone (5.8 g, 31 mmole), potassium carbonate (5.7 g, 41.32 mmole), potassium iodide (257 mg, 1.55 mmole), and dimethylformamide (50 mL) were added to 6-bromo-5-nitro-2H-indazole (5 g, 20.66 mmole), subjected to a reaction in the same manner, and then purified by MPLC (combiFlash NEXTGEN 300+) to obtain a title compound (1.5 g).
¹H-NMR (DMSO-d⁶) 2.90(s, 3H), 3.73(t, 2H), 4.76(t, 2H), 7.70(s, 1H), 8.42(s, 1H), 8.56(s, 1H)

### [Step 2] Preparation of 4-(2-(2-(methylsulfonyl)ethyl)-5-nitro-2H-indazol-6-yl)morpholine

Under the same conditions as in [Step 1] of Example 2, morpholine (10 mL) was added to the compound (1.5 g, 4.31 mmole) obtained in above [Step 1] and subjected to a reaction in the same manner, and then purified by MPLC (combiFlash NEXTGEN 300+) to obtain a title compound (594 mg).
¹H-NMR (DMSO-d⁶) 2.86(s, 3H), 2.94(m, 4H), 3.59(m, 4H), 3.76(t, 2H), 4.78(t, 2H), 7.72(s, 1H), 8.31(s, 1H), 8.57(s, 1H)

### [Step 3] Preparation of 2-(2-(methylsulfonyl)ethyl)-6-morpholino-2H-indazol-5-amine

Under the same conditions as in [Step 3] of Example 1, the compound (594 mg, 1.83 mmole) obtained in above [Step 2] was dissolved in ethyl acetate (100 mL) and hydrogenated by adding palladium on activated carbon (109 mg, 0.2 w/w). A reaction solution was filtered through celite and a filtrate was concentrated under reduced pressure to obtain a title compound (544 mg).
¹H-NMR (DMSO-d⁶) 2.82(s, 3H), 2.84(m, 4H), 3.43(m, 4H), 3.63(t, 2H), 4.30(s, 2H), 4.60(t, 2H), 7.52(s, 1H), 8.11(s, 1H), 8.27(s, 1H)

### [Step 4] Preparation of N-(2-(2-(methylsulfonyl)ethyl)-6-morpholino-2H-indazol-5-yl)-3-nitrobenzamide (Compound 13)

Under the same conditions as in [Step 4] of Example 11, the compound (136 mg, 0.42 mmole) obtained in above [Step 3], 3-nitrobenzoic acid (64 mg, 0.383 mmole), dimethylformamide (10 mL), EDC·HCl(0.22 g, 1.15 mmole) and HOBt·H₂O (0.155 g, 1.15 mmole) were used and subjected to a reaction in the same manner, purified by MPLC (combiFlash NEXTGEN 300+), and concentrated to obtain a title compound (44 mg).
¹H-NMR (DMSO-d⁶) 2.87(s, 3H), 2.94(m, 4H), 3.59(m, 4H), 3.75(t, 2H), 4.78(t, 2H), 6.99(d, 1H), 7.63(d, 1H), 7.71(m, 1H), 8.40(m, 3H), 8.78(s, 1H), 10.14(s, 1H)
LC-MS (ESI, m/z) = 474.5 (M+H⁺)

### Example 14. N-(2-(2-(methylsulfonyl)ethyl)-6-morpholino-2H-indazol-5-yl)-3-(morpholinosulfonyl)benzamide

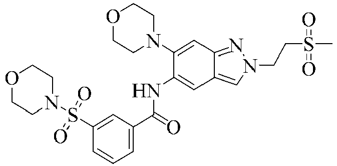

### [Step 4] Preparation of N-(2-(2-(methylsulfonyl)ethyl)-6-morpholino-2H-indazol-5-yl)-3-(morpholinosulfonyl)benzamide (Compound 14)

Under the same conditions as in [Step 4] of Example 1, the compound (136 mg, 0.42 mmole) obtained in [Step 3] of Example 13, 3-(morpholinosulfonyl)benzoic acid (136 mg, 0.462 mmole), dimethylformamide (10 mL), HATU (217 mg, 0.63 mmole), and DIPEA (0.2 mL, 1.68 mmole) were used and subjected to a reaction in the same manner, purified by MPLC (combiFlash NEXTGEN 300+), and concentrated to obtain a title compound (26 mg).
¹H-NMR (DMSO-d⁶) 2.87(s, 3H), 2.89(m, 8H), 3.62(m, 4H), 3.78(m, 4H), 3.82(m, 2H), 4.80(m, 2H), 7.41(1H), 7.87(m, 1H), 7.94(m, 1H), 8.22(m, 1H), 8.26(m, 1H), 8.40(m, 2H), 9.97(s, 1H)
LC-MS (ESI, m/z) = 578.7 (M+H⁺)

### Example 15. N-(2-(3-hydroxy-3-methylbutyl)-6-morpholino-2H-indazol-5-yl)-4-sulfamoylbenzamide

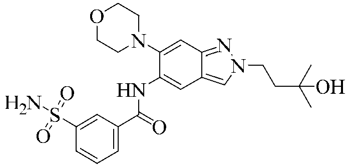

### [Step 4] Preparation of N-(2-(3-hydroxy-3-methylbutyl)-6-morpholino-2H-indazol-5-yl)-4-sulfamoylbenzamide (Compound 15)

Under the same conditions as in [Step 4] of Example 11, 3-sulfamoylbenzoic acid (110 mg, 0.547 mmole), dimethylformamide (5 mL), EDC·HCl(315 mg, 1.641 mmole) and HOBt·H₂O (222 mg, 1.642 mmole) were added to the compound (0.199 g, 0.42 mmole) obtained in [Step 3] of Example 2, subjected to a reaction in the same manner, purified by MPLC (combiFlash NEXTGEN 300+), and concentrated to obtain a title compound (106 mg).
¹H-NMR (DMSO-d⁶) 1.12(d, 6H), 2.00(t, 2H), 2.88(m, 4H), 3.77(m, 4H), 4.41(m, 2H), 4.47(s, 1H), 7.42(s, 1H), 7.48(s, 2H), 7.78(t, 1H), 8.02(d, 1H), 8.12(d, 1H), 8.31(s, 1H), 8.37(s, 1H), 8.41(s, 1H), 9.88(s, 1H)
LC-MS (ESI, m/z) = 488.6 (M+H⁺)

### Example 16. N-(7-(furan-3-yl)-2-(3-hydroxy-3-methylbutyl)-2H-indazol-5-yl)-3-nitrobenzamide

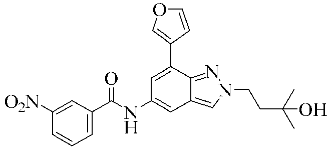

### [Step 1] Preparation of 4-(7-bromo-5-nitro-2H-indazol-2-yl)-2-methylbutan-2-ol

Under the same conditions as in [Step 1] of Example 1, 7-bromo-5-nitro-1H-indazole (2 g, 8.26 mmole), dimethylformamide (20 mL), potassium carbonate (4.56 g, 27.30 mmole), potassium iodide (0.14 g, 0.83 mmole), and 4-bromo-2-methylbutan-2-ol were used and subjected to a reaction in the same manner, purified by MPLC (combiFlash NEXTGEN 300+), and concentrated to obtain a title compound (0.5 g).
¹H-NMR (DMSO-d⁶) 1.14(s, 6H), 2.07(t, 2H), 4.49(s, 1H), 4.51(t, 2H), 8.21(s, 1H), 8.42(s, 1H), 8.51(s, 1H)

### [Step 2] Preparation of 4-(7-(furan-3-yl)-5-nitro-2H-indazol-2-yl)-2-methylbutan-2-ol

Under the same conditions as in [Step 2] of Example 1, the compound (0.5 g, 1.52 mmole) obtained in above [Step 1], sodium carbonate (0.81 g, 7.62 mmole), tetrakis(triphenylphosphine)palladium (0.09 g, 0.08 mmole), and 2-(furan-3-yl)-4,4,5,5-tetramethyl-1,3,2-dioxaborolane (0.44 g, 2.29 mmole) were used and subjected to a reaction in the same manner, purified by MPLC (combiFlash NEXTGEN 300+), and concentrated to obtain a title compound (0.5 g).
¹H-NMR (DMSO-d⁶) 1.14(s, 6H), 2.07(t, 2H), 4.49(s, 1H), 4.51(t, 2H), 7.21(d, 1H), 8.06(d, 1H), 8.21(s, 1H), 8.42(s, 1H), 8.51(s, 1H), 8.58(s, 1H)

### [Step 3] Preparation of 4-(5-amino-7-(furan-3-yl)-2H-indazol-2-yl)-2-methylbutan-2-ol

Under the same conditions as in [Step 3] of Example 1, palladium on activated carbon (0.10 g, 0.2 w/w) and ethyl acetate (10 mL) were added to the compound (0.5 g, 1.59 mmole) obtained in above [Step 2] and hydrogenated. A reaction mixture was filtered through celite and a filtrate was concentrated to obtain a title compound (0.44 g).
¹H-NMR (DMSO-d⁶) 1.14(s, 6H), 2.07(t, 2H), 4.49(s, 1H), 4.51(t, 2H), 7.11(s, 2H), 7.21(d, 1H), 8.06(d, 1H), 8.21(s, 1H), 8.42(s, 1H), 8.51(s, 1H), 8.58(s, 1H)

### [Step 4] Preparation of N-(7-(furan-3-yl)-2-(3-hydroxy-3-methylbutyl)-2H-indazol-5-yl)-3-nitrobenzamide (Compound 16)

Under the same conditions as in [Step 4] of Example 1, the compound (0.173 g, 0.61 mmole) obtained in above [Step 3], 3-nitrobenzoic acid (0.085 g, 0.51 mmole), HATU (0.777 g, 2.04 mmole) and DIPEA (0.35 mL, 2.04 mmole) were used and subjected to a reaction in the same manner, purified by MPLC (combiFlash NEXTGEN 300+), and concentrated to obtain a title compound (85 mg).
¹H-NMR (DMSO-d⁶) 1.11(s, 6H), 1.42(m, 2H), 1.81(d, 2H), 2.02(t, 2H), 2.32(m, 1H), 2.99(t, 2H), 3.77(d, 2H), 4.43(t, 2H), 4.47(s, 1H), 6.82(s, 1H), 7.48(s, 1H), 7.57(s, 1H), 7.85(s, 1H), 7.99(s, 1H), 8.36(s, 1H), 8.56(s, 1H), 9.63(s, 1H)
LC-MS (ESI, m/z) = 435.4 (M+H⁺)

### Example 17. N-(2-(2-(2-amino-2-oxoethoxy)ethyl)-6-morpholino-2H-indazol-5-yl)-3-sulfamoylbenzamide

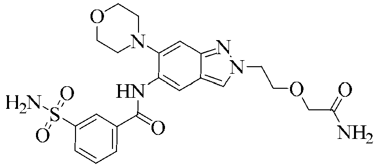

### [Step 2] Preparation of 2-(2-(6-morpholino-5-nitro-2H-indazol-2-yl)ethoxy)acetamide

Under the same conditions as in [Step 1] of Example 1, 2-(2-chloroethoxy)acetamide (0.83 g, 6.05 mmole), potassium carbonate (2.23 g, 16.12 mmole), potassium iodide (100 mg, 0.6 mmole), and dimethylformamide (20 mL) were added to the compound (1 g, 4.03 mmole) obtained in [Step 1] of Example 2, subjected to a reaction in the same manner, purified by MPLC (combiFlash NEXTGEN 300+), and concentrated to obtain a title compound (430 mg).
¹H-NMR (DMSO-d⁶) 2.93(m, 4H), 3.75(m, 6H), 3.93(t, 2H), 4.56(t, 2H), 7.32(s, 1H), 8.31(s, 1H), 8.56(s, 1H)

### [Step 3] Preparation of 2-(2-(5-amino-6-morpholino-2H-indazol-2-yl)ethoxy)acetamide

Under the same conditions as in [Step 3] of Example 1, the compound (430 mg, 1.23 mmole) obtained in above [Step 2] was dissolved in ethyl acetate (10 mL) and hydrogenated by adding palladium on activated carbon (65 mg, 0.15 w/w). A reaction solution was filtered through celite and a filtrate was concentrated under reduced pressure to obtain a title compound (393 mg).
¹H-NMR (DMSO-d⁶) 2.86(m, 4H), 3.63(m, 6H), 3.79(t, 2H), 4.43(t, 2H), 4.49(s, 2H), 7.29(s, 1H), 7.63(s, 1H), 8.05(s, 1H)

### [Step 4] Preparation of N-(2-(2-(2-amino-2-oxoethoxy)ethyl)-6-morpholino-2H-indazol-5-yl)-3-sulfamoylbenzamide (Compound 17)

Under the same conditions as in [Step 4] of Example 11, the compound (98 mg, 0.31 mmole) obtained in above [Step 3], 3-sulfamoylbenzoic acid (94 mg, 0.465 mmole), dimethylformamide (5 mL), EDC·HCl(178 mg, 0.93 mmole) and HOBt·H₂O (126 mg, 0.93 mmole) were used and subjected to a reaction in the same manner, purified by MPLC (combiFlash NEXTGEN 300+), and concentrated to obtain a title compound (67 mg).
¹H-NMR (DMSO-d⁶) 2.91(m, 4H), 3.76-3.78(m, 6H), 3.93(t, 2H), 4.56(t, 2H), 7.19-7.23(m, 2H), 7.42(s, 1H), 7.48(s, 2H), 7.78(t, 1H), 8.02(d, 1H), 8.14(d, 1H), 8.36(s, 1H), 8.37(s, 1H), 8.43(s, 1H), 9.89(s, 1H)
LC-MS (ESI, m/z) = 503.5 (M+H⁺)

### Example 18. N-(2-(2-(2-amino-2-oxoethoxy)ethyl)-6-morpholino-2H-indazol-5-yl)isophthalamide

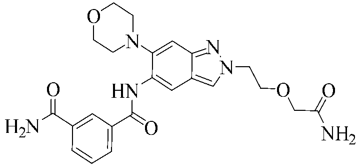

### [Step 4] Preparation of N-(2-(2-(2-amino-2-oxoethoxy)ethyl)-6-morpholino-2H-indazol-5-yl)isophthalamide (Compound 18)

Under the same conditions as in [Step 4] of Example 1, the compound (150 mg, 0.47 mmole) obtained in [Step 3] of Example 17, 3-carbamoylbenzoic acid (65 mg, 0.39 mmole), dimethylformamide (10 mL), HATU (0.268 g, 0.71 mmole) and DIPEA (0.27 mL, 1.56 mmole) were used and subjected to a reaction in the same manner, purified by MPLC (combiFlash NEXTGEN 300+), and concentrated to obtain a title compound (29 mg).
¹H-NMR (DMSO-d⁶) 2.90(br. s, 4H), 3.76(s, 2H), 3.78(br. s, 4H), 3.91(t, 2H), 4.56(t, 2H), 7.20(d, 2H), 7.40(s, 1H), 7.87(t, 1H), 8.19(d, 2H), 8.36(s, 3H), 8.42(d, 1H), 8.71(s, 1H), 10.00(s, 1H)
LC-MS (ESI, m/z) = 467.5 (M+H⁺)

### Example 19. N-(6-morpholino-2-(2-morpholinoethyl)-2H-indazol-5-yl)-3-nitrobenzamide

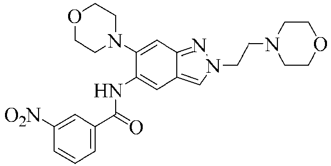

### [Step 1] Preparation of 4-(2-(6-bromo-5-nitro-2H-indazol-2-yl)ethyl)morpholine

Under the same conditions as in [Step 1] of Example 1, 6-bromo-5-nitro-2H-indazole (5 g, 20.66 mmole), potassium carbonate (11.42 g, 82.64 mmole), potassium iodide (0.5 g, 3.012 mmole), 4-(2-chloroethyl)morpholine hydrochloride (5.77 g, 31 mmole), and dimethylformamide (50 mL) were used and subjected to a reaction in the same manner, purified by MPLC (combiFlash NEXTGEN 300+), and concentrated to obtain a title compound (1.5 g).
¹H-NMR (DMSO-d⁶) 2.83(t, 2H), 3.48(t, 4H), 3.78(m, 4H), 4.53(t, 2H), 7.22(s, 1H), 8.16(s, 1H), 8.36(s, 1H)

### [Step 2] Preparation of 4-(2-(6-morpholino-5-nitro-2H-indazol-2-yl)ethyl)morpholine

Under the same conditions as in [Step 1] of Example 2, morpholine (10 mL) was added to the compound (1 g, 2.82 mmole) obtained in above [Step 1] and subjected to a reaction in the same manner, and then purified by MPLC (combiFlash NEXTGEN 300+) to obtain a title compound (1.15 g).
¹H-NMR (DMSO-d⁶) 2.43(m, 4H), 2.83(t, 2H), 2.90(m, 4H), 3.48(t, 4H), 3.78(m, 4H), 4.53(t, 2H), 7.26(s, 1H), 8.31(s, 1H), 8.54(s, 1H)

### [Step 3] Preparation of 6-morpholino-2-(2-morpholinoethyl)-2H-indazol-5-amine

Under the same conditions as in [Step 3] of Example 1, the compound (520 mg, 1.44 mmole) obtained in above [Step 2] was dissolved in ethyl acetate (10 mL) and hydrogenated by adding palladium on activated carbon (78 mg, 0.2 w/w). A reaction solution was filtered through celite and a filtrate was concentrated under reduced pressure to obtain a title compound (476 mg).
¹H-NMR (DMSO-d⁶) 2.41(m, 4H), 2.76(t, 2H), 2.80(m, 4H), 3.41(t, 4H), 3.62(m, 4H), 4.50(t, 2H), 4.53(s, 2H), 7.15(s, 1H), 8.16(s, 1H), 8.39(s, 1H)

### [Step 4] Preparation of N-(6-morpholino-2-(2-morpholinoethyl)-2H-indazol-5-yl)-3-nitrobenzamide (Compound 19)

Under the same conditions as in [Step 4] of Example 1, the compound (238 mg, 0.72 mmole) obtained in above [Step 3], 3-nitrobenzoic acid (120 mg, 0.72 mmole), dimethylformamide (10 mL), HATU (1.1 g, 2.88 mmole), and DIPEA (0.49 mL, 2.88 mmole) were used and subjected to a reaction in the same manner, purified by MPLC (combiFlash NEXTGEN 300+), and concentrated to obtain a title compound (38 mg).
¹H-NMR (DMSO-d⁶) 2.41(m, 4H), 2.83(t, 2H), 2.90(m, 4H), 3.51(t, 4H), 3.78(m, 4H), 4.53(t, 2H), 7.39(s, 1H), 7.86(m, 1H), 8.31(s, 1H), 8.35(m, 2H), 8.37(m, 1H), 8.70(s, 1H), 10.01(s, 1H)
LC-MS (ESI, m/z) = 481.5 (M+H⁺)

### Example 20. N-(2-(3-hydroxy-3-methylbutyl)-6-morpholino-2H-indazol-5-yl)-6-(1H-pyrazol-4-yl)picolinamide

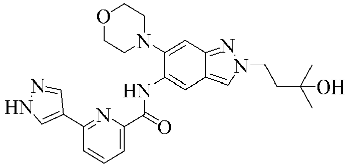

### [Step 4] Preparation of 6-bromo-N-(2-(3-hydroxy-3-methylbutyl)-6-morpholino-2H-indazol-5-yl)picolinamide

EDC·HCl (453 mg, 2.366 mmole), HOBt·H₂O (320 mg, 2.366 mmole), and dichloromethane (20 mL) were added to 6-bromopicolinic acid (382 mg, 1.892 mmole), and stirred at room temperature for two hours. After that, the compound (480 mg, 1.577 mmole) obtained in [Step 3] of Example 2 was added thereto and stirred at room temperature for three hours. After completion of a reaction, dichloromethane (40 mL) was added thereto and washed with purified water (40 mL). Anhydrous magnesium sulfate was added, filtered, concentrated under reduced pressure, and purified by MPLC (combiFlash NEXTGEN 300+). A separated compound was concentrated to add ethyl acetate (3 mL) thereto, and then a resulting solid was filtered to obtain a title compound (610 mg).
¹H-NMR (DMSO-d⁶) 1.12(s, 6H), 1.99(m, 2H), 2.90(t, 4H), 3.93(t, 4H), 4.41(m, 2H), 4.48(s, 1H), 7.43(s, 1H), 7.92(d, 1H), 8.01(t, 1H), 8.17(d, 1H), 8.31(s, 1H), 8.68(s, 1H), 11.03(s, 1H)

### [Step 5] Preparation of N-(2-(3-hydroxy-3-methylbutyl)-6-morpholino-2H-indazol-5-yl)-6-(1H-pyrazol-4-yl)picolinamide (Compound 20)

4-pyrazole boronic acid pinacol ester (50 mg, 0.26 mmole), (1,1'-bis(diphenylphosphino)ferrosine)dichloropalladium-dichloromethane (1:1) (8 mg, 0.013 mmole), potassium carbonate (83 mg, 0.6 mmole), 1,4-dioxane (1 mL), and purified water (0.2 mL) were added to the compound (100 mg, 0.2 mmole) obtained in above [Step 4] and stirred at 100°C for 23 hours. After completion of a reaction, a reaction solution was concentrated and ethyl acetate (50 mL) was added thereto and washed with purified water (50 mL). After that, anhydrous magnesium sulfate was added, filtered, concentrated under reduced pressure, and purified by MPLC (combiFlash NEXTGEN 300+) to obtain a title compound (18 mg) as a light brown solid.
¹H-NMR (DMSO-d⁶) 1.12(s, 6H), 2.00(m, 2H), 2.90(t, 4H), 3.79(t, 4H), 4.42(m, 2H), 4.48(s, 1H), 7.39(s, 1H), 7.94(m, 1H), 7.97(m, 1H), 9.30(s, 1H), 8.31(s, 1H), 8.50(s, 1H), 8.67(s, 1H), 10.94(s, 1H)
LC-MS (ESI, m/z) = 476.1 (M+H⁺)

### Example 21. N-(2-(3-hydroxy-3-methylbutyl)-6-morpholino-2H-indazol-5-yl)-6-(1H-pyrazol-5-yl)benzamide

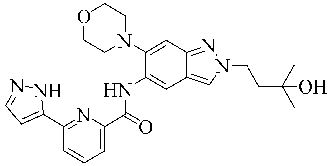

### [Step 4] Preparation of N-(2-(3-hydroxy-3-methylbutyl)-6-morpholino-2H-indazol-5-yl)-6-(1H-pyrazol-5-yl)benzamide (Compound 21)

Under the same conditions as in [Step 4] of Example 11, the compound (40 mg, 0.132 mmole) obtained in [Step 3] of Example 2, 6-(1H-pyrazol-5-yl)picolinic acid (30 mg, 0.159 mmole), EDC·HCl (38 mg, 0.198 mmole), HOBt·H₂O (27 mg, 0.198 mmole) and dichloromethane (6 mL) were used and subjected to a reaction in the same manner, purified by MPLC (combiFlash NEXTGEN 300+), and concentrated to obtain a title compound (10 mg).
¹H-NMR (DMSO-d⁶) 1.12(s, 6H), 2.00(m, 2H), 2.89(s, 4H), 3.83(s, 4H), 4.42(m, 2H), 4.48(s, 1H), 7.10(s, 1H), 7.39(s, 1H), 7.94(s, 1H), 8.09(s, 1H), 8.17(m, 1H), 8.31(s, 1H), 8.69(s, 1H), 11.00(s, 1H)
LC-MS (ESI, m/z) = 476.1 (M+H⁺)

### Example 22. 2-fluoro-N-(2-(3-hydroxy-3-methylbutyl)-6-morpholino-2H-indazol-5-yl)-3-nitrobenzamide

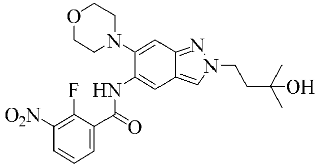

### [Step 4] Preparation of 2-fluoro-N-(2-(3-hydroxy-3-methylbutyl)-6-morpholino-2H-indazol-5-yl)-3-nitrobenzamide (Compound 22)

Under the same conditions as in [Step 4] of Example 11, 2-fluoro-3-nitrobenzoic acid (90 mg, 0.542 mmole), dimethylformamide (5 mL), EDC·HCl (207 mg, 1.08 mmole) and HOBt·H₂O (146 mg, 1.08 mmole) were added to the compound (0.11 g, 0.36 mmole) obtained in [Step 3] of Example 2, subjected to a reaction in the same manner, purified by MPLC (combiFlash NEXTGEN 300+), and concentrated to obtain a title compound (77 mg).
¹H-NMR (DMSO-d⁶) 1.12(s, 6H), 1.99(t, 2H), 2.88(m, 4H), 3.77(m, 4H), 4.42(t, 2H), 4.44(s, 1H), 7.43(s, 1H), 7.59(t, 1H), 8.23(t, 1H), 8.29-8.32(m, 2H), 8.53(s, 1H), 10.03(s, 1H)
LC-MS (ESI, m/z) = 472.5 (M+H⁺)

### Example 23. 4-amino-N-(2-(3-hydroxy-3-methylbutyl)-6-morpholino-2H-indazol-5-yl)-3-nitrobenzamide

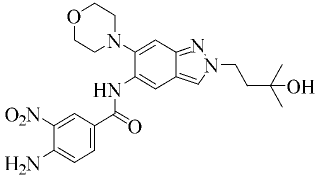

### [Step 4] Preparation of 4-amino-N-(2-(3-hydroxy-3-methylbutyl)-6-morpholino-2H-indazol-5-yl)-3-nitrobenzamide (Compound 23)

Under the same conditions as in [Step 4] of Example 11, 4-amino-3-nitrobenzoic acid (99 mg, 0.542 mmole), dimethylformamide (5 mL), EDC·HCl (207 mg, 1.08 mmole) and HOBt·H₂O (146 mg, 1.08 mmole) were added to the compound (0.11 g, 0.36 mmole) obtained in [Step 3] of Example 2, subjected to a reaction in the same manner, purified by MPLC (combiFlash NEXTGEN 300+), and concentrated to obtain a title compound (23 mg).
¹H-NMR (DMSO-d⁶) 1.11(s, 6H), 1.98(t, 2H), 2.88(m, 4H), 3.81(m, 4H), 4.40(t, 2H), 4.47(s, 1H), 7.11(d, 1H), 7.40(s, 1H), 7.87(s, 2H), 7.92(d, 1H), 8.28(s, 1H), 8.39(s, 1H), 8.59(s, 1H), 9.69(s, 1H)
LC-MS (ESI, m/z) = 469.5 (M+H⁺)

### Example 24. 3-fluoro-N-(2-(3-hydroxy-3-methylbutyl)-6-morpholino-2H-indazol-5-yl)-5-nitrobenzamide

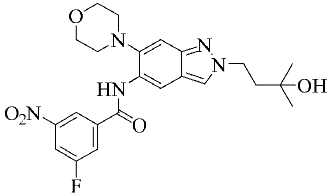

### [Step 4] Preparation of 3-fluoro-N-(2-(3-hydroxy-3-methylbutyl)-6-morpholino-2H-indazol-5-yl)-5-nitrobenzamide (Compound 24)

Under the same conditions as in [Step 4] of Example 11, 3-fluoro-5-nitrobenzoic acid (100 mg, 0.542 mmole), dimethylformamide (5 mL), EDC·HCl (207 mg, 1.08 mmole) and HOBt·H₂O (146 mg, 1.08 mmole) were added to the compound (0.11 g, 0.36 mmole) obtained in [Step 3] of Example 2, subjected to a reaction in the same manner, purified by MPLC (combiFlash NEXTGEN 300+), and concentrated to obtain a title compound (23 mg).
¹H-NMR (DMSO-d⁶) 1.12(s, 6H), 1.91(t, 2H), 2.89(m, 4H), 3.76(m, 4H), 4.41(t, 2H), 4.48(s, 1H), 7.38(s, 1H), 8.22(d, 1H), 8.26(s, 1H), 8.32(s, 1H), 8.36(d, 1H), 8.57(s, 1H), 10.04(s, 1H)
LC-MS (ESI, m/z) = 472.5 (M+H⁺)

### Example 25. N-(2-(3-hydroxy-3-methylbutyl)-6-morpholino-2H-indazol-5-yl)isophthalamide

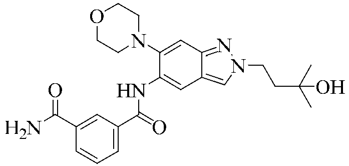

### [Step 4] Preparation of N-(2-(3-hydroxy-3-methylbutyl)-6-morpholino-2H-indazol-5-yl)isophthalamide (Compound 25)

Under the same conditions as in [Step 4] of Example 11, 3-carbamoylbenzoic acid (154 mg, 0.93 mmole), dimethylformamide (5 mL), EDC·HCl (357 mg, 1.86 mmole) and HOBt·H₂O (251 mg, 1.86 mmole) were added to the compound (0.189 g, 0.62 mmole) obtained in [Step 3] of Example 2, subjected to a reaction in the same manner, purified by MPLC (combiFlash NEXTGEN 300+), and concentrated to obtain a title compound (74 mg).
¹H-NMR (DMSO-d⁶) 1.12(d, 6H), 2.00(t, 2H), 2.88(m, 4H), 3.76(m, 4H), 4.41(t, 2H), 4.47(s, 1H), 7.40(s, 1H), 7.87(t, 1H), 8.19(d, 2H), 8.31(s, 1H), 8.33(m, 2H), 8.41(d, 1H), 8.69(s, 1H), 10.01(s, 1H)
LC-MS (ESI, m/z) = 452.5 (M+H⁺)

### Example 26. 4-fluoro-N-(2-(3-hydroxy-3-methylbutyl)-6-morpholino-2H-indazol-5-yl)-3-nitrobenzamide

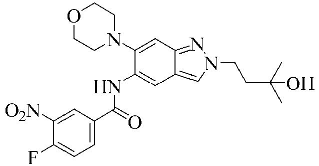

### [Step 4] Preparation of 4-fluoro-N-(2-(3-hydroxy-3-methylbutyl)-6-morpholino-2H-indazol-5-yl)-3-nitrobenzamide (Compound 26)

Under the same conditions as in [Step 4] of Example 11, 4-fluoro-3-nitrobenzoic acid (100 mg, 0.542 mmole), dimethylformamide (5 mL), EDC·HCl (207 mg, 1.08 mmole) and HOBt·H₂O (146 mg, 1.08 mmole) were added to the compound (0.11 g, 0.36 mmole) obtained in [Step 3] of Example 2, subjected to a reaction in the same manner, purified by MPLC (combiFlash NEXTGEN 300+), and concentrated to obtain a title compound (7 mg).
¹H-NMR (DMSO-d⁶) 1.12(s, 6H), 1.99(t, 2H), 2.88(m, 4H), 3.76(m, 4H), 4.43(t, 2H), 4.47(s, 1H), 7.38(s, 1H), 7.79(t, 1H), 8.29(s, 1H), 8.31(s, 1H), 8.34(d, 1H), 8.65(d, 1H), 9.95(s, 1H)
LC-MS (ESI, m/z) = 472.5 (M+H⁺)

### Example 27. N-(2-(3-hydroxy-3-methylbutyl)-6-morpholino-2H-indazol-5-yl)-3-(thiazol-2-vl)benzamide

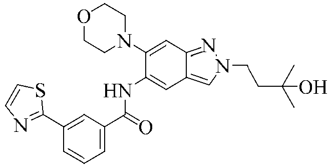

### [Step 4] Preparation of N-(2-(3-hydroxy-3-methylbutyl)-6-morpholino-2H-indazol-5-yl)-3-(thiazol-2-yl)benzamide (Compound 27)

Under the same conditions as in [Step 4] of Example 11, the compound (0.086 g, 0.283 mmole) obtained in [Step 3] of Example 2, 3-(1,3-thiazol-2-yl)benzoic acid (0.076 g, 0.37 mmole), dichloromethane (8 mL), EDC·HCl (0.081 g, 0.425 mmole) and HOBt·H₂O (0.057 g, 0.425 mmole) were used and subjected to a reaction in the same manner, purified by MPLC (combiFlash NEXTGEN 300+), and concentrated to obtain a title compound (47 mg).
¹H-NMR (DMSO-d⁶) 1.12(s, 6H), 2.00(m, 2H), 2.91(t, 4H), 3.82(t, 4H), 4.42(m, 2H), 4.48(s, 1H), 7.43(s, 1H), 7.70(t, 1H), 7.86(d, 1H), 7.97(d, 1H), 8.03(d, 1H), 8.16(d, 1H), 8.31(s, 1H), 8.45(s, 1H), 8.51(s, 1H), 9.94(s, 1H)
LC-MS (ESI, m/z) = 492.1 (M+H⁺)

### Example 28. N-(1-(3-hydroxy-3-methylbutyl)-6-morpholino-1H-indazol-5-yl)-3-nitrobenzamide

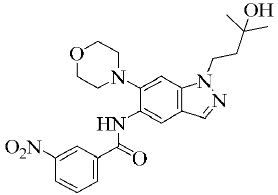

### [Step 2] Preparation of 2-methyl-4-(6-morpholino-5-nitro-1H-indazol-1-yl)butan-2-ol

Under the same conditions as in [Step 1] of Example 1, 4-bromo-2-methylbutan-2-ol (1.89 mL, 15.5 mmole), potassium carbonate (5.7 g, 41.32 mmole), potassium iodide (257 mg, 1.55 mmole) and dimethylformamide (10 mL) were added to the compound (2.57 g, 10.33 mmole) obtained in [Step 1] of Example 2, subjected to a reaction in the same manner, purified by MPLC (combiFlash NEXTGEN 300+), and concentrated to obtain a title compound (1.8 g).
¹H-NMR (DMSO-d⁶) 1.12(s, 6H), 1.99(m, 2H), 2.89(t, 4H), 3.66(t, 4H), 4.47(m, 2H), 4.48(s, 1H), 7.57(s, 1H), 7.86(s, 1H), 8.17(s, 1H)

### [Step 3] Preparation of 4-(5-amino-6-morpholino-1H-indazol-1-yl)-2-methylbutan-2-ol

Under the same conditions as in [Step 3] of Example 1, the compound (100 mg, 0.3 mmole) obtained in above [Step 2] was dissolved in ethyl acetate (10 mL) and hydrogenated by adding palladium on activated carbon (20 mg, 0.2 w/w). A reaction solution was filtered through celite and a filtrate was concentrated under reduced pressure to obtain a title compound (91 mg).
¹H-NMR (DMSO-d⁶) 1.11(s, 6H), 1.95(m, 2H), 2.75(t, 4H), 3.48(t, 4H), 4.35(m, 2H), 4.36(s, 1H), 4.50(s, 2H), 7.41(s, 1H), 7.75(s, 1H), 8.05(s, 1H)

### [Step 4] Preparation of N-(1-(3-hydroxy-3-methylbutyl)-6-morpholino-1H-indazol-5-yl)-3-nitrobenzamide (Compound 28)

Under the same conditions as in [Step 4] of Example 11, the compound (91 mg, 0.3 mmole) of above [Step 3], 3-nitrobenzoic acid (50 mg, 0.3 mmole), dimethylformamide (10 mL), EDC·HCl (173 mg, 0.9 mmole) and HOBt·H₂O (122 mg, 0.9 mmole) were used and subjected to a reaction in the same manner, purified by MPLC (combiFlash NEXTGEN 300+), and concentrated to obtain a title compound (84 mg).
¹H-NMR (DMSO-d⁶) 1.12(d, 6H), 1.90(t, 2H), 2.80(m, 4H), 3.69(m, 4H), 4.39(t, 2H), 4.42(s, 1H), 7.35(s, 1H), 7.70(m, 1H), 8.20(s, 1H), 8.24(m, 2H), 8.29(m, 1H), 8.60(s, 1H), 10.01(s, 1H)
LC-MS (ESI, m/z) = 456.5 (M+H⁺).

### Example 29, N-(2-(3-hydroxy-3-methylbutyl)-6-(4-(2-hydroxypropan-2-yl)piperidin-1-yl)-2H-indazol-5-yl)-3-sulfamoylbenzamide

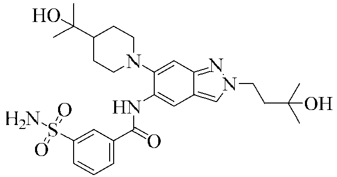

### [Step 1] Preparation of 2-(1-(5-nitro-1H-indazol-6-yl)piperidin-4-yl)propan-2-ol

2-piperidin-4-ylpropan-2-ol (9.47 g, 66.11 mmole) and DMSO (15 mL) were added to 6-bromo-5-nitro-lH-indazole (4 g, 16.53 mmole), and stirred at 120°C for three days. After completion of a reaction, ethyl acetate (150 mL) was added to wash with purified water (150 mL), and anhydrous magnesium sulfate was added thereto and filtered. A resulting product was concentrated under reduced pressure and purified by MPLC (combiFlash NEXTGEN 300+) to obtain a title compound (2.82 g).
¹H-NMR (DMSO-d⁶) 1.06(s, 6H), 1.35(m, 1H), 1.80(m, 2H), 1.97(m, 2H), 2.64(m, 2H), 3.07(d, 2H), 4.11(s, 1H), 7.32(s, 1H), 8.31(s, 1H), 8.55(s, 1H)

### [Step 2] Preparation of 4-(6-(4-(2-hydroxypropan-2-yl)piperidin-1-yl)-5-nitro-2H-indazol-2-yl)-2-methylbutan-2-ol

Under the same conditions as in [Step 2] of Example 2, the compound (2.8 g, 9.2 mmole) obtained in above [Step 1], 4-bromo-2-methylbutan-2-ol (1.68 mL, 13.8 mmole), potassium carbonate (5.09 g, 36.8 mmole), potassium iodide (0.28 g, 1.69 mmole) and dimethylformamide (56 mL) were used and subjected to a reaction in the same manner, purified by MPLC (combiFlash NEXTGEN 300+), and concentrated to obtain a title compound (0.9 g).
¹H-NMR (DMSO-d⁶) 1.06(s, 6H), 1.11(s, 6H), 1.35(m, 1H), 1.42(m, 2H), 1.80(m, 2H), 1.97(m, 2H), 2.64(m, 2H), 3.07(d, 2H), 4.11(s, 1H), 4.40(t, 2H), 4.47(s, 1H), 7.29(s, 1H), 8.25(s, 1H), 8.56(s, 1H)

### [Step 3] Preparation of 4-(5-amino-6-(4-(2-hydroxypropan-2-yl)piperidin-1-yl)-2H-indazol-2-yl)-2-methylbutan-2-ol

Under the same conditions as in [Step 3] of Example 2, the compound (900 mg, 2.3 mmole) obtained in above [Step 2] was dissolved in ethyl acetate (45 mL) and hydrogenated by adding palladium on activated carbon (200 mg, 0.2 w/w). A reaction solution was filtered through celite and a filtrate was concentrated under reduced pressure to obtain a title compound (320 mg).
¹H-NMR (DMSO-d⁶) 1.06(s, 6H), 1.11(s, 6H), 1.35(m, 1H), 1.42(m, 2H), 1.80(m, 2H), 1.97(m, 2H), 2.64(m, 2H), 3.07(d, 2H), 4.11(s, 1H), 4.40(t, 2H), 4.47(s, 1H), 7.29(s, 1H), 7.75(s, 1H), 8.06(s, 1H)

### [Step 4] Preparation of N-(2-(3-hydroxy-3-methylbutyl)-6-(4-(2-hydroxypropan-2-yl)piperidin-1-yl)-2H-indazol-5-yl)-3-sulfamoylbenzamide (Compound 29)

Under the same conditions as in [Step 4] of Example 11, the compound (80 mg, 0.222 mmole) obtained in above [Step 3], 3-sulfamoylbenzoic acid (54.2 mg, 0.266 mmole), EDC·HCl (64 mg, 0.333 mmole), HOBt·H₂O (46 mg, 0.333 mmole) and dichloromethane (5 mL) were used and subjected to a reaction in the same manner, purified by MPLC (combiFlash NEXTGEN 300+), and concentrated to obtain a title compound (18 mg).
¹H-NMR (DMSO-d⁶) 1.06(s, 6H), 1.11(s, 6H), 1.35(m, 1H), 1.43(m, 2H), 1.80(m, 2H), 1.99(m, 2H), 2.62-2.67(m, 2H), 3.07(d, 2H), 4.11(s, 1H), 4.41(t, 2H), 4.47(s, 1H), 7.36(s, 1H), 7.46(s, 2H), 7.74(t, 1H), 8.02(d, 1H), 8.12(d, 1H), 8.29(s, 1H), 8.36(s, 1H), 8.44(s, 1H), 9.81(s, 1H)
LC-MS (ESI, m/z) = 544.7 (M+H⁺)

### Example 30. N-(2-(3-hydroxy-3-methylbutyl)-6-(4-(2-hydroxypropan-2-yl)piperidin-1-yl)-2H-indazol-5-yl)-3-nitrobenzamide

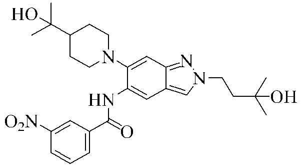

### [Step 4] Preparation of N-(2-(3-hydroxy-3-methylbutyl)-6-(4-(2-hydroxypropan-2-yl)piperidin-1-yl)-2H-indazol-5-yl)-3-nitrobenzamide (Compound 30)

Under the same conditions as in [Step 4] of Example 11, the compound (80 mg, 0.222 mmole) obtained in [Step 3] of above Example 29, 3-nitrobenzoic acid (44.4 mg, 0.266 mmole), EDC·HCl (64 mg, 0.333 mmole), HOBt·H₂O (46 mg, 0.333 mmole) and dichloromethane (5 mL) were used and subjected to a reaction in the same manner, purified by MPLC (combiFlash NEXTGEN 300+), and concentrated to obtain a title compound (28 mg).
¹H-NMR (DMSO-d⁶) 1.05(s, 6H), 1.12(s, 6H), 1.34-1.37(m, 1H), 1.44-1.46(m, 2H), 1.78-1.81(m, 2H), 1.97-2.00(m, 2H), 2.64(t, 2H), 3.09-3.11(m, 2H), 4.11(s, 1H), 4.41(t, 2H), 4.47(s, 1H), 7.35(s, 1H), 7.83(t, 1H), 8.29(s, 1H), 8.36(d, 1H), 8.39(s, 1H), 8.42(m, 1H), 8.67(s, 1H), 9.94(s, 1H)
LC-MS (ESI, m/z) = 510.6 (M+H⁺)

### Example 31, N-(2-(3-hydroxy-3-methylbutyl)-6-(4-hydroxypiperidin-1-yl)-2H-indazol-5-yl)-3-sulfamoylbenzamide

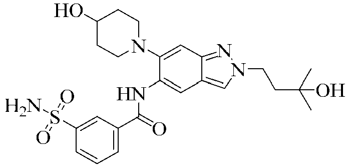

### [Step 2] Preparation of 1-(2-(3-hydroxy-3-methylbutyl)-5-nitro-2H-indazol-6-yl)piperidin-4-ol

Under the same conditions as in [Step 1] of Example 29, 4-hydroxypiperidine (9.247 g, 91.4 mmole) and DMSO (5 mL) were added to the compound (3 g, 9.142 mmole) obtained in [Step 1] of Example 4 and subjected to a reaction in the same manner, and then purified by MPLC (combiFlash NEXTGEN 300+) to obtain a title compound (0.33 g).
¹H-NMR (DMSO-d⁶) 1.12(s, 6H), 1.51-1.55(m, 2H), 1.82-1.85(m, 2H), 2.10(t, 2H), 2.65-2.67(m, 2H), 3.23-3.25(m, 2H), 3.52-3.54(m, 1H), 4.40(m, 2H), 4.51(s, 1H), 7.30(s, 1H), 8.30(s, 1H), 8.57(s, 1H)

### [Step 3] Preparation of 1-(5-amino-2-(3-hydroxy-3-methylbutyl)-2H-indazol-6-yl)piperidin-4-ol

Under the same conditions as in [Step 3] of Example 2, the compound (330 mg, 0.947 mmole) obtained in above [Step 2] was dissolved in ethyl acetate (10 mL) and hydrogenated by adding palladium on activated carbon (66 mg, 0.2 w/w). A reaction solution was filtered through celite and a filtrate was concentrated under reduced pressure to obtain a title compound (302 mg).
¹H-NMR (DMSO-d⁶) 1.12(s, 6H), 1.40-1.42(m, 2H), 1.68-1.69(m, 2H), 1.89(t, 2H), 2.48-2.53(m, 2H), 3.05-3.08(m, 2H), 3.40-3.44(m, 1H), 4.20(m, 2H), 4.31(s, 1H), 4.44(s, 2H), 7.28(s, 1H), 7.69(s, 1H), 8.02(s, 1H)

### [Step 4] Preparation of N-(2-(3-hydroxy-3-methylbutyl)-6-(4-hydroxypiperidin-1-yl)-2H-indazol-5-yl)-3-sulfamoylbenzamide (Compound 31)

Under the same conditions as in [Step 4] of Example 11, the compound (100 mg, 0.314 mmole) obtained in above [Step 3], 3-sulfamoylbenzoic acid (95 mg, 0.471 mmole), EDC·HCl (181 mg, 0.942 mmole), HOBt·H₂O (127 mg, 0.942 mmole) and dimethylformamide (5 mL) were used and subjected to a reaction in the same manner, purified by MPLC (combiFlash NEXTGEN 300+), and concentrated to obtain a title compound (21 mg).
¹H-NMR (DMSO-d⁶) 1.12(s, 6H), 1.50-1.52(m, 2H), 1.79-1.81(m, 2H), 2.00(t, 2H), 2.60-2.62(m, 2H), 3.20-3.23(m, 2H), 3.52-3.54(m, 1H), 4.43(m, 3H), 7.36(s, 1H), 7.47(s, 2H), 7.74(t, 1H), 8.04(d, 1H), 8.15(d, 1H), 8.21(s, 1H), 8.30(s, 1H), 8.35(s, 1H), 9.79(s, 1H)
LC-MS (ESI, m/z) = 502.6 (M+H⁺)

### Example 32. N-(2-(3-hydroxy-3-methylbutyl)-6-(4-hydroxypiperidin-1-yl)-2H-indazol-5-yl)-3-nitrobenzamide

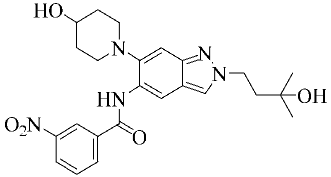

### [Step 4] Preparation of N-(2-(3-hydroxy-3-methylbutyl)-6-(4-hydroxypiperidin-1-yl)-2H-indazol-5-yl)-3-nitrobenzamide (Compound 32)

Under the same conditions as in [Step 4] of Example 11, the compound (100 mg, 0.314 mmole) obtained in [Step 3] of above Example 31, 3-nitrobenzoic acid (79 mg, 0.471 mmole), EDC·HCl (181 mg, 0.942 mmole), HOBt·H₂O (127 mg, 0.942 mmole) and dimethylformamide (5 mL) were used and subjected to a reaction in the same manner, purified by MPLC (combiFlash NEXTGEN 300+), and concentrated to obtain a title compound (28 mg).
¹H-NMR (DMSO-d⁶) 1.12(s, 6H), 1.46-1.52(m, 2H), 1.78-1.82(m, 2H), 2.00(t, 2H), 2.60-2.62(m, 2H), 3.19-3.22(m, 2H), 3.51-3.54(m, 1H), 4.41(m, 2H), 4.48(s, 1H), 7.35(s, 1H), 7.83(t, 1H), 8.29(s, 1H), 8.36(d, 1H), 8.38(s, 1H), 8.41(m, 1H), 8.62(s, 1H), 10.01(s, 1H)
LC-MS (ESI, m/z) = 468.5 (M+H⁺)

### Example 33. N-(2-(3-hydroxy-3-methylbutyl)-6-thiomorpholino-2H-indazol-5-yl)isophthalamide

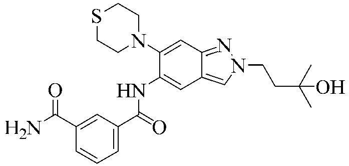

### [Step 2] Preparation of 2-methyl-4-(5-nitro-6-thiomorpholino-2H-indazol-2-yl)butan-2-ol

Thiomorpholine (10 mL), DIPEA (3.1 mL, 18.28 mmole) and DMSO (10 mL) were added to the compound (2 g, 6.09 mmole) obtained in [Step 1] of Example 4 and stirred at 100°C for seven days. After completion of a reaction, ethyl acetate (50 mL) was added to wash with purified water (50 mL), and anhydrous magnesium sulfate was added thereto and filtered. A resulting product was concentrated under reduced pressure and purified by MPLC (combiFlash NEXTGEN 300+) to obtain a title compound (670 mg).
¹H-NMR (DMSO-d⁶) 1.12(d, 6H), 2.00(t, 2H), 2.59(m, 4H), 3.41(m, 4H), 4.41(t, 2H), 4.47(s, 1H), 7.32(s, 1H), 8.31(s, 1H), 8.57(s, 1H)

### [Step 3] Preparation of 4-(5-amino-6-thiomorpholino-2H-indazol-2-yl)-2-methylbutan-2-ol

Under the same conditions as in [Step 3] of Example 1, the compound (670 mg, 1.91 mmole) obtained in above [Step 2] was dissolved in ethyl acetate (10 mL) and hydrogenated with palladium on activated carbon (101 mg, 0.15 w/w). A reaction solution was filtered through celite and a filtrate was concentrated under reduced pressure to obtain a title compound (613 mg).
¹H-NMR (DMSO-d⁶) 1.12(d, 6H), 2.00(t, 2H), 2.41(m, 4H), 3.29(m, 4H), 4.29(t, 2H), 4.40(s, 1H), 4.52(s, 2H), 7.29(s, 1H), 7.75(s, 1H), 8.05(s, 1H)

### [Step 4] Preparation of N-(2-(3-hydroxy-3-methylbutyl)-6-thiomorpholino-2H-indazol-5-yl)isophthalamide (Compound 33)

Under the same conditions as in [Step 4] of Example 11, the compound (0.150 g, 0.468 mmole) obtained in above [Step 3], 3-carbamoylbenzoic acid (65 mg, 0.394 mmole), dimethylformamide (5 mL), EDC·HCl (227 mg, 1.18 mmole) and HOBt·H₂O (158 mg, 1.169 mmole) were used and subjected to a reaction in the same manner, purified by MPLC (combiFlash NEXTGEN 300+), and concentrated to obtain a title compound (20 mg).
¹H-NMR (DMSO-d⁶) 1.12(d, 6H), 2.00(t, 2H), 2.59(m, 4H), 3.41(m, 4H), 4.41(t, 2H), 4.47(s, 1H), 7.40(s, 1H), 7.87(t, 1H), 8.19(d, 2H), 8.35(m, 3H), 8.41(d, 1H), 8.69(s, 1H), 10.01(s, 1H)
LC-MS (ESI, m/z) = 468.6 (M+H⁺)

### Example 34. N-(6-(4-fluorophenyl)-2-(2-morpholinoethyl)-2H-indazol-5-yl)-3-nitrobenzamide

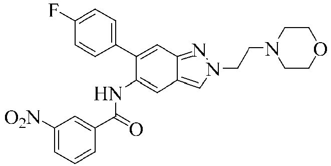

### [Step 2] Preparation of 4-(2-(6-(4-fluorophenyl)-5-nitro-2H-indazol-2-yl)ethyl)morpholine

Under the same conditions as in [Step 2] of Example 1, the compound (1.0 g, 2.82 mmole) obtained in [Step 1] of Example 19, sodium carbonate (1.49 g, 14.8 mmole), tetrakis(triphenylphosphine)palladium (0.16 g, 0.14 mmole), and (4-fluorophenyl)boronic acid (0.59 g, 4.22 mmole) were used and subjected to a reaction in the same manner, purified by MPLC (combiFlash NEXTGEN 300+), and concentrated to obtain a title compound (0.86 g).
¹H-NMR (DMSO-d⁶) 2.40-2.41(br. s, 4H), 2.84(t, 2H), 3.50-3.51(m, 4H), 4.53(t, 2H), 7.39(t, 2H), 7.67(d, 2H), 8.44(s, 1H), 8.52(s, 1H), 8.57(s, 1H)

### [Step 3] Preparation of 6-(4-fluorophenyl)-2-(2-morpholinoethyl)-2H-indazol-5-amine

Under the same conditions as in [Step 3] of Example 1, ethyl acetate (80 mL) was added to the compound (0.8 g, 2.16 mmole) obtained in above [Step 2] and hydrogenated by adding palladium on activated carbon (0.16 g, 0.2 w/w). A reaction mixture was filtered through celite and a filtrate was concentrated to obtain a title compound (0.61 g).
¹H-NMR (DMSO-d⁶) 2.35-2.36(br. s, 4H), 2.84(t, 2H), 3.45-3.46(m, 4H), 4.51(s, 2H), 4.53(t, 2H), 7.34(t, 2H), 7.63(d, 2H), 8.40(s, 1H), 8.48(s, 1H), 8.52(s, 1H)

### [Step 4] Preparation of N-(6-(4-fluorophenyl)-2-(2-morpholinoethyl)-2H-indazol-5-yl)-3-nitrobenzamide (Compound 34)

Under the same conditions as in [Step 4] of Example 1, the compound (0.2 g, 0.59 mmole) obtained in above [Step 3], 3-nitrobenzoic acid (99 mg, 0.59 mmole), dimethylformamide (2 mL), HATU (0.45 g, 1.18 mmole), and DIPEA (0.41 mL, 2.35 mmole) were used and subjected to a reaction in the same manner, purified by MPLC (combiFlash NEXTGEN 300+), and concentrated to obtain a title compound (0.2 g).
¹H-NMR (DMSO-d⁶) 2.41(br. S, 4H), 2.83(t, 2H), 3.51(t, 4H), 4.53(t, 2H), 7.32(t, 2H), 7.52(s, 1H), 7.58(t, 2H), 7.78(t, 1H), 8.17(d, 1H), 8.37(d, 1H), 8.43(s, 1H), 8.47(s, 1H), 8.49(s, 1H), 9.63(s, 1H)
LC-MS (ESI, m/z) = 490.5 (M+H⁺)

### Example 35. N-(2-(3-hydroxy-3-methylbutyl)-6-(3-(morpholine-4-carbonyl)phenyl)-2H-indazol-5-yl)-3-nitrobenzamide

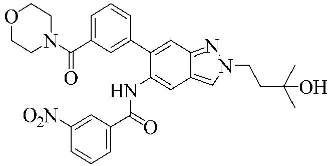

### [Step 1] Preparation of 3-(2-(3-hydroxy-3-methylbutyl)-5-nitro-2H-indazol-6-yl)benzoic acid

Under the same conditions as in [Step 2] of Example 1, the compound (1.2 g, 3.66 mmole) obtained in [Step 1] of Example 4, sodium carbonate (1.94 g, 18.3 mmole), tetrakis(triphenylphosphine)palladium (0.21 g, 0.18 mmole), and 3-carboxyphenylboronic acid pinacol ester (1.44 g, 5.48 mmole) were used and subjected to a reaction in the same manner, purified by MPLC (combiFlash NEXTGEN 300+), and concentrated to obtain a title compound (1.01 g).
¹H-NMR (DMSO-d⁶) 1.13(s, 6H), 2.06(t, 2H), 4.50(s, 1H), 4.51(t, 2H), 7.43(m, 2H), 8.15(s, 2H), 8.27(s, 1H), 8.44(s, 1H), 10.27(s, 1H)

### [Step 2] Preparation of (3-(2-(3-hydroxy-3-methylbutyl)-5-nitro-2H-indazol-6-yl)phenyl)(morpholino)methanone

Under the same conditions as in [Step 4] of Example 11, the compound (0.5 g, 1.66 mmole) obtained in above [Step 1], morpholine (0.14 g, 1.66 mmole), EDC·HCl (0.48 g, 2.49 mmole), HOBt·H₂O (0.34 g, 2.49 mmole) and dichloromethane (2 mL) were used and subjected to a reaction in the same manner, purified by MPLC (combiFlash NEXTGEN 300+), and concentrated to obtain a title compound (120 mg).
¹H-NMR (DMSO-d⁶) 1.13(s, 6H), 2.06(t, 2H), 3.13(t, 4H), 3.59(t, 4H), 4.50(s, 1H), 4.51(t, 2H), 7.43(m, 2H), 8.15(s, 2H), 8.27(s, 1H), 8.44(s, 1H), 10.27(s, 1H)

### [Step 3] Preparation of (3-(5-amino-2-(3-hydroxy-3-methylbutyl)-2H-indazol-6-yl)phenyl)(morpholino)methanone

Under the same conditions as in [Step 3] of Example 1, the compound (0.5 g, 1.14 mmole) obtained in above [Step 2] was dissolved in ethyl acetate (10 mL) and hydrogenated by adding palladium on activated carbon (0.02 g, 0.2 w/w). After a reaction, a reaction product was filtered through celite and a filtrate was concentrated to obtain a title compound (0.1 g).
¹H-NMR (DMSO-d⁶) 1.13(s, 6H), 2.06(t, 2H), 3.13(t, 4H), 3.59(t, 4H), 4.50(s, 1H), 4.51(t, 2H), 7.18(s, 2H), 7.44(m, 2H), 8.14(s, 2H), 8.29(s, 1H), 8.44(s, 1H), 10.27(s, 1H)

### [Step 4] Preparation of N-(2-(3-hydroxy-3-methylbutyl)-6-(3-(morpholin-4-carbonyl)phenyl)-2H-indazol-5-yl)-3-nitrobenzamide (Compound 35)

Under the same conditions as in [Step 4] of Example 1, the compound (0.4 g, 0.98 mmole) obtained in above [Step 3], 3-nitrobenzoic acid (0.16 g, 0.98 mmole), dimethylformamide (10 mL), HATU (0.74 g, 1.96 mmole), and DIPEA (0.68 mL, 3.92 mmole) were used and subjected to a reaction in the same manner, purified by MPLC (combiFlash NEXTGEN 300+), and concentrated to obtain a title compound (0.4 g).
¹H-NMR (DMSO-d⁶) 1.13(s, 6H), 2.06(t, 2H), 3.13(t, 4H), 3.59(t, 4H), 4.50(s, 1H), 4.51(t, 2H), 7.18(s, 1H), 7.44(m, 2H), 7.58(m, 3H), 8.14(s, 2H), 8.29(s, 1H), 8.44(s, 1H), 8.59(s, 1H), 10.27(s, 1H)
LC-MS (ESI, m/z) = 558.1 (M+H⁺)

### Example 36. N-(2-(3-hydroxy-3-methylbutyl)-6-morpholino-2H-indazol-5-yl)-6-(thiophen-3-yl)picolinamide

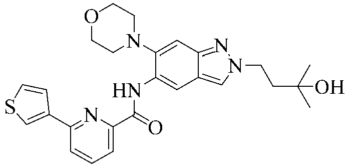

### [Step 4] Preparation of N-(2-(3-hydroxy-3-methylbutyl)-6-morpholino-2H-indazol-5-yl)-6-(thiophen-3-yl)picolinamide (Compound 36)

Under the same conditions as in [Step 4] of Example 11, the compound (0.086 g, 0.283 mmole) obtained in [Step 3] of Example 2, 6-(thiophen-3-yl)picolinic acid (76 mg, 0.37 mmole), dichloromethane (8 mL), EDC·HCl (0.081 g, 0.425 mmole) and HOBt·H₂O (0.057 g, 0.425 mmole) were used and subjected to a reaction in the same manner, purified by MPLC (combiFlash NEXTGEN 300+), and concentrated to obtain a title compound (48 mg).
¹H-NMR (DMSO-d⁶) 1.12(s, 6H), 1.99(m, 2H), 2.89(t, 4H), 3.76(t, 4H), 4.43(m, 2H), 4.48(s, 1H), 7.40(s, 1H), 7.75(dd, 1H), 7.99(dd, 1H), 8.10(m, 3H), 8.32(s, 1H), 8.39(dd, 1H), 8.69(s, 1H), 11.00(s, 1H)
LC-MS (ESI, m/z) = 492.1 (M+H⁺)

### Example 37. N-(2-(3-hydroxy-3-methylbutyl)-6-(4-morpholinophenyl)-2H-indazol-5-yl)-3-nitrobenzamide

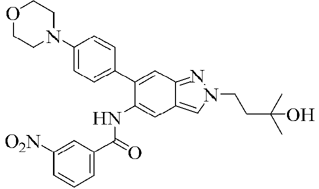

### [Step 2] Preparation of 2-methyl-4-(6-(4-morpholinophenyl)-5-nitro-2H-indazol-2-yl)butan-2-ol

Under the same conditions as in [Step 2] of Example 1, the compound (0.5 g, 1.52 mmole) obtained in [Step 1] of Example 4, sodium carbonate (0.81 g, 7.61 mmole), tetrakis(triphenylphosphine)palladium (0.09 g, 0.08 mmole), 4-(4-(4,4,5,5-tetramethyl-1,3,2-dioxaborolan-2-yl)phenyl)morpholine (0.66 g, 2.29 mmole), 1,4-dioxane (20 mL) and purified water (2 mL) were used and subjected to a reaction in the same manner, purified by MPLC (combiFlash NEXTGEN 300+), and concentrated to obtain a title compound (0.5 g).
¹H-NMR (DMSO-d⁶) 1.13(s, 6H), 2.03(t, 2H), 3.07(t, 4H), 3.68(t, 4H), 4.48(t, 2H), 4.50(s, 1H), 6.91(d, 2H), 7.32(d, 2H), 7.48(s, 1H), 7.75(s, 1H), 8.39(s, 1H)

### [Step 3] Preparation of 4-(5-amino-6-(4-morpholinophenyl)-2H-indazol-2-yl)-2-methylbutan-2-ol

Under the same conditions as in [Step 3] of Example 1, the compound (0.5 g, 1.22 mmole) obtained in above [Step 2] was dissolved in ethyl acetate (10 mL) and hydrogenated by adding palladium on activated carbon (0.10 g, 0.093 mmole). A resulting product was purified by MPLC (combiFlash NEXTGEN 300+) and concentrated to obtain a title compound (0.36 g).
¹H-NMR (DMSO-d⁶) 1.13(s, 6H), 2.03(t, 2H), 3.07(t, 4H), 3.68(t, 4H), 4.48(t, 2H), 4.50(s, 1H), 6.91(d, 2H), 7.32(d, 2H), 7.48(s, 1H), 7.75(s, 1H), 8.39(s, 1H), 8.51(s, 2H)

### [Step 4] Preparation of N-(2-(3-hydroxy-3-methylbutyl)-6-(4-morpholinophenyl)-2H-indazol-5-yl)-3-nitrobenzamide (Compound 37)

Under the same conditions as in [Step 4] of Example 1, the compound (0.4 g, 1.05 mmole) obtained in above [Step 3], 3-nitrobenzoic acid (0.18 g, 1.05 mmole), dimethylformamide (5 mL), HATU (0.80 g, 2.10 mmole), and DIPEA (0.73 mL, 4.21 mmole) were used and subjected to a reaction in the same manner, purified by MPLC (combiFlash NEXTGEN 300+), and concentrated to obtain a title compound (30 mg).
¹H-NMR (DMSO-d⁶) 1.13(s, 6H), 2.03(t, 2H), 3.07(t, 4H), 3.68(t, 4H), 4.48(t, 2H), 4.50(s, 1H), 6.91(d, 2H), 7.32(d, 2H), 7.48(s, 1H), 7.75(s, 1H), 8.33(t, 2H), 8.39(s, 1H), 8.51(s, 2H), 10.07(s, 1H)
LC-MS (ESI, m/z) = 530.4 (M+H⁺)

### Example 38. N-(6-(dimethylamino)-2-(3-hydroxy-3-methylbutyl)-2H-indazol-5-yl)-3-nitrobenzamide

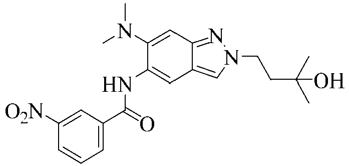

### [Step 1] Preparation of N,N-dimethyl-5-nitro-1H-indazol-6-amine

Dimethylformamide (20 mL) and potassium iodide (500 mg, 3.012 mmole) were added to 6-bromo-5-nitro-1H-indazole (5 g, 20.66 mmole) and stirred at 120°C for three days. After completion of a reaction, ethyl acetate (200 mL) was added to wash with purified water (200 mL), and anhydrous magnesium sulfate was added thereto and filtered. A resulting product was concentrated under reduced pressure and purified by MPLC (combiFlash NEXTGEN 300+) to obtain a title compound (2.12 g) as an orange-colored solid.
¹H-NMR (DMSO-d⁶) 2.74(s, 6H), 7.20(s 1H), 8.14(s, 1H), 8.32(s, 1H)

### [Step 2] Preparation of 4-(6-(dimethylamino)-5-nitro-2H-indazol-2-yl)-2-methylbutan-2-ol

4-bromo-2-methylbutan-2-ol (1.874 mL, 15.42 mmole), potassium carbonate (5.68 g, 41.12 mmole), potassium iodide (212 mg, 1.277 mmole), and dimethylformamide (20 mL) were added to the compound (2.12 g, 10.28 mmole) obtained in above [Step 1] and stirred at 90°C for five hours. After completion of a reaction, ethyl acetate (50 mL) was added to wash with purified water (50 mL), and anhydrous magnesium sulfate was added thereto and filtered. A resulting product was concentrated under reduced pressure and purified by MPLC (combiFlash NEXTGEN 300+) to obtain a title compound (1.3 g) as an orange-colored solid.
¹H-NMR (DMSO-d⁶) 1.11(s, 6H), 1.99(m, 2H), 2.67(s, 6H), 4.47(t, 2H), 4.48(s, 1H), 7.30(s, 1H), 8.29(s, 1H), 8.54(s, 1H)

### [Step 3] Preparation of 4-(5-amino-6-(dimethylamino)-2H-indazol-2-yl)-2-methylbutan-2-ol

Under the same conditions as in [Step 3] of Example 2, palladium on activated carbon (20 mg, 10 w/w), dichloromethane (20 mL) and ethyl acetate (20 mL) were added to the compound (100 mg, 0.342 mmole) obtained in above [Step 2] and hydrogenated in the same manner. A reaction solution was filtered through celite and washed with ethyl acetate, and then a filtrate was concentrated under reduced pressure to obtain a title compound (90 mg).
¹H-NMR (DMSO-d⁶) 1.11(s, 6H), 1.95(m, 2H), 2.54(s, 6H), 4.35(m, 2H), 4.36(s, 1H), 4.40(s, 2H), 7.29(s, 1H), 7.75(s, 1H), 8.05(s, 1H)

### [Step 4] Preparation of N-(6-(dimethylamino)-2-(3-hydroxy-3-methylbutyl)-2H-indazol-5-yl)-3-nitrobenzamide (Compound 38)

Under the same conditions as in [Step 4] of Example 11, the compound (90 mg, 0.342 mmole) obtained in above [Step 3], 3-nitrobenzoic acid (68.6 mg, 0.41 mmole), EDC·HCl (98 mg, 0.513 mmole), HOBt·H₂O (69 mg, 0.513 mmole) and dichloromethane (5 mL) were used and subjected to a reaction in the same manner, and then dichloromethane (20 mL) was added thereto and washed with purified water (20 mL). Anhydrous magnesium sulfate was added thereto, filtered, and concentrated under reduced pressure, methanol (1.5 mL) was added thereto, and a resulting solid was filtered to obtain a title compound (47 mg).
¹H-NMR (DMSO-d⁶) 1.12(s, 6H), 1.98(t, 2H), 2.67(s, 6H), 4.41(m, 2H), 4.48(s, 1H), 7.27(s, 1H), 7.83(t, 1H), 8.17(s, 1H), 8.27(s, 1H), 8.35(d, 1H), 8.41(d, 1H), 8.70(s, 1H), 9.91(s, 1H)
LC-MS (ESI, m/z) = 412.3 (M+H⁺)

### Example 39. N-(2-(3-hydroxy-3-methylbutyl)-6-(4-(morpholinomethyl)phenyl)-2H-indazol-5-yl)-3-sulfamoylbenzamide

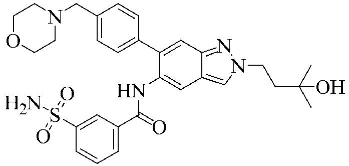

### [Step 2] Preparation of 2-methyl-4-(5-nitro-6-(4-(trifluoromethyl)phenyl)-2H-indazol-2-yl)butan-2-ol

Under the same conditions as in [Step 2] of Example 1, the compound (1.0 g, 3.05 mmole) obtained in [Step 1] of Example 4, sodium carbonate (1.61 g, 15.24 mmole), tetrakis(triphenylphosphine)palladium (0.18 g, 0.15 mmole), 4-(4-(4,4,5,5-tetramethyl-1,3,2-dioxaborolan-2-yl)benzoyl)morpholine (1.39 g, 4.57 mmole), 1,4-dioxane (10 mL) and purified water (1 mL) were used and subjected to a reaction in the same manner, purified by MPLC (combiFlash NEXTGEN 300+), and concentrated to obtain a title compound (1.10 g).
¹H-NMR (DMSO-d⁶) 1.12(s, 6H), 2.02(t, 2H), 3.61(br. s, 4H), 3.50(t, 4H), 3.61(s, 2H), 4.48-5.10(m, 3H), 7.49-7.50(d, 2H), 8.12(dt, 1H), 8.44(d, 2H), 8.46(s, 1H), 8.66(s, 1H)

### [Step 3] Preparation of 4-(5-amino-6-(4-(trifluoromethyl)phenyl)-2H-indazol-2-yl)-2-methylbutan-2-ol

Under the same conditions as in [Step 3] of Example 1, the compound (1.0 g, 2.36 mmole) obtained in above [Step 2] was dissolved in ethyl acetate (20 mL) and hydrogenated by adding palladium on activated carbon (0.2 g, 0.2 w/w). A reaction mixture was filtered through celite and a filtrate was concentrated to obtain a title compound (0.72 g).
¹H-NMR (DMSO-d⁶) 1.06(s, 6H), 1.96(t, 2H), 3.55(br. s, 4H), 3.44(t, 4H), 3.55(s, 2H), 4.42-5.04(m, 3H), 4.56(s, 2H), 7.43-7.54(d, 2H), 8.06(dt, 1H), 8.38(d, 2H), 8.40(s, 1H), 8.61(s, 1H)

### [Step 4] Preparation of N-(2-(3-hydroxy-3-methylbutyl)-6-(4-(morpholinomethyl)phenyl)-2H-indazol-5-yl)-3-sulfamoylbenzamide (Compound 39)

Under the same conditions as in [Step 4] of Example 1, the compound (0.11 g, 0.28 mmole) obtained in above [Step 3], 3-sulfamoylbenzoic acid (0.06 g, 0.28 mmole), HATU (0.21 g, 0.56 mmole) and DIPEA (0.19 mL, 1.12 mmole) were used and subjected to a reaction in the same manner, purified by MPLC (combiFlash NEXTGEN 300+), and concentrated to obtain a title compound (0.13 g).
¹H-NMR (DMSO-d⁶) 1.13(s, 6H), 2.05(t, 2H), 2.28(br. s, 4H), 3.48(br. s, 4H), 3.62(s, 2H), 4.47-4.51(m, 3H), 7.13(d, 2H), 7.28(d, 2H), 7.40(br. s, 2H), 7.49(s, 1H), 7.61(t, 1H), 7.72(s, 1H), 7.91(d, 2H), 8.20(s, 1H), 8.40(s, 1H), 9.97(s, 1H)
LC-MS (ESI, m/z) = 578.1 (M+H⁺)

### Example 40. N-(2-(3-hydroxy-3-methylbutyl)-6-thiomorpholino-2H-indazol-5-yl)-3-nitrobenzamide

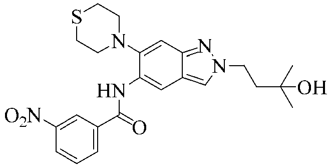

### [Step 4] Preparation of N-(2-(3-hydroxy-3-methylbutyl)-6-thiomorpholino-2H-indazol-5-yl)-3-nitrobenzamide (Compound 40)

Under the same conditions as in [Step 4] of Example 11, the compound (0.150 g, 0.468 mmole) obtained in [Step 3] of Example 33, 3-nitrobenzoic acid (65 mg, 0.389 mmole), dimethylformamide (5 mL), EDC·HCl (224 mg, 1.17 mmole) and HOBt·H₂O (158 mg, 1.169 mmole) were used and subjected to a reaction in the same manner, purified by MPLC (combiFlash NEXTGEN 300+), and concentrated to obtain a title compound (14 mg).
¹H-NMR (DMSO-d⁶) 1.12(d, 6H), 2.00(t, 2H), 2.59(m, 4H), 3.42(m, 4H), 4.41(t, 2H), 4.47(s, 1H), 7.20(s, 1H), 7.76(m, 1H), 8.33(s, 1H), 8.35(m, 2H), 8.37(m, 1H), 8.74(s, 1H), 10.05(s, 1H)
LC-MS (ESI, m/z) = 470.6 (M+H⁺)

### Example 41. N-(2-(3-hydroxy-3-methylbutyl)-6-(4-((2-(methylamino)-2-oxoethyl)carbamoyl)phenyl)-2H-indazol-5-yl)-3-nitrobenzamide

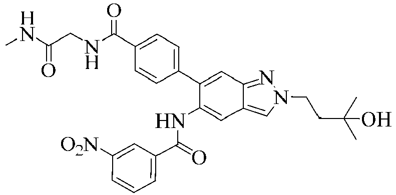

### [Step 2] Preparation of methyl (4-(2-(3-hydroxy-3-methylbutyl)-5-nitro-2H-indazol-6-yl)benzoyl)glycinate

Under the same conditions as in [Step 2] of Example 1, the compound (1.9 g, 5.79 mmole) obtained in [Step 1] of Example 4, sodium carbonate (3.07 g, 28.9 mmole), tetrakis(triphenylphosphine)palladium (0.33 g, 0.29 mmole), (4-((2-methoxy-2-oxoethyl)carbamoyl)phenyl)boronic acid (2.06 g, 8.68 mmole), 1,4-dioxane (20 mL) and purified water (2 mL) were used and subjected to a reaction in the same manner, purified by MPLC (combiFlash NEXTGEN 300+), and concentrated to obtain a title compound (1.84 g).
¹H-NMR (DMSO-d⁶) 1.13(s, 6H), 2.03-2.05(m, 2H), 3.64(s, 3H), 4.38(d, 2H), 4.53-4.57(m, 3H), 7.45(s, 1H), 7.56(s, 1H), 7.88(d, 2H), 7.99(s, 1H), 8.62(s, 1H), 8.78(s, 1H), 9.00(t, 1H)

### [Step 3] Preparation of methyl (4-(5-amino-2-(3-hydroxy-3-methylbutyl)-2H-indazol-6-yl)benzoyl)glycinate

Under the same conditions as in [Step 3] of Example 1, palladium on activated carbon (0.3 g, 0.2 w/w) and ethyl acetate (20 mL) were added to the compound (1.5 g, 3.41 mmole) obtained in above [Step 2] and hydrogenated. A reaction mixture was filtered through celite and a filtrate was concentrated to obtain a title compound (1.92 g).
¹H-NMR (DMSO-d⁶) 1.11(s, 6H), 2.01-2.03(m, 2H), 3.62(s, 3H), 4.35(d, 2H), 4.45(s, 2H), 4.51-4.55(m, 3H), 7.42(s, 1H), 7.45(s, 1H), 7.85(d, 2H), 7.96(s, 1H), 8.58(s, 1H), 8.74(s, 1H), 8.96(t, 1H)

### [Step 4] Preparation of methyl (4-(2-(3-hydroxy-3-methylbutyl)-5-(3-nitrobenzamido)-2H-indazol-6-yl)benzoyl)glycinate

Under the same conditions as in [Step 4] of Example 11, the compound (1.5 g, 3.65 mmole) obtained in above [Step 3], 3-nitrobenzoic acid (0.67 g, 3.65 mmole), dichloromethane (15 mL), EDC·HCl (1.05 g, 5.48 mmole) and HOBt·H₂O (0.74 g, 5.48 mmole) were used and subjected to a reaction in the same manner, purified by MPLC (combiFlash NEXTGEN 300+), and concentrated to obtain a title compound (0.60 g).
¹H-NMR (DMSO-d⁶) 1.11(s, 6H), 2.03(t, 2H), 3.88(d, 2H), 4.49-4.50(m, 3H), 7.52(d, 2H), 7.75(m, 2H), 7.81(d, 2H), 8.18(d, 1H), 8.35(d, 1H), 8.48(s, 1H), 8.51(s, 1H), 8.60(t, 1H), 10.01(s, 1H)

### [Step 5] Preparation of N-(2-(3-hydroxy-3-methylbutyl)-6-(4-((2-(methylamino)-2-oxoethyl)carbamoyl)phenyl)-2H-indazol-5-yl)-3-nitrobenzamide (Compound 41)

A solution of 40% N-methylamine in methanol (10 ml) was added to the compound (0.1 g, 0.17 mmole) obtained in above [Step 4], stirred at 120°C for six hours, and then concentrated and purified by MPLC (combiFlash NEXTGEN 300+) to obtain a title compound (0.25 g).
¹H-NMR (DMSO-d⁶) 1.13(s, 6H), 2.03-2.05(m, 2H), 2.64(d, 3H), 4.38(d, 2H), 4.53-4.57(m, 3H), 7.45(s, 1H), 7.59(s, 1H), 7.73-7.74(m, 2H), 7.82(d, 2H), 7.99(d, 1H), 8.62(d, 1H), 8.57-8.58(m, 2H), 8.78(s, 1H), 9.01(t, 1H), 9.79(s, 1H)
LC-MS (ESI, m/z) = 559.0 (M+H⁺)

### Example 42, N-(2-(3-hydroxy-3-methylbutyl)-6-(4-((2,2,2-trifluoroethyl)carbamoyl)phenyl)-2H-indazol-5-yl)-3-sulfamoylbenzamide

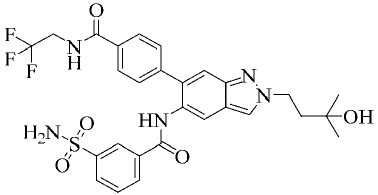

### [Step 2] Preparation of methyl 4-(2-(3-hydroxy-3-methylbutyl)-5-nitro-2H-indazol-6-yl)benzoate

Under the same conditions as in [Step 2] of Example 1, the compound (1.9 g, 5.79 mmole) obtained in [Step 1] of Example 4, sodium carbonate (3.07 g, 28.9 mmole), tetrakis(triphenylphosphine)palladium (0.33 g, 0.29 mmole), and methyl 4-(4,4,5,5-tetramethyl-1,3,2-dioxaborolan-2-yl)benzoate (2.28 g, 8.68 mmole) were used and subjected to a reaction in the same manner, purified by MPLC (combiFlash NEXTGEN 300+), and concentrated to obtain a title compound (1.61 g).
¹H-NMR (DMSO-d⁶) 1.12(s, 6H), 2.02-2.04(m, 2H), 3.84(s, 3H), 4.53-4.55(m, 2H), 4.62(s, 1H), 7.46(s, 1H), 7.48(s, 1H), 7.66(s, 1H), 7.97(s, 1H), 7.98(s, 1H), 8.62(s, 1H), 8.75(s, 1H)

### [Step 3] Preparation of methyl 4-(5-amino-2-(3-hydroxy-3-methylbutyl)-2H-indazol-6-yl)benzoate

Under the same conditions as in [Step 3] of Example 2, the compound (1.5 g, 3.91 mmole) obtained in above [Step 2] was dissolved in ethyl acetate (30 mL) and hydrogenated with palladium on activated carbon (0.3 g, 0.2 w/w). A reaction mixture was filtered through celite and a filtrate was concentrated to obtain a title compound (1.18 g).
¹H-NMR (DMSO-d⁶) 1.07(s, 6H), 1.97-1.99(m, 2H), 3.79(s, 3H), 4.48-4.52(m, 4H), 4.57(s, 1H), 7.41(s, 1H), 7.42(d, 1H), 7.62(s, 1H), 7.91(t, 1H), 7.92(s, 1H), 8.56(s, 1H), 8.69(s, 1H)

### [Step 4] Preparation of methyl 4-(2-(3-hydroxy-3-methylbutyl)-5-(3-sulfamoylbenzamido)-2H-indazol-6-yl)benzoate

Under the same conditions as in [Step 4] of Example 11, 3-sulfamoyl benzoic acid (0.68 g, 3.11 mmole), dichloromethane (11 mL), EDC·HCl (0.90 g, 4.67 mmole) and HOBt·H₂O (0.63 g, 4.67 mmole) were added to the compound (1.1 g, 3.11 mmole) obtained in above [Step 3], subjected to a reaction under the same conditions, purified by MPLC (combiFlash NEXTGEN 300+), concentrated and dried to obtain a title compound (1.2 g).
¹H-NMR (DMSO-d⁶) 1.12(s, 6H), 2.02-2.04(m, 2H), 3.84(s, 3H), 4.53-4.55(m, 2H), 4.62(s, 1H), 7.46(s, 1H), 7.48(d, 1H), 7.66(s, 1H), 7.72(t, 1H), 7.82(d, 2H), 7.87(d, 1H), 7.91(s, 1H), 7.97(d, 1H), 7.98(s, 1H), 8.37(s, 1H), 8.62(s, 1H), 8.75(s, 1H)

### [Step 5] Preparation of 4-(2-(3-hydroxy-3-methylbutyl)-5-(3-sulfamoylbenzamide)-2H-indazol-6-yl)benzoic acid

Tetrahydrofuran (22 mL) and a 2 N-sodium hydroxide aqueous solution (11 mL) were added to the compound (1.1 g, 2.05 mmole) obtained in above [Step 4], and stirred at room temperature for three hours or more. After completion of a reaction, a 2 N-hydrochloric acid aqueous solution was added to adjust the pH to 4-5, concentrated, filtered, washed with purified water, and dried to obtain a title compound (0.81 g).
¹H-NMR (DMSO-d⁶) 1.12(s, 6H), 2.02-2.04(m, 2H), 4.53-4.55(m, 2H), 4.62(s, 1H), 7.46(s, 1H), 7.48(d, 1H), 7.66(s, 1H), 7.72(t, 1H), 7.82(d, 2H), 7.87(d, 1H), 7.91(s, 1H), 7.97(d, 1H), 7.98(s, 1H), 8.37(s, 1H), 8.62(s, 1H), 8.75(s, 1H), 10.2(s, 1H)

### [Step 6] Preparation of N-(2-(3-hydroxy-3-methylbutyl)-6-(4-((2,2,2-trifluoroethyl)carbamoyl)phenyl)-2H-indazol-5-yl)-3-sulfamoylbenzamide (Compound 42)

Under the same conditions as in [Step 4] of Example 11, 2,2,2-trifluoroethan-1-amine (0.04 g, 0.40 mmole), dichloromethane (2 mL), EDC·HCl (0.12 g, 0.61 mmole) and HOBt·H₂O (0.08 g, 0.61 mmole) were added to the compound (0.2 g, 0.40 mmole) obtained in above [Step 5], subjected to a reaction in the same manner, purified by MPLC (combiFlash NEXTGEN 300+), concentrated and dried to obtain a title compound (0.16 g).
¹H-NMR (DMSO-d⁶) 1.13(s, 6H), 2.04-2.05(m, 2H), 3.26(s, 2H), 4.01-4.04(m, 2H), 4.50-4.53(m, 3H), 7.40(s, 1H), 7.56(d, 1H), 7.59(s, 1H), 7.62(t, 1H), 7.72(s, 1H), 7.82(d, 2H), 7.91(t, 2H), 8.21(s, 1H), 8.44(s, 1H), 9.00(t, 1H), 10.20(s, 1H)
LC-MS (ESI, m/z) = 604.0 (M+H⁺)

### Example 43. N-(6-(4-((2-amino-2-oxoethyl)carbamoyl)phenyl)-2-(3-hydroxy-3-methylbutyl)-2H-indazol-5-yl)-3-nitrobenzamide

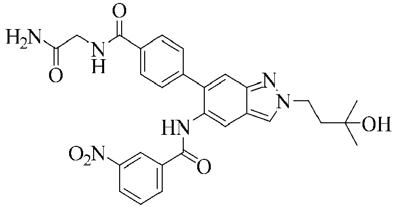

### [Step 5] Preparation of 4-(2-(3-hydroxy-3-methylbutyl)-5-(3-nitrobenzamido)-2H-indazol-6-yl)benzoic acid

Tetrahydrofuran (10 mL) and a 2 N-sodium hydroxide aqueous solution (5 mL) were added to the compound (0.5 g, 1 mmole) obtained in above [Step 4] of Example 41, and stirred at room temperature for three hours. After completion of a reaction, a 2 N-hydrochloric acid aqueous solution was added to adjust the pH to 4-5, concentrated, filtered, washed with purified water, and dried to obtain a title compound (0.3 g).
¹H-NMR (DMSO-d⁶) 1.13(s, 6H), 2.03(m, 2H), 4.50-4.53(m, 3H), 6.97(s, 1H), 7.32(s, 1H), 7.56(d, 2H), 7.59(s, 1H), 7.83(d, 2H), 8.16(d, 1H), 8.36(d, 1H), 8.45(s, 1H), 8.59(m, 2H), 10.29(s, 1H), 12.1(s, 1H)

### [Step 6] Preparation of N-(6-(4-((2-amino-2-oxoethyl)carbamoyl)phenyl)-2-(3-hydroxy-3-methylbutyl)-2H-indazol-5-yl)-3-nitrobenzamide (Compound 43)

Under the same conditions as in [Step 4] of Example 11, glycinamide hydrochloride (0.06 g, 0.51 mmole), dichloromethane (3 ml), EDC·HCl (0.15 g, 0.76 mmole) and HOBt·H₂O (0.1 g, 0.76 mmole) were added to the compound (0.3 g, 0.42 mmole) obtained in above [Step 5], subjected to a reaction in the same manner, purified by MPLC (combiFlash NEXTGEN 300+), and concentrated to obtain a title compound (0.2 g).
¹H-NMR (DMSO-d⁶) 1.13(s, 6H), 2.04-2.06(m, 2H), 3.75(d, 2H), 4.50-4.53(m, 3H), 6.97(s, 1H), 7.30(s, 1H), 7.54(d, 2H), 7.59(s, 1H), 7.74(d, 2H), 7.82(d, 2H), 8.16(d, 1H), 8.36(d, 1H), 8.45(s, 1H), 8.56-8.58(m, 2H), 10.29(s, 1H)
LC-MS (ESI, m/z) = 545.6 (M+H⁺)

### Example 44. N-(2-(3-hydroxy-3-methylbutyl)-6-(4-(methoxycarbamoyl)phenyl)-2H-indazol-5-yl)-3-sulfamoylbenzamide

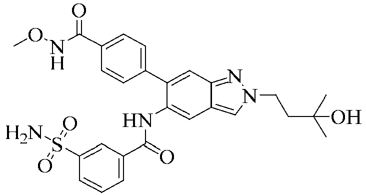

### [Step 4] Preparation of N-(2-(3-hydroxy-3-methylbutyl)-6-(4-(methoxycarbamoyl)phenyl)-2H-indazol-5-yl)-3-sulfamoylbenzamide (Compound 44)

Under the same conditions as in [Step 4] of Example 11, hydroxyamine-O-sulfonic acid (0.05 g, 0.40 mmole), dichloromethane (2 mL), EDC·HCl (0.12 g, 0.61 mmole) and HOBt·H₂O (0.08 g, 0.61 mmole) were added to the compound (0.2 g, 0.40 mmole) obtained in above [Step 5] of Example 42, subjected to a reaction in the same manner, purified by MPLC (combiFlash NEXTGEN 300+), concentrated and dried to obtain a title compound (0.12 g).
¹H-NMR (DMSO-d⁶) 1.14(s, 6H), 2.03(t, 2H), 3.61(s, 3H), 4.49-4.50(m, 3H), 7.36-7.40(m, 5H), 7.50-7.53(m, 2H), 7.70(s, 1H), 7.92-7.98(m, 3H), 8.24(s, 1H), 8.40(s, 1H), 9.60(s, 1H), 9.99(s, 1H)
LC-MS (ESI, m/z) = 552.0 (M+H⁺)

### Example 45. N-(2-(2-hydroxy-2-methylpropyl)-6-thiomorpholino-2H-indazol-5-yl)-3-nitrobenzamide

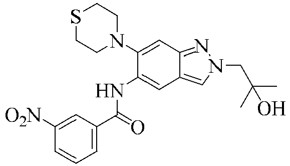

### [Step 1] Preparation of 4-(5-nitro-1H-indazol-6-yl)thiomorpholine

Under the same conditions as in [Step 2] of Example 33, thiomorpholine (20 mL), DIPEA (6.2 mL, 36.56 mmole) and DMSO (20 mL) were added to 6-bromo-5-nitro-1H-indazole (2.9 g, 12 mmole), subjected to a reaction in the same manner, and then purified by MPLC (combiFlash NEXTGEN 300+) to obtain a title compound (1.34 g).
¹H-NMR (DMSO-d⁶) 2.57(t, 4H), 3.37(t, 4H), 7.20(s 1H), 8.12(s, 1H), 8.30(s, 1H)

### [Step 2] Preparation of 2-methyl-1-(5-nitro-6-thiomorpholino-2H-indazol-2-yl)propan-2-ol

Under the same conditions as in [Step 1] of Example 1, the compound (1 g, 3.78 mmole) obtained in above [Step 1], potassium carbonate (2.1 g, 15.12 mmole), potassium iodide (0.1 g, 0.6 mmole), 1-chloro-2-methyl-2-propanol (0.5 mL, 5.67 mmole), and dimethylformamide (50 mL) were used and subjected to a reaction in the same manner, purified by MPLC (combiFlash NEXTGEN 300+), and concentrated to obtain a title compound (0.32 g).
¹H-NMR (DMSO-d⁶) 1.08(s, 6H), 2.51(m, 4H), 3.38(m, 4H), 4.23(t, 2H), 4.72(s, 1H), 7.32(s, 1H), 8.30(s, 1H), 8.54(s, 1H)

### [Step 3] Preparation of 1-(5-amino-6-thiomorpholino-2H-indazol-2-yl)-2-methylpropan-2-ol

Under the same conditions as in [Step 3] of Example 1, the compound (320 mg, 0.95 mmole) obtained in above [Step 2] was dissolved in ethyl acetate (20 mL) and hydrogenated with palladium on activated carbon (64 mg, 0.2 w/w). A reaction solution was filtered through celite and a filtrate was concentrated under reduced pressure to obtain a title compound (291 mg).
¹H-NMR (DMSO-d⁶) 1.08(s, 6H), 2.47(m, 4H), 3.28(m, 4H), 4.19(t, 2H), 4.62(s, 1H), 4.66(s, 2H), 7.29(s, 1H), 7.72(s, 1H), 8.04(s, 1H)

### [Step 4] Preparation of N-(2-(2-hydroxy-2-methylpropyl)-6-thiomorpholino-

### 2H-indazol-5-yl)-3-nitrobenzamide (Compound 45)

Under the same conditions as in [Step 4] of Example 11, the compound (97 mg, 0.317 mmole) obtained in above [Step 3], 3-nitrobenzoic acid (43 mg, 0.257 mmole), dimethylformamide (10 mL), EDC·HCl (165 mg, 0.95 mmole) and HOBt·H₂O (116 mg, 0.95 mmole) were used and subjected to a reaction in the same manner, purified by MPLC (combiFlash NEXTGEN 300+), and concentrated to obtain a title compound (20 mg).
¹H-NMR (DMSO-d⁶) 1.10(s, 6H), 2.59(m, 4H), 3.42(m, 4H), 4.31(t, 2H), 4.82(s, 1H), 7.18(s, 1H), 7.73(m, 1H), 8.30(s, 1H), 8.31(m, 2H), 8.37(m, 1H), 8.74(s, 1H), 10.05(s, 1H)
LC-MS (ESI, m/z) = 456.5 (M+H⁺)

### Example 46. N-(6-(4-carbamoylphenyl)-2-(3-hydroxy-3-methylbutyl)-2H-indazol-5-yl)-3-sulfamoylbenzamide

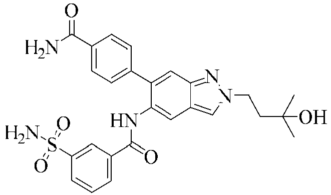

### [Step 6] Preparation of N-(6-(4-carbamoylphenyl)-2-(3-hydroxy-3-methylbutyl)-2H-indazol-5-yl)-3-sulfamoylbenzamide (Compound 46)

A solution of 7 N ammonia in methanol (20 mL) was added to the compound (0.2 g, 0.40 mmole) obtained in [Step 5] of Example 42, and stirred at 120°C for 24 hours or more. After completion of a reaction, a resulting product was cooled, concentrated, and then purified by MPLC (combiFlash NEXTGEN 300+) to obtain a title compound (0.08 g).
¹H-NMR (DMSO-d⁶) 1.14(s, 6H), 2.03(t, 2H), 4.50-4.52(m, 3H), 7.29(s, 1H), 7.40(s, 2H), 7.52(d, 2H), 7.58(s, 1H), 7.63(t, 1H), 7.72(s, 1H), 7.81(d, 2H), 7.89(s, 1H), 7.93(d, 2H), 8.21(s, 1H), 8.44(s, 1H), 10.10(s, 1H)
LC-MS (ESI, m/z) = 522.0 (M+H+)

### Example 47. Ethyl (4-(2-(3-hydroxy-3-methylbutyl)-5-(3-sulfamoylbenzamido)-2H-indazol-6-yl)benzoyl)-D-valinate

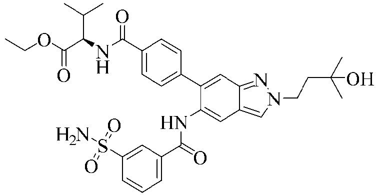

### [Step 6] Preparation of ethyl (4-(2-(3-hydroxy-3-methylbutyl)-5-(3-sulfamoylbenzamido)-2H-indazol-6-yl)benzoyl)-D-valinate (Compound 47)

L-valine ethylester hydrochloride (0.02 g, 0.40 mmole), dichloromethane (2 mL), EDC·HCl (0.12 g, 0.61 mmole) and HOBt·H₂O (0.08 g, 0.61 mmole) were added to the compound (0.2 g, 0.40 mmole) obtained in [Step 5] of Example 42, subjected to a reaction in the same manner, purified by MPLC (combiFlash NEXTGEN 300+), concentrated and dried to obtain a title compound (0.11 g).
¹H-NMR (DMSO-d⁶) 0.90(d, 3H), 0.93(d, 3H), 1.13-1.15(m, 9H), 2.04-2.05(t, 2H), 2.12-2.16(m, 1H), 4.45-4.49(m, 5H), 4.62-4.64(m, 1H), 7.40(s, 2H), 7.54(d, 2H), 7.58(s, 1H), 7.73(s, 1H), 7.83(d, 2H), 7.90(d, 2H), 7.96(d, 1H), 8.23(s, 1H), 8.44(s, 1H), 8.48(d, 1H), 10.10(s, 1H)
LC-MS (ESI, m/z) = 650.0 (M+H+)

### Example 48. Methyl 3-(5-(2-(3-hydroxy-3-methylbutyl)-5-(3-sulfamoylbenzamido)-2H-indazol-6-yl)nicotinamido)propanoate

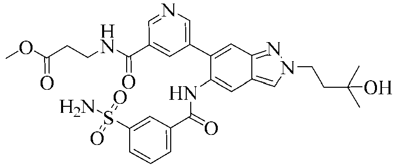

### [Step 2] Preparation of methyl 5-(2-(3-hydroxy-3-methylbutyl)-5-nitro-2H-indazol-6-yl)nicotinate

Under the same conditions as in [Step 2] of Example 1, the compound (0.5 g, 1.52 mmole) obtained in [Step 1] of Example 4, sodium carbonate (0.81 g, 7.61 mmole), tetrakis(triphenylphosphine)palladium (0.09 g, 0.08 mmole), 4-methyl 5-(4,4,5,5-tetramethyl-1,3,2-dioxaborolan-2-yl)nicotinate (0.60 g, 2.29 mmole), 1,4-dioxane (10 mL) and purified water (1 mL) were used and subjected to a reaction in the same manner, purified by MPLC (combiFlash NEXTGEN 300+), and concentrated to obtain a title compound (0.5 g).
¹H-NMR (DMSO-d⁶) 1.14(s, 6H), 2.04(t, 2H), 3,73(s, 3H), 4.50-4.54(m, 3H), 7.72(s, 1H), 8.15(s, 1H), 8.21(d, 1H), 8.37(s, 1H), 8.48(s, 1H), 8.68(d, 1H)

### [Step 3] Preparation of methyl 5-(5-amino-2-(3-hydroxy-3-methylbutyl)-2H-indazol-6-yl)nicotinate

Under the same conditions as in [Step 3] of Example 1, the compound (0.5 g, 1.30 mmole) obtained in above [Step 2] was dissolved in ethyl acetate (10 mL) and hydrogenated by adding palladium on activated carbon (0.10 g, 0.093 mmole). A resulting product was purified by MPLC (combiFlash NEXTGEN 300+) to obtain a title compound (0.4 g).
¹H-NMR (DMSO-d⁶) 1.09(s, 6H), 1.99(t, 2H), 3,68(s, 3H), 4.45-4.50(m, 3H), 4.59(s, 2H), 7.68(s, 1H), 8.10(s, 1H), 8.16(d, 1H), 8.32(s, 1H), 8.42(s, 1H), 8.62(d, 1H)

### [Step 4] Preparation of methyl 5-(2-(3-hydroxy-3-methylbutyl)-5-(3-sulfamoylbenzamido)-2H-indazol-6-yl)nicotinate

Under the same conditions as in [Step 4] of Example 1, the compound (0.4 g, 1.13 mmole) obtained in above [Step 3], 3-sulfamoylbenzoic acid (0.23 g, 1.13 mmole), dimethylformamide (20 mL), HATU (0.86 g, 2.26 mmole), and DIPEA (0.79 mL, 4.51 mmole) were used and subjected to a reaction in the same manner, purified by MPLC (combiFlash NEXTGEN 300+), and concentrated to obtain a title compound (210 mg).
¹H-NMR (DMSO-d⁶) 1.14(s, 6H), 2.04(t, 2H), 3,73(s, 3H), 4.50-4.54(m, 3H), 7.72(s, 1H), 7.78(t, 1H), 7.82(s, 2H), 8.02(d, 1H), 8.15(s, 1H), 8.34(s, 1H), 8.37(s, 1H), 8.39(s, 1H), 8.48(s, 1H), 8.68(d, 1H), 8.94(s, 1H), 9.94(s, 1H)

### [Step 5] Preparation of 5-(2-(3-hydroxy-3-methylbutyl)-5-(3-sulfamoylbenzamido)-2H-indazol-6-yl)nicotic acid

The compound (0.5 g, 0.93 mmole) obtained in above [Step 4] was dissolved in tetrahydrofuran (5 mL), methanol (2.5 mL) and purified water (2.5 mL), and then potassium hydroxide (0.21 g, 3.74 mmole) was added thereto and stirred at 60°C for three hours. After completion of a reaction, a resulting product was cooled to room temperature and concentrated under reduced pressure. Distilled water (10 mL) was added thereto, a resulting mixture was washed with ethyl acetate (10 mL), the pH of an aqueous layer was adjusted to 4 using 1 N hydrochloric acid, and a precipitated solid was filtered and dried to obtain a title compound (0.4 g).
¹H-NMR (DMSO-d⁶) 1.14(s, 6H), 2.04(t, 2H), 4.50-4.54(m, 3H), 7.72(s, 1H), 7.78(t, 1H), 7.82 (s, 2H), 8.02(d, 1H), 8.15(s, 1H), 8.34(s, 1H), 8.37(s, 1H), 8.39(s, 1H), 8.48(s, 1H), 8.68(d, 1H), 8.94(s, 1H), 9.94(s, 1H), 10.21(s, 1H)

### [Step 6] Preparation of methyl 3-(5-(2-(3-hydroxy-3-methylbutyl)-5-(3-sulfamoylbenzamido)-2H-indazol-6-yl)nicotinamido)propanoate (Compound 48)

Under the same conditions as in [Step 4] of Example 1, methyl 3-aminopropanoate hydrochloride (0.11 g, 0.76 mmole), dimethylformamide (10 mL), HATU (0.58 g, 1.53 mmole) and DIPEA (0.53 mL, 3.06 mmole) were added to the compound (0.4 g, 0.76 mmole) obtained in above [Step 5], subjected to a reaction in the same manner, and then purified by MPLC (combiFlash NEXTGEN 300+) to obtain a title compound (0.4 g).
¹H-NMR (DMSO-d⁶) 1.13(s, 6H), 2.06(t, 2H), 2.64(t, 2H), 2.85(t, 2H), 3.56(s, 3H), 4.50(s, 1H), 4.53(t, 2H), 7.39(d, 2H), 7.70(s, 1H), 7.74(s, 1H), 7.93(d, 2H), 7.97(s, 1H), 8.15(s, 1H), 8.27(s, 1H), 8.46(s, 1H), 8.68(s, 1H), 8.73(d, 1H), 8.82(d, 1H), 10.35(s, 1H)
LC-MS (ESI, m/z) = 609.4 (M+H⁺)

### Example 49. N-(6-(4-((furan-3-ylmethyl)carbamoyl)phenyl)-2-(3-hydroxy-3-methylbutyl)-2H-indazol-5-yl)isophthalamide

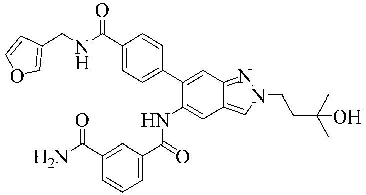

### [Step 2] Preparation of N-(furan-3-ylmethyl)-4-(2-(3-hydroxy-3-methylbutyl)-5-nitro-2H-indazol-6-yl)benzamide

Under the same conditions as in [Step 2] of Example 1, the compound (1.0 g, 3.05 mmole) obtained in [Step 1] of Example 4, sodium carbonate (1.61 g, 15.24 mmole), tetrakis(triphenylphosphine)palladium (0.18 g, 0.15 mmole), and N-(furan-3-ylmethyl)-4-(4,4,5,5-tetramethyl-1,3,2-dioxaborolan-2-yl)benzamide (1.5 g, 4.57 mmole) were used and subjected to a reaction in the same manner, purified by MPLC (combiFlash NEXTGEN 300+), and concentrated to obtain a title compound (1.18 g).
¹H-NMR (DMSO-d⁶) 1.13(s, 6H), 2.03(t, 2H), 4.48-4.53(m, 5H), 6.27(d, 1H), 6.36(d, 1H), 7.23(s, 1H), 8.37(s, 1H), 8.45(s, 1H), 8.71(s, 1H), 9.13 (t, 1H)

### [Step 3] Preparation of 4-(5-amino-2-(3-hydroxy-3-methylbutyl)-2H-indazol-6-yl)-N-(furan-3-ylmethyl)benzamide

Under the same conditions as in [Step 3] of Example 1, palladium on activated carbon (0.2 g, 0.2 w/w) and ethyl acetate (20 mL) were added to the compound (1.0 g, 2.23 mmole) obtained in above [Step 2] and hydrogenated. A reaction mixture was filtered through celite and a filtrate was concentrated to obtain a title compound (0.77 g).
¹H-NMR (DMSO-d⁶) 1.07(s, 6H), 1.97(t, 2H), 4.42-4.48(m, 5H), 4.56 (s, 2H), 6.21(d, 1H), 6.30(d, 1H), 7.17(s, 1H), 8.30(s, 1H), 8.38(s, 1H), 8.63(s, 1H), 9.07(t, 1H)

### [Step 4] Preparation of N-(6-(4-((furan-3-ylmethyl)carbamoyl)phenyl)-2-(3-hydroxy-3-methylbutyl)-2H-indazol-5-yl)isophthalamide (Compound 49)

Under the same conditions as in [Step 4] of Example 1, the compound (0.20 g, 0.48 mmole) obtained in above [Step 3], 3-carbamoylbenzoic acid (0.08 g, 0.48 mmole), dimethylformamide (2 mL), HATU (0.36 g, 0.96 mmole) and DIPEA (0.33 mL, 1.91 mmole) were used and subjected to a reaction in the same manner, purified by MPLC (combiFlash NEXTGEN 300+), and concentrated to obtain a title compound (0.2 g).
¹H-NMR (DMSO-d⁶) 1.13(s, 6H), 2.04(m, 2H), 4.41(d, 2H), 4.47-4.50(m, 3H), 5.72(s, 1H), 6.21(d, 1H), 6.34(s, 1H), 7.40(s, 1H), 7.48(t, 1H), 7.52(d, 2H), 7.54(s, 1H), 7.57(s, 1H), 7.72(s, 1H), 7.82-7.88(m, 2H), 7.98(d, 1H), 8.00(s, 1H), 8.27(s, 1H), 8.43(s, 1H), 8.89(t, 1H), 9.96(s, 1H)
LC-MS (ESI, m/z) = 566.1 (M+H⁺)

### Example 50. N-(2-(2-hydroxy-2-methylpropyl)-6-(thiophen-3-yl)-2H-indazol-5-yl)-3-nitrobenzamide

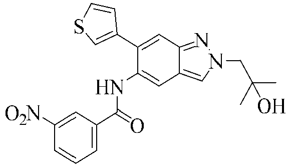

### [Step 1] Preparation of 1-(6-bromo-5-nitro-2H-indazol-2-yl)-2-methylpropan-2-ol

Dimethylformamide (20 mL), potassium carbonate (4.56 g, 27.30 mmole), potassium iodide (0.14 g, 0.83 mmole), and 1-chloro-2-methylpropan-2-ol (1.312 g, 12.39 mmole) were added to 6-bromo-5-nitro-2H-indazole (2 g, 8.26 mmole) and stirred at 100°C for five hours. After a reaction, ethyl acetate (50 mL) was added thereto and washed with purified water. A resulting product was dried over magnesium sulfate and filtered, and then a filtrate was concentrated and purified by MPLC (combiFlash NEXTGEN 300+) to obtain a title compound (1.16 g).
¹H-NMR(DMSO-d⁶) 1.09(s, 6H), 4.32(s, 2H), 4.85(s, 1H), 7.81(s, 1H), 7.82(s, 1H), 8.60(s, 1H)

### [Step 2] Preparation of 2-methyl-1-(5-nitro-6-(thiophen-3-yl)-2H-indazol-2-yl)propan-2-ol

Under the same conditions as in [Step 2] of Example 1, sodium carbonate (1.72 g, 16.23 mmole), tetrakis(triphenylphosphine)palladium (0.19 g, 0.16 mmole), 4,4,5,5-tetramethyl-2-(thiophen-3-yl)-1,3,2-dioxoborane (1.02 g, 4.875 mmole), 1,4-dioxane (15 mL) and purified water (1.5 mL) were added to the compound (1.02 g, 3.25 mmole) obtained in above [Step 1], subjected to a reaction in the same manner, purified by MPLC (combiFlash NEXTGEN 300+), and concentrated to obtain a title compound (0.79 g).
¹H-NMR(DMSO-d⁶) 1.09(s, 6H), 4.32(s, 2H), 4.85(s, 1H), 7.40(d, 1H), 7.60(s, 1H), 7.81(s, 1H), 7.82(s, 1H), 7.91(s, 1H), 8.60(s, 1H)

### [Step 3] Preparation of 1-(5-amino-6-(thiophen-3-yl)-2H-indazol-2-yl)-2-methylpropan-2-ol

Under the same conditions as in [Step 3] of Example 2, the compound (0.36 g, 2 mmole) obtained in above [Step 2] was dissolved in ethyl acetate (5 mL) and hydrogenated by adding palladium on activated carbon (0.128 g, 0.11 mmole). A reaction mixture was filtered through celite and a filtrate was concentrated to obtain a title compound (0.26 g).
¹H-NMR (DMSO-d⁶) 1.09(s, 6H), 4.32(s, 2H), 4.80(s, 2H), 4.85(s, 1H), 7.22(s, 1H), 7.40(d, 1H), 7.42(s, 1H), 7.60(s, 1H), 7.81(s, 1H), 7.91(s, 1H)

### [Step 4] Preparation of N-(2-(2-hydroxy-2-methylpropyl)-6-(thiophen-3-yl)-2H-indazol-5-yl)-3-nitrobenzamide (Compound 50)

Under the same conditions as in [Step 4] of Example 1, 3-nitrobenzoic acid (0.23 g, 1.39 mmole), dimethylformamide (10 mL), HATU (1.06 g, 2.78 mmole), and DIPEA (0.97 mL, 5.57 mmole) were added to the compound (0.4 g, 1.39 mmole) obtained in above [Step 3], subjected to a reaction in the same manner, purified by MPLC (combiFlash NEXTGEN 300+), and concentrated to obtain a title compound (0.3 g).
¹H-NMR (DMSO-d⁶) 1.10(s, 6H), 4.34(s, 2H), 4.86(s, 1H), 7.29(d, 1H), 7.50(m, 1H), 7.59(s, 1H), 7.67(s, 1H), 7.76-7.79(m, 2H), 8.24(d, 1H), 8.32(s, 1H), 8.37(d, 1H), 8.62(s, 1H)
LC-MS (ESI, m/z) = 437.2 (M+H⁺)

### Example 51. (R)-5-(2-(3-hydroxy-3-methylbutyl)-5-(3-sulfamoylbenzamido)-2H-indazol-6-yl)-N-(1-(methylamino)-1-oxopropan-2-yl)nicotinamide

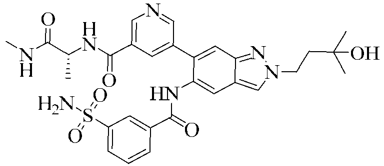

### [Step 7] Preparation of (R)-5-(2-(3-hydroxy-3-methylbutyl)-5-(3-sulfamoylbenzamido)-2H-indazol-6-yl)-N-(1-(methylamino)-1-oxopropan-2-yl)nicotinamide (Compound 51)

Under the same conditions as in [Step 4] of Example 11, (R)-2-amino-N-methylpropanamide (0.13 g, 0.94 mmole), dichloromethane (5 mL), EDC·HCl (0.27 g, 1.41 mmole), and HOBt·H₂O (0.19 g, 1.41 mmole) were added to the compound (0.5 g, 0.94 mmole) obtained in [Step 6] of Example 48, subjected to a reaction in the same manner, purified by MPLC (combiFlash NEXTGEN 300+), concentrated and dried to obtain a title compound (0.41 g).
¹H-NMR (DMSO-d⁶) 1.14(s, 6H), 1.15(d, 3H), 2.04(t, 2H), 2.65(d, 3H), 4.50-4.54(m, 3H), 4.62-4.65(m, 1H), 7.72(s, 1H), 7.78(t, 1H), 7.82 (s, 2H), 8.02(d, 1H), 8.15(s, 1H), 8.34(s, 1H), 8.37(s, 1H), 8.39(s, 1H), 8.48(s, 1H), 8.68(d, 1H), 8.94(s, 1H), 9.21(d, 1H), 9.46(m, 1H), 9.94(s, 1H)
LC-MS (ESI, m/z) = 608.0 (M+H⁺)

### Example 52. N-(1-(2-(methylsulfonyl)ethyl)-6-morpholino-1H-indazol-5-yl)-3-nitrobenzamide

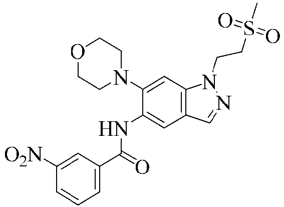

### [Step 1] Preparation of 6-bromo-1-(2-(methylsulfonyl)ethyl)-5-nitro-1H-indazole

Under the same conditions as in [Step 1] of Example 1, 2-bromoethyl methyl sulfone (5.8 g, 31 mmole), potassium carbonate (5.7 g, 41.32 mmole), potassium iodide (257 mg, 1.55 mmole), and dimethylformamide (50 mL) were added to 6-bromo-5-nitro-2H-indazole (5 g, 20.66 mmole), subjected to a reaction in the same manner, purified by MPLC (combiFlash NEXTGEN 300+), and concentrated to obtain a title compound (3.5 g).
¹H-NMR (DMSO-d⁶) 2.90(s, 3H), 3.73(t, 2H), 4.76(t, 2H), 7.92(s, 1H), 8.12(s, 1H), 8.36(s, 1H)

### [Step 2] Preparation of 4-(2-(2-(methylsulfonyl)ethyl)-5-nitro-2H-indazol-6-yl)morpholine

Under the same conditions as in [Step 1] of Example 2, morpholine (10 mL) was added to the compound (3.5 g, 10 mmole) obtained in above [Step 1], subjected to a reaction in the same manner, purified by MPLC (combiFlash NEXTGEN 300+), and concentrated to obtain a title compound (1.3 g).
¹H-NMR (DMSO-d⁶) 2.86(s, 3H), 2.94(m, 4H), 3.59(m, 4H), 3.76(t, 2H), 4.78(t, 2H), 7.91(s, 1H), 8.09(s, 1H), 8.29(s, 1H)

### [Step 3] Preparation of 2-(2-(methylsulfonyl)ethyl)-6-morpholino-2H-indazol-5-amine

Under the same conditions as in [Step 3] of Example 1, the compound (1.3 g, 4 mmole) obtained in above [Step 2] was dissolved in ethyl acetate (100 mL) and hydrogenated by adding palladium on activated carbon (109 mg, 0.2 w/w). A reaction solution was filtered through celite and a filtrate was concentrated under reduced pressure to obtain a title compound (1.15 g).
¹H-NMR (DMSO-d⁶) 2.82(s, 3H), 2.94(m, 4H), 3.59(m, 4H), 3.63(t, 2H), 4.30(s, 2H), 4.60(t, 2H), 7.72(s, 1H), 8.01(s, 1H), 8.20(s, 1H)

### [Step 4] Preparation of N-(1-(2-(methylsulfonyl)ethyl)-6-morpholino-1H-indazol-5-yl)-3-nitrobenzamide (Compound 52)

Under the same conditions as in [Step 4] of Example 11, the compound (136 mg, 0.42 mmole) obtained in above [Step 3], 3-nitrobenzoic acid (64 mg, 0.383 mmole), dimethylformamide (10 mL), EDC·HCl (0.22 g, 1.15 mmole) and HOBt·H₂O (0.155 g, 1.15 mmole) were used and subjected to a reaction in the same manner, purified by MPLC (combiFlash NEXTGEN 300+), and concentrated to obtain a title compound (44 mg).
¹H-NMR (DMSO-d⁶) 2.90(s, 3H), 2.95(m, 4H), 3.72(t, 2H), 3.78(m, 4H), 4.79(t, 2H), 7.60(s, 1H), 7.87(t, 1H), 8.07(s, 1H), 8.31(s, 1H), 8.38(d, 1H), 8.43(d, 1H), 8.72(s, 1H), 10.01(s, 1H)
LC-MS (ESI, m/z) = 474.5 (M+H⁺)

### Example 53. N-(2-(2-(methylsulfonyl)ethyl)-6-morpholino-2H-indazol-5-yl)-3-sulfamoylbenzamide

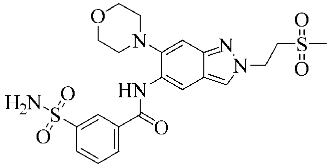

### [Step 4] Preparation of N-(2-(2-(methylsulfonyl)ethyl)-6-morpholino-2H-indazol-5-yl)-3-sulfamoylbenzamide (Compound 53)

Under the same conditions as in [Step 4] of Example 1, the compound (136 mg, 0.42 mmole) obtained in [Step 3] of Example 13, 3-sulfamoylbenzoic acid (93 mg, 0.462 mmole), dimethylformamide (10 mL), HATU (217 mg, 0.63 mmole), and DIPEA (0.2 mL, 1.68 mmole) were used and subjected to a reaction in the same manner, purified by MPLC (combiFlash NEXTGEN 300+), and concentrated to obtain a title compound (10 mg).
¹H-NMR (DMSO-d⁶) 2.90(s, 3H), 2.98(m, 4H), 3.73(t, 2H), 3.79(m, 4H), 4.78(t, 2H), 7.59(s, 1H), 7.69(s, 2H), 7.80(t, 1H), 7.99(d, 1H), 8.07(s, 1H), 8.18(m, 1H), 8.33(s, 1H), 8.38(s, 1H), 9.91(s, 1H)
LC-MS (ESI, m/z) = 508.6 (M+H⁺)

### <Experimental Example> Evaluation of Physiological Efficacy

### Experimental Example 1. Experiment on IRAK4 enzyme activity inhibition

An experiment on IRAK4 enzyme activity inhibition of the compound of the present invention was conducted.

An IRAK4 enzyme kit (purchased from Promega, product number: V2621) was used to dilute a mixed solution between 0.1 µg/µL of myelin basic protein (MBP), which is an enzyme-reactive substrate, and 1.25 mM of ATP with a reaction buffer (40 mM Tris-HCl, pH 7.5; 20 mM MgCl₂; 0.1 mg/mL BSA; 50 µM DTT) according to the instructions, so as to dispense 2 µL in a 384-well white plate, and then to add 1 µL of a test compound, which was gradient diluted starting from 1 mM, to a 384-well plate. After that, an IRAK4 enzyme was diluted with a reaction buffer to a final concentration of 5 ng/µL, then 2 µL/well was dispensed into a 384-well white plate and reacted at 37°C for 60 minutes. After completion of a reaction, an ADP-GloTM enzyme test kit (purchased from Promega, product number: V9102) was used to add 5 µL of ADP-Glo reaction reagent and cause a reaction at 37°C for 40 minutes, and then 10 µL of enzyme chromogenic reagent was added again to measure a luminescence signal using equipment for measuring absorbance (THECAN, Microplate Reader). An activity inhibition rate was calculated from a measured value, and a 50% inhibition concentration (IC₅₀ value) was analyzed through a non-linear regression analysis based on the % inhibition for each concentration of the test compound, and is shown in Table 2.

**[Table 2]**

| | **IRAK4 %enzyme inhibition (1 uM)** | **IRAK4 %enzyme inhibition (0.1 uM)** | **IRAK4 enzyme IC₅₀(nM)** |
|---|---|---|---|
| **Example 1** | | 19.1 | |
| **Example 2** | 86.5 | 72.7 | 56.7 |
| **Example 3** | 94.4 | 81.7 | 0.2 |
| **Example 4** | 67.4 | 42.1 | |
| **Example 5** | 35.4 | 27.9 | |
| **Example 6** | 67 | 35.5 | |
| **Example 7** | 57.7 | 28.1 | |
| **Example 8** | 50.3 | 21.7 | |
| **Example 9** | 79.5 | 56.9 | |
| **Example 10** | 83.4 | 49.5 | 99.7 |
| **Example 11** | 56.9 | 40.4 | |
| **Example 12** | | 31.5 | |
| **Example 13** | 19.2 | 18.6 | |
| **Example 14** | 35.6 | 21.1 | |
| **Example 15** | 95.2 | 88.8 | 3.7 |
| **Example 16** | 71 | 48.8 | |
| **Example 17** | | 76.7 | 329.6 |
| **Example 18** | 65.6 | 54.8 | |
| **Example 19** | 90.1 | 84.7 | 31.7 |
| **Example 20** | | 89.6 | 15.5 |
| **Example 21** | 97.9 | 89.3 | 1.5 |
| **Example 22** | | 88.3 | 40.3 |
| **Example 23** | | 88 | 10.5 |
| **Example 24** | | 83.9 | 13.1 |
| **Example 25** | | 81.3 | 78.5 |
| **Example 26** | | 71.3 | 191.6 |
| **Example 27** | | 57.3 | |
| **Example 28** | 89.9 | 56.1 | |
| **Example 29** | | 93.3 | 0.88 |
| **Example 30** | | 82.8 | 0.88 |
| **Example 31** | | 88 | 13.4 |
| **Example 32** | | 87.1 | 2 |
| **Example 33** | 95.6 | 71.3 | 22.1 |
| **Example 34** | 88.2 | 69.4 | |
| **Example 35** | | 64.6 | |
| **Example 36** | | 55.2 | |
| **Example 37** | | 52.2 | |
| **Example 38** | | 87.9 | 5.1 |
| **Example 39** | | 69.7 | |
| **Example 40** | | 69.7 | |
| **Example 41** | | 69 | |
| **Example 42** | | 65.1 | |
| **Example 43** | | 63 | |
| **Example 44** | | 61.9 | |
| **Example 45** | 75 | 59.6 | |
| **Example 46** | | 57.5 | |
| **Example 47** | | 56.8 | |
| **Example 48** | | 56.7 | |
| **Example 49** | | 55.4 | |
| **Example 50** | 68.8 | 55.1 | |
| **Example 51** | | 48.8 | |
| **Example 52** | | 21.2 | |
| **Example 53** | | 2.7 | |

As can be confirmed from above Table 2, the compound of the present invention may exhibit an excellent IRAK4 inhibitory activity.

### Experimental Example 2. Experiment on IRAK1 enzyme activity inhibition

An experiment on IRAK1 enzyme activity inhibition of the compound of the present invention was conducted.

An IRAK1 enzyme kit (purchased from Promega, product number: VA7477) was used to dilute a mixed solution between 0.1 µg/µL of protein kinase B (AKT), which is an enzyme-reactive substrate, and 1.25 mM of ATP with a reaction buffer (40 mM Tris-HCl, pH 7.5; 20 mM MgCl₂; 0.1 mg/mL BSA; 50 µM DTT) according to the instructions, so as to dispense 2 µL in a 384-well white plate, and then to add 1 µL of a test compound, which was gradient diluted starting from 1 mM, to a 384-well white plate. After that, an IRAK1 enzyme was diluted with a reaction buffer to a final concentration of 5 ng/µL, then 2 µL/well was dispensed into a 384-well white plate and reacted at 37°C for 60 minutes. After completion of a reaction, an ADP-GloTM enzyme test kit (purchased from Promega, product number: V9102) was used to add 5 µL of ADP-Glo reaction reagent and cause a reaction at 37°C for 40 minutes, and then 10 µL of enzyme chromogenic reagent was added again to measure a luminescence signal using equipment for measuring absorbance (THECAN, Microplate Reader). An activity inhibition rate was calculated from a measured value, and a 50% inhibition concentration (IC₅₀ value) was analyzed through a non-linear regression analysis based on the % inhibition for each concentration of the test compound, and is shown in Table 3.

**[Table 3]**

| | **IRAK1 %enzyme inhibition (1 uM)** | **IRAK1 %enzyme inhibition (0.1 uM)** | **IRAK1 enzyme IC₅₀(uM)** |
|---|---|---|---|
| **Example 1** | | 42.40 | |
| **Example 2** | | 32.10 | |
| **Example 3** | | | 2.60 |
| **Example 13** | 0.00 | 1.30 | |
| **Example 14** | 0.00 | | |
| **Example 15** | 66.50 | 28.50 | |
| **Example 16** | 17.20 | | |
| **Example 17** | 63.40 | 33.10 | |
| **Example 18** | 23.20 | | |
| **Example 19** | 38.70 | | |
| **Example 20** | 57.00 | 16.50 | |
| **Example 21** | 82.40 | 67.20 | 0.07 |
| **Example 22** | 47.20 | 30.30 | |
| **Example 23** | 45.30 | 26.00 | |
| **Example 24** | 45.50 | 26.90 | |
| **Example 25** | 54.60 | 24.90 | |
| **Example 26** | 40.20 | 31.70 | |
| **Example 27** | 25.30 | 17.60 | |
| **Example 28** | 9.10 | | |
| **Example 29** | | 54.20 | |
| **Example 30** | | 8.60 | |
| **Example 31** | 66.70 | 30.60 | |
| **Example 32** | 52.00 | 17.50 | |
| **Example 33** | 39.30 | 24.00 | |
| **Example 34** | 17.40 | 4.60 | |
| **Example 35** | 60.80 | 33.10 | |
| **Example 36** | 12.90 | 5.70 | |
| **Example 37** | 31.00 | 26.30 | |
| **Example 38** | | 4.60 | |
| **Example 39** | 35.20 | 23.00 | |
| **Example 40** | 11.20 | 6.50 | |
| **Example 41** | | 0.40 | |
| **Example 43** | | 1.70 | |
| **Example 45** | 0.00 | | |
| **Example 46** | | 3.20 | |
| **Example 47** | | 1.30 | |
| **Example 48** | | 39.70 | |
| **Example 49** | 31.60 | 27.70 | |
| **Example 50** | 9.60 | | |
| **Example 51** | | 0.00 | |
| **Example 52** | | 0.00 | |
| **Example 53** | | 13.90 | |

As can be confirmed from above Table 3, the compound of the present invention may exhibit an excellent IRAK1 inhibitory activity.

### Experimental Example 3: Experiment on cell-based inflammatory cytokine secretion inhibition

An experiment on inflammatory cytokine secretion inhibition of the compound of the present invention was conducted.

PBMC (LONZA, peripheral blood mononuclear cells) isolated from human whole blood was dispensed into a 96-well plate at a density of 1.0 X 10⁵ cells/well, and the cells were stabilized for 20 hours. After that, a test compound diluted at each of four concentrations was added to a Roswell Park Memorial Institute (RPMI) medium, reacted for one hour, treated with lipopolysaccharide (LPS) to reach a final concentration of 1 µg/mL, and further reacted for one hour. hTNF-α, which is an inflammatory cytokine, was measured by an enzyme linked immunosorbent assay using an ELISA kit from Goma Biotech. A cell supernatant, a standard drug, and an antibody were added to an ELISA-plate and reacted for four hours in total, after which the plate was washed, reacted with streptavidin-HRP for 30 minutes, and washed again. After that, a TMB substrate was added and reacted in a dark place, and when a color was sufficiently developed, a stop solution was added to stop the reaction. hTNF-α was measured by measuring absorbance at 450 nM using equipment for absorption measurement (Microplate Reader from BioTeK). An activity inhibition rate was calculated from a measured absorbance value, a % inhibition for each concentration of the test compound was calculated, a 50% inhibition concentration (IC₅₀ value) was analyzed through a non-linear regression analysis, and the results are shown in Table 4.

**[Table 4]**

| | **hPBMC TNFα IC₅₀_mean (nM)** |
|---|---|
| **Example 2** | 525.9 |
| **Example 3** | 51.8 |
| **Example 15** | 575.0 |
| **Example 21** | 11.9 |
| **Example 23** | 601.2 |
| **Example 29** | 20.4 |
| **Example 32** | 113.2 |

As can be confirmed from above Table 4, the compound of the present invention may exhibit an excellent inflammatory cytokine secretion inhibitory effect.

While the present invention has been described in detail above, it is apparent to those skilled in the art that such detailed descriptions are set forth to illustrate exemplary embodiments only, but are not construed to limit the scope of the present invention. Thus, it should be understood that the substantial scope of the present invention is defined by the accompanying claims and equivalents thereto.

## Claims

1. A compound represented by chemical formula I, a stereoisomer thereof, a pharmaceutically acceptable salt thereof, or a hydrate or solvate thereof: 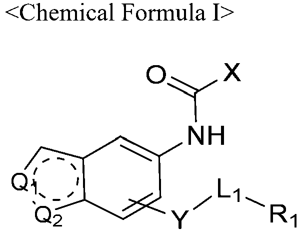 wherein in above chemical formula I,
X is phenyl or pyridinyl;
at least one H of X is substituted with halogen, C1-C6 alkyl, C(=O)Ra, NH₂, NO₂, S(=O)₂Rb, 5- to 12-membered heteroaryl including one to three heteroatoms independently selected from the group consisting of N, O and S in a ring;
Ra and Rb are each independently C1-C6 alkyl, NH₂, or 3- to 12-membered heterocycloalkyl including one to three heteroatoms independently selected from the group consisting of N, O and S in a ring;
Y is a single bond, 6- to 14-membered arylene, 3- to 12-membered heterocycloalkylene including one to three heteroatoms independently selected from the group consisting of N, O and S in a ring, or 5- to 12-membered heteroarylene including one to three heteroatoms independently selected from the group consisting of N, O and S in a ring;
L₁ is a single bond, C1-C6 alkylene, C(=O), C(=O)NH, C(=O)NH-(C1-C6 alkylene), C(=O)NH-(C1-C6 alkylene)-C(=O)O, or C(=O)NH-(C1-C6 alkylene)-C(=O)NH;
R₁ is H, halogen, C1-C6 alkyl, C1-C6 alkoxy, OH, CF₃, NRcRd, 3- to 12-membered heterocycloalkyl including one to three heteroatoms independently selected from the group consisting of N, O and S in a ring, or 5- to 12-membered heteroaryl including one to three heteroatoms independently selected from the group consisting of N, O and S in a ring;
Rc and Rd are each independently H or C1-C6 alkyl;
Q₁ and Q₂ are each independently N or N-L₂-R₂, in which Q₁ and Q₂ are not simultaneously N;
when Q₂ is N, 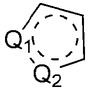 is 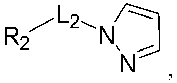 and when Q₁ is N, 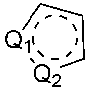 is 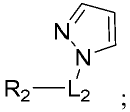
L₂ is C1-C6 alkylene or (C1-C6 alkylene)-O-(C1-C6 alkylene);
R₂ is 3- to 12-membered heterocycloalkyl including one to three heteroatoms independently selected from the group consisting of N, O and S in a ring, C1-C6 alkoxy, CONH₂, NReRf, SO₂Rg or OH;
Re, Rf and Rg are each independently H or C1-C6 alkyl.

2. The compound represented by chemical formula I, the stereoisomer thereof, the pharmaceutically acceptable salt thereof, or the hydrate or solvate thereof of claim 1,
wherein in above chemical formula I,
X is phenyl or pyridinyl;
at least one H of X is substituted with halogen, C(=O)Ra, NH₂, NO₂, S(=O)₂Rb, 5-to 12-membered heteroaryl including one to three heteroatoms independently selected from the group consisting of N, O and S in a ring;
Ra and Rb are each independently NH₂, or 3- to 12-membered heterocycloalkyl including one to three heteroatoms independently selected from the group consisting of N, O and S in a ring;
Y is a single bond, 6- to 14-membered arylene, 3- to 12-membered heterocycloalkylene including one to three heteroatoms independently selected from the group consisting of N, O and S in a ring, or 5- to 12-membered heteroarylene including one to three heteroatoms independently selected from the group consisting of N, O and S in a ring;
L₁ is a single bond, C1-C6 alkylene, C(=O), C(=O)NH, C(=O)NH-(C1-C6 alkylene), C(=O)NH-(C1-C6 alkylene)-C(=O)O, or C(=O)NH-(C1-C6 alkylene)-C(=O)NH;
R₁ is H, halogen, C1-C6 alkyl, C1-C6 alkoxy, OH, CF₃, NRcRd, 3- to 12-membered heterocycloalkyl including one to three heteroatoms independently selected from the group consisting of N, O and S in a ring, or 5- to 12-membered heteroaryl including one to three heteroatoms independently selected from the group consisting of N, O and S in a ring;
Rc and Rd are each independently C1-C6 alkyl;
Q₁ and Q₂ are each independently N or N-L₂-R₂, in which Q₁ and Q₂ are not simultaneously N;
when Q₂ is N, 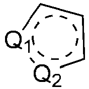 is 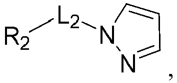 and when Q₁ is N, 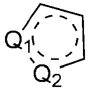 is 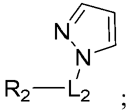
L₂ is C1-C6 alkylene or (C1-C6 alkylene)-O-(C1-C6 alkylene);
R₂ is 3-to 12-membered heterocycloalkyl including one to three heteroatoms independently selected from the group consisting of N, O and S in a ring, C1-C6 alkoxy, CONH₂, NReRf, SO₂Rg or OH;
Re, Rf and Rg are each independently C1-C6 alkyl.

3. The compound represented by chemical formula I, the stereoisomer thereof, the pharmaceutically acceptable salt thereof, or the hydrate or solvate thereof of claim 1,
wherein in above chemical formula I,
X is phenyl or pyridinyl;
at least one H of X is substituted with F, Cl, C(=O)Ra, NH₂, NO₂, S(=O)₂Rb, 5- or 6-membered heteroaryl including one to three heteroatoms independently selected from the group consisting of N and S in a ring;
Ra and Rb are each independently NH₂, or 5- to 7-membered heterocycloalkyl including one to three heteroatoms independently selected from the group consisting of N and O in a ring;
Y is a single bond, 6- to 12-membered arylene, 5- to 7-membered heterocycloalkylene including one to three heteroatoms independently selected from the group consisting of N, O and S in a ring, or 5- or 6-membered heteroarylene including one or two heteroatoms independently selected from the group consisting of N, O and S in a ring;
L₁ is a single bond, C1-C4 alkylene, C(=O), C(=O)NH, C(=O)NH-(C1-C2 alkylene), C(=O)NH-(C1-C5 alkylene)-C(=O)O, or C(=O)NH-(C1-C3 alkylene)-C(=O)NH;
R₁ is H, F, Cl, C1-C3 alkyl, C1-C3 alkoxy, OH, CF₃, NRcRd, 5- to 7-membered heterocycloalkyl including one to three heteroatoms independently selected from the group consisting of N and O in a ring, or 5- or 6-membered heteroaryl including one or two heteroatoms independently selected from the group consisting of N and O in a ring;
Rc and Rd are each independently C1-C3 alkyl;
Q₁ and Q₂ are each independently N or N-L₂-R₂, in which Q₁ and Q₂ are not simultaneously N;
when Q₂ is N, 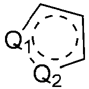 is 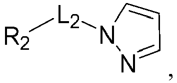 and when Q₁ is N, 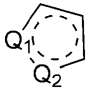 is 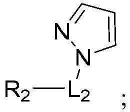
L₂ is C1-C6 alkylene or (C1-C3 alkylene)-O-(C1-C3 alkylene);
R₂ is 5- to 7-membered heterocycloalkyl including one to three heteroatoms independently selected from the group consisting of N and O in a ring, C1-C3 alkoxy, CONH₂, NReRf, SO₂Rg or OH;
Re, Rf and Rg are each independently C1-C3 alkyl.

4. A compound described in a following table, a stereoisomer thereof, a pharmaceutically acceptable salt thereof, or a hydrate or solvate thereof:
| **No.** | **Structure** | **No.** | **Structure** |
|---|---|---|---|
| **1** | 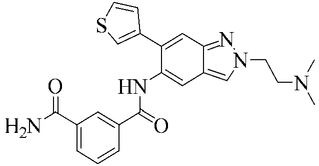 | **2** | 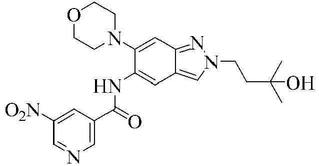 |
| **3** | 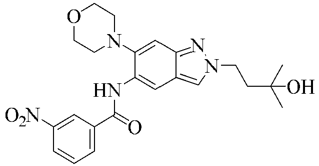 | **4** | 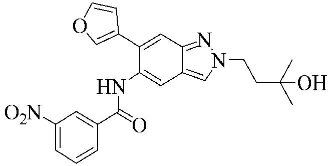 |
| **5** | 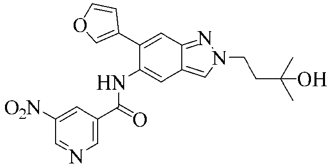 | **6** | 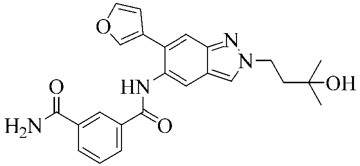 |
| **7** | 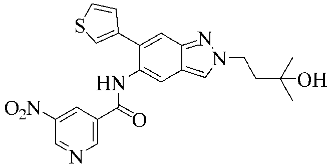 | **8** | 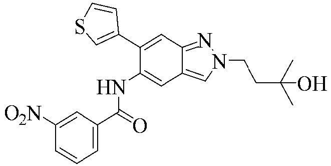 |
| **9** | 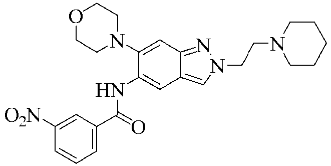 | **10** | 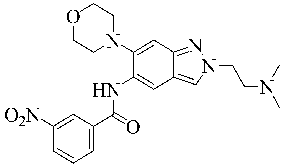 |
| **11** | 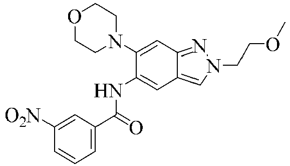 | **12** | 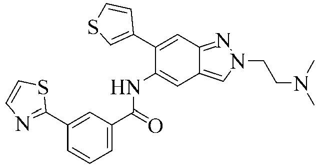 |
| **13** | 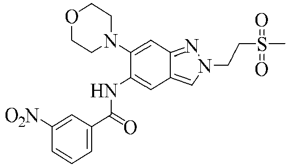 | **14** | 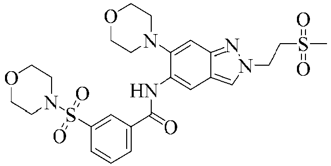 |
| **15** | 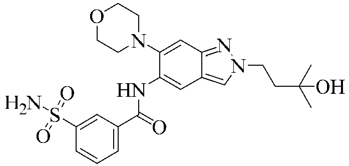 | **16** | 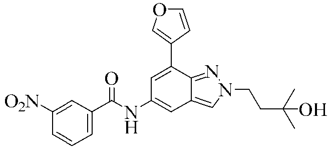 |
| **17** | 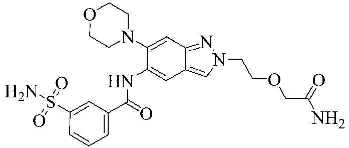 | **18** | 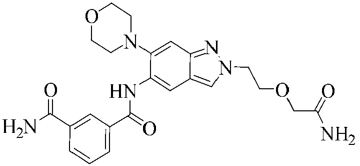 |
| **19** | 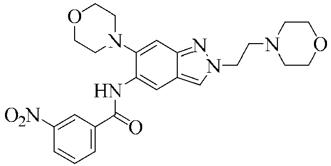 | **20** | 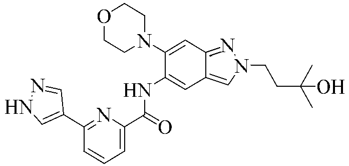 |
| **21** | 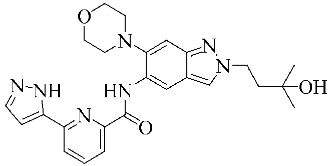 | **22** | 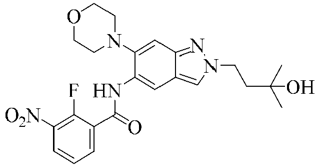 |
| **23** | 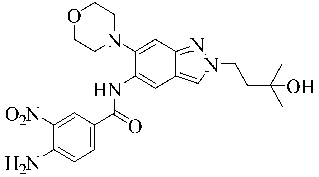 | **24** | 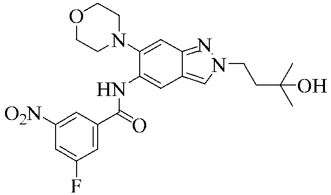 |
| **25** | 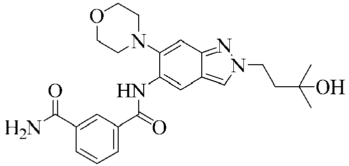 | **26** | 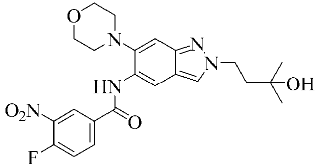 |
| **27** | 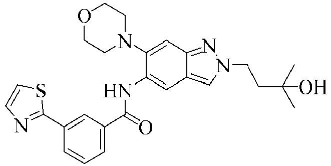 | **28** | 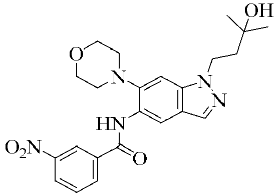 |
| **29** | 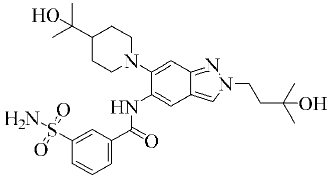 | **30** | 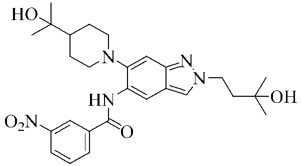 |
| **31** | 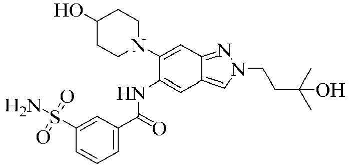 | **32** | 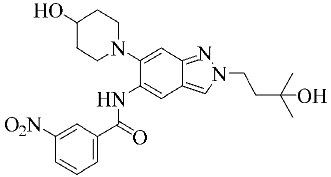 |
| **33** | 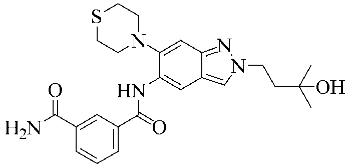 | **34** | 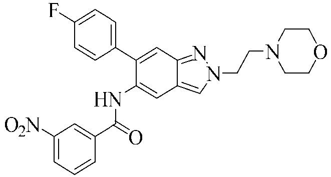 |
| **35** | 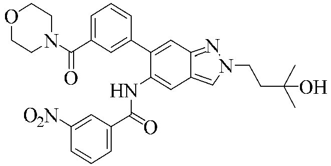 | **36** | 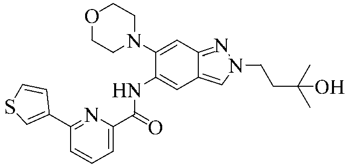 |
| **37** | 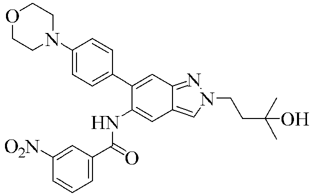 | **38** | 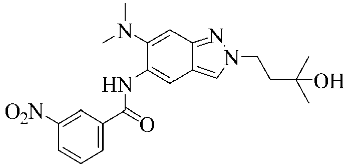 |
| **39** | 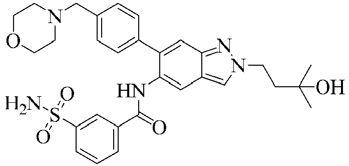 | **40** | 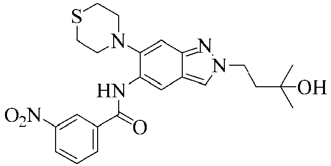 |
| **41** | 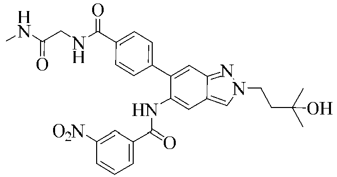 | **42** | 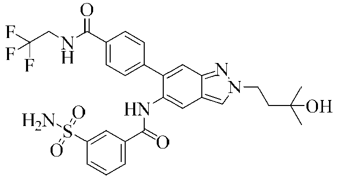 |
| **43** | 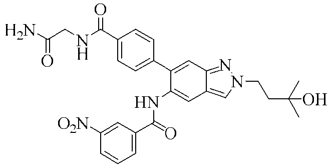 | **44** | 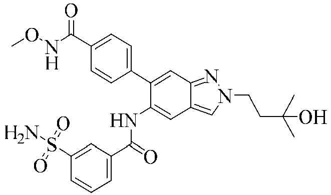 |
| **45** | 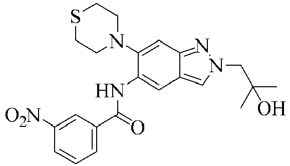 | **46** | 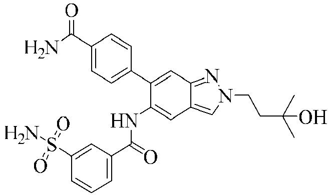 |
| **47** | 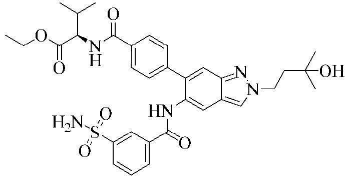 | **48** | 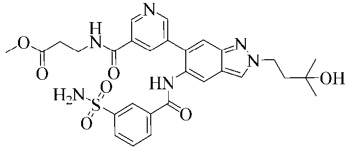 |
| **49** | 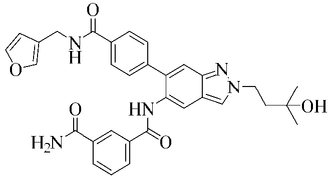 | **50** | 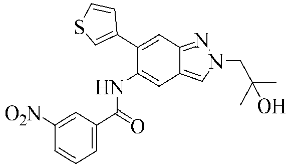 |
| **51** | 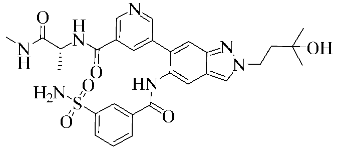 | **52** | 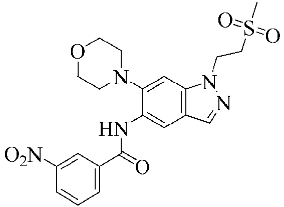 |
| **53** | 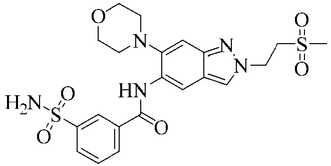 | | |

5. A pharmaceutical composition comprising a compound according to any one of claims 1 to 4, a stereoisomer thereof, a pharmaceutically acceptable salt thereof, or a hydrate or solvate thereof as an active ingredient.

6. The pharmaceutical composition of claim 5, wherein the pharmaceutical composition is for preventing or treating IRAK-4- or IRAK-1-related diseases.

7. The pharmaceutical composition of claim 6, wherein the IRAK-4- or IRAK-1-related disease is an autoimmune disease, an inflammatory disease or a tumor.

8. A method for preventing or treating IRAK-4- or IRAK-1-related diseases, the method comprising administering a compound according to any one of claims 1 to 4, a stereoisomer thereof, a pharmaceutically acceptable salt thereof, or a hydrate or solvate thereof into a subject.

9. A use of a compound according to any one of claims 1 to 4, a stereoisomer thereof, a pharmaceutically acceptable salt thereof, or a hydrate or solvate thereof for preventing or treating IRAK-4- or IRAK-1-related diseases.

10. A use of a compound according to any one of claims 1 to 4, a stereoisomer thereof, a pharmaceutically acceptable salt thereof, or a hydrate or solvate thereof for preparing a medicament for preventing or treating IRAK-4- or IRAK-1-related diseases.
